# EUROPÄISCHE PATENTSCHRIFT

(11) **EP 0 541 594 B1**
(45) Veröffentlichungstag und Bekanntmachung des Hinweises auf die Patenterteilung: **03.05.1995**
(21) Anmeldenummer: 91912997.3
(22) Anmeldetag: 22.07.1991
(51) Int. Cl.: C07C 405/00, A61K 31/557

(54) **CYCLOPENTANDERIVATE, VERFAHREN ZU IHRER HERSTELLUNG UND IHRE PHARMAZEUTISCHE VERWENDUNG**
CYCLOPENTANE DERIVATIVES, PROCESS FOR PRODUCING THEM AND THEIR PHARMACEUTICAL USE
DERIVES DE CYCLOPENTANE, PROCEDE POUR LEUR FABRICATION, AINSI QUE LEUR UTILISATION PHARMACEUTIQUE

(30) Priorität: 27.07.1990 DE 4024347
(43) Veröffentlichungstag der Anmeldung: 19.05.1993
(73) Patentinhaber: SCHERING AKTIENGESELLSCHAFT, 13353 Berlin (DE)
(72) Erfinder: KLAR, Ulrich, Dr., W-1000 Berlin 27 (DE); REHWINKEL, Hartmut, Dr., W-1000 Berlin 44 (DE); VORBRÜGGEN, Helmut, Prof., W-1000 Berlin 27 (DE); THIERAUCH, Karl-Heinz, Dr., W-1000 Berlin 37 (DE); VERHALLEN, Peter, Dr., O-1110 Berlin (DE)
(86) Internationale Anmeldenummer: DE9100604
(87) Internationale Veröffentlichungsnummer: WO9202495

(56) Entgegenhaltungen:
- EP-A- 0 069 696
- EP-A- 0 299 914
- WO-A-85/00367
- WO-A-86/05488
- WO-A-91/01302
- FR-A- 2 215 961
- FR-A- 2 285 866
- FR-A- 2 349 328

## Beschreibung

Die Erfindung betrifft Cyclopentan-Derivate, Verfahren zu ihrer Herstellung sowie ihre Verwendung als Hilfsstoffe für pharmakologische Untersuchungen und als Arzneimittel.
Cyclopentan-Derivate sind in den letzten Jahren intensiv bearbeitet worden, da vom Cyclopentan-System abgeleitete Prostaglandine wie z.B. PGA₂, PGB₂, PGE₂, 6-Oxo-PGE₁, PGD₂, PGF_{2α}, PGJ₂ und ihre Analoga unterschiedlichste biologische Wirkungen z.B. auf das Herz-Kreislauf-, ZNS- oder Immun-System besitzen.
Es wurde überraschenderweise gefunden, daß durch die Einführung eines Fluoratomes oder einer Hydroxygruppe in Position 9 (Prostaglandin-Zählweise) des Prostangerüstes in Kombination mit unterschiedlichsten Strukturmerkmalen in der unteren Kette sowie in Position 11 chemisch und metabolisch stabile Prostaglandin-Analoga erhalten werden, die in der Lage sind, die pharmakologischen Eigenschaften des instabilen Thromboxan-A₂ (TXA₂) bzw. PGH₂ sowie seiner stabilen Analoga wie z.B. U46619 oder U44069 am Rezeptor zu antagonisieren.
Die Verbindungen dieser Erfindung stellen deshalb wertvolle Hilfsmittel zur selektiven Therapie von Erkrankungen, die auf einen Überschuß an TXA₂ bzw. PGH₂ zurückzuführen sind, dar.

Die Erfindung betrifft Cyclopentanderivate der Formel I, 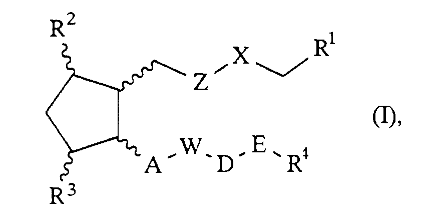
worin
R¹ 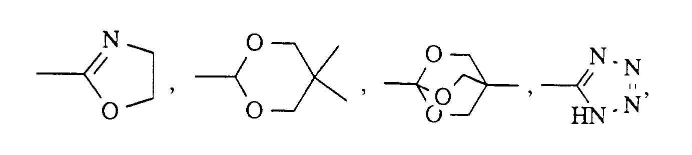
oder COOR⁵, wobei R⁵
Wasserstoff oder gegebenenfalls durch Halogen, Phenyl, C₁-C₄-Alkoxy oder Di-(C₁-C₄)-alkylamino substituiertes C₁-C₁₀-Alkyl, C₃-C₁₀-Cycloalkyl, C₇-C₁₆-Aralkyl, durch Y substituiertes Phenacyl oder C₆-C₁₂-Aryl oder einen 5- oder 6- gliedrigen heterocyclischen Rest mit wenigstens einem N-, O- oder S-Atom bedeuten kann, oder -CONHR⁷ mit R⁷ in der Bedeutung Wasserstoff, C₁-C₁₀-Alkyl, C₆-C₁₂-Arylsulfonyl, C₁-C₁₀-Alkanoyl oder C₁-C₁₀-Alkansulfonyl sein kann,
X -(CH₂)ₚ-, -CH₂-O-, oder -CH₂-S-,
Z, A unabhängig voneinander eine Direktbindung, (Z)-CH=CH-, (E)-CH=CH-, oder -C≡C-,
p 0 bis 5,
R² Fluor, OH,
n, r unabhängig voneinander 0 bis 2,
R³ OR⁶ oder R⁶,
W eine Direktbindung, eine -[(CH₂)ₙ-V]_{q}-Gruppe, eine -(CH₂)ₙ-V-(CH₂)_{q}-V-Gruppe, eine freie oder funktionell abgewandelte Hydroxymethylengruppe,oder eine freie oder funktionell abgewandelte 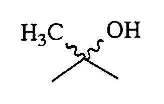
-Gruppe, wobei die Hydroxygruppe jeweils α- oder β-ständig sein kann,
q 1 oder 2,
D eine Direktbindung, eine geradkettige gesättigte Alkylengruppe mit 1-5 C-Atomen, eine verzweigte gesättigte Alkylengruppe oder eine geradkettige oder verzweigte ungesättigte Alkylengruppe mit 2-5 C-Atomen, die gegebenenfalls durch Fluoratome substituiert sein können, - (CH₂)ₙ-NH-SO₂-, 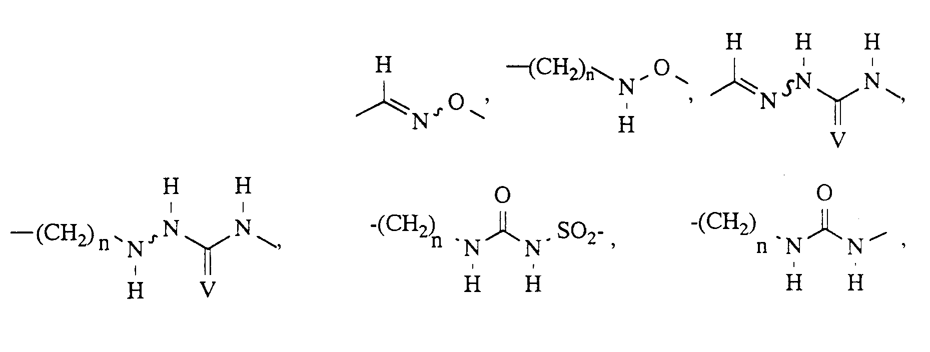 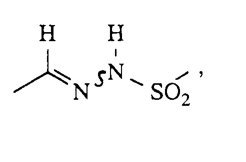
-(CH₂)ₙ-NH-NH-SO₂-, oder 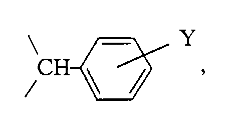
V ein O- oder S-Atom,
E eine Direktbindung, -C≡C- oder -CH=CR⁸-, wobei R⁸ Wasserstoff, C₁-C₅-Alkyl, Halogen oder Trifluormethyl,
AWDE gemeinsam eine Direktbindung,
AW gemeinsam eine Direktbindung,
DE gemeinsam eine Direktbindung sein können,
R⁴ 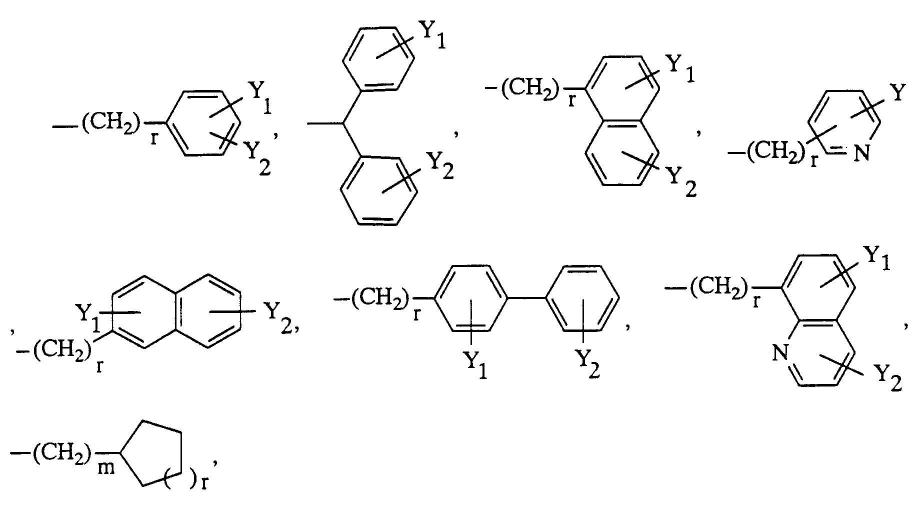
C₃-C₁₀-Cycloalkyl oder gegebenenfalls durch Y substituiertes C₁-C₁₀-Alkyl
m 1 oder 2,
Y₁ und Y₂ gleich oder verschieden sind und Y bedeuten,
Y Wasserstoff, Halogen, N₃, CF₃, OR⁶, NO₂_{,} NH₂, CN, COOR⁶ oder C₁-C₁₀-Alkyl,
R⁶ Wasserstoff, C₁-C₁₀-Alkyl, gegebenenfalls durch Halogen substituiertes C₆-C₁₂-Aryl oder C₇-C₁₆-Aralkyl sein kann und, falls R⁵ Wasserstoff bedeutet, deren Salze mit physiologisch verträglichen Basen, sowie die α-, β- oder γ-Cyclodextrinclathrate, sowie die mit Liposomen verkapselten Verbindungen der Formel I bedeuten.
Die Definition 5- oder 6-gliedriger heterocyclischer Rest betrifft Heterocyclen, die wenigstens ein Heteroatom, vorzugsweise Stickstoff, Sauerstoff oder Schwefel enthalten und mono- oder bicyclisch sind. Beispielsweise seien genannt 2-Furyl, 3-Furyl, 2-Thienyl, 3-Thienyl, 2-Pyridyl, 3-Pyridyl, 4-Pyridyl, Chinolyl, Isochinolyl.
Als Alkylgruppen R⁴, R⁵, R⁶, E und Y sind gerad- oder verzweigtkettige Alkylgruppen mit 1 - 10 C-Atomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.
Die Alkylgruppen R⁴, R⁵, R⁶, E und Y können substituiert sein durch Halogenatome, Hydroxygruppen, C₁-C₄-Alkoxygruppen, C₆-C₁₂-Arylgruppen, die durch Halogen substituiert sein können, Di-(C₁-C₄)-Alkylamine und Tri-(C₁-C₄)-Alkylammonium. Bevorzugt sind solche Alkylgruppen, die einfach substituiert sind.
Als Substituenten seien beispielsweise genannt Fluor-, Chlor- oder Bromatome, Phenyl, Dimethylamino, Diethylamino, Methoxy, Ethoxy.
Als bevorzugte Alkylgruppen R⁴, R⁵, R⁶, E und Y sind solche mit 1 - 5 C-Atomen, wie z. B. Methyl, Ethyl, Propyl, Isobutyl, Butyl, Neopentyl, tert.-Butyl, Chlorethyl, 1-und 2-Chlorpropyl, Hydroxyethyl und 1- und 2-Hydroxypropyl zu nennen.
Als Arylgruppen R⁵ und R⁶ kommen beispielsweise in Betracht: Phenyl, Diphenyl, 1-Naphthyl und 2-Naphthyl, die substituiert sein können durch 1 - 3 Halogenatome, eine Phenylgruppe, 1 - 3 Alkylgruppen mit jeweils 1 - 4 C-Atomen eine Chlormethyl-, Fluormethyl-, Carboxyl-, C₁-C₄-Alkoxy- oder Hydroxygruppe. Bevorzugt ist die Substitution in 3- und 4-Stellung am Phenylring zum Beispiel durch Fluor, Chlor, C₁-C₄- Alkoxy oder Trifluormethyl oder in 4-Stellung durch Hydroxy.
Die Cycloalkylgruppen R⁵ können im Ring 3-10, vorzugsweise 3-6 Kohlenstoffatome enthalten. Die Ringe können durch Alkylgruppen mit 1-4 Kohlenstoffatomen substituiert sein. Beispielsweise seien genannt Cyclopropyl, Cyclobutyl, Cyclopentyl, Cyclohexyl, Methylcyclopentyl, Methylcyclohexyl.
Die unter den Definitionen genannten C₁-C₁₀-Alkylgruppen sollen geradkettige oder verzweigte Alkylgruppen sein, wie sie für die vorstehenden Alkylgruppen bereits genannt wurden.
Die Hydroxygruppen in R², R³, Y und W können funktionell abgewandelt sein, beispielsweise durch Veretherung oder Veresterung, wobei die freie oder abgewandelte Hydroxygruppe in R² und W α- oder β-ständig sein kann, wobei freie Hydroxygruppen bevorzugt sind.
Als Ether- und Acylreste kommen die dem Fachmann bekannten Reste in Betracht. Bevorzugt sind leicht abspaltbare Etherreste, wie beispielsweise der Tetrahydropyranyl-, Tetrahydrofuranyl-, tert.-Butyldimethylsilyl-, tert.-Butyldiphenylsilyl-, Tribenzylsilylrest. Als Acylreste kommen z.B. Acetyl, Propionyl, Butyryl, Benzoyl in Frage. Halogen in den Definitionen für R⁵, R⁶, E und Y bedeutet Fluor, Chlor, Brom und Iod. Die Reste "C₁-C₁₀-Alkanoyl" oder "C₁-C₁₀-Alkansulfonyl" für R⁷ entsprechen den bereits genannten Alkylgruppen gleicher Länge mit dem Unterschied, daß sie an eine Carboxylgruppe bzw. Sulfonylgruppe gebunden sind. Bevorzugt sind C₁-C₄-Alkanoyl bzw. -Alkansulfonyl.
Zur Salzbildung mit den freien Säuren (R⁵ = H) sind anorganische und organische Basen geeignet, wie sie dem Fachmann zur Bildung physiologisch verträglicher Salze bekannt sind. Beispielsweise seien genannt: Alkalihydroxide, wie Natrium- oder Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin u.s.w.

Bevorzugt werden die Verbindungen der Formel I, in denen
R¹ die Gruppe COOR⁵ oder CONHR⁷,
R³ Wasserstoff, Hydroxyl, gegebenenfalls durch Halogen substituiertes C₁-C₄-Alkyl, C₆-C₁₂-Aryl oder C₇-C₁₆-Aralkyl,
R⁵ Wasserstoff, oder gegebenenfalls durch Halogen substituiertes C₇-C₁₆-Aralkyl, C₅-C₆-Cycloalkyl oder C₁-C₁₀-Alkyl
R⁷ C₁-C₇-Alkanoyl, C₆-C₁₂-Arylsulfonyl oder C₁-C₇-Alkansulfonyl,
p 0 bis 4
n,r unabhängig voneinander 0 oder 1 bedeuten.

Besonders bevorzugt werden die Verbindungen der Formel I, in denen
R¹ die Gruppe COOR⁵,
R³ Wasserstoff, Hydroxyl, Phenyl oder Phenylethyl,
R⁵ Wasserstoff oder Methyl,
R⁷ Methansulfonyl,
p 0 bis 4
n, r unabhängig voneinander 0 oder 1 bedeuten.

Die anmeldungsgemäßen Verbindungen der Formel I lassen sich, wie nachfolgend näher beschrieben, herstellen: 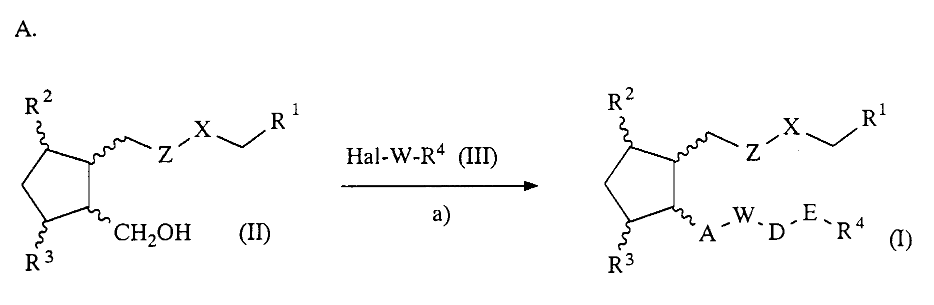
mit R¹, R², R³, R⁴, X, Z in den oben angegebenen Bedeutungen,
Hal als Brom oder Chlor,
A, DE als Direktbindung,
R¹ in der Bedeutung eines -COOR⁵-Esters. 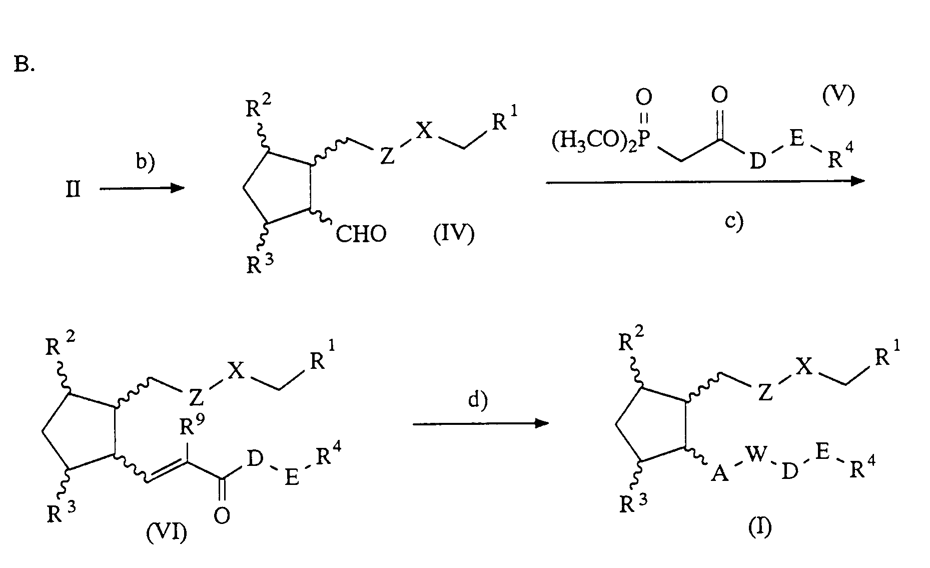
mit R², R³, R⁴, A, E, X, Z in den oben angegebenen Bedeutungen,
D als gegebenenfalls durch Alkyl substituiertes Alkylen,oder

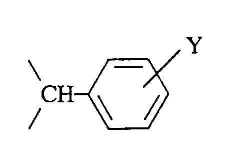

R¹ in der Bedeutung eines -COOR⁵-Esters,
R⁹ als Wasserstoff oder Brom,
W -CH(OH)-. 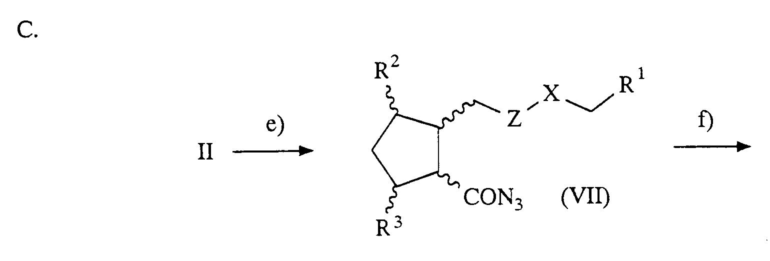 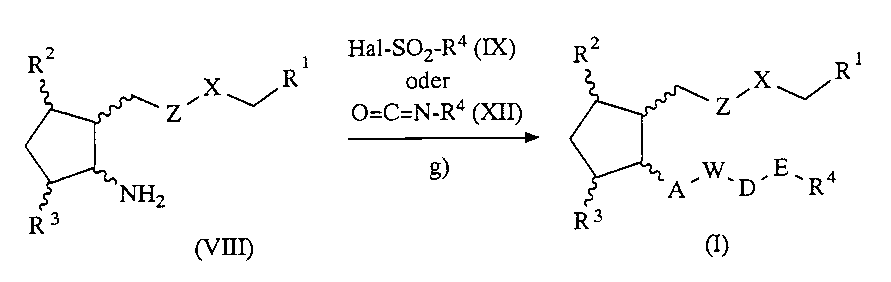
mit R², R³` R⁴, X, Z, Hal in den oben angegebenen Bedeutungen,
AW, E als Direktbindung,
D in der Bedeutung 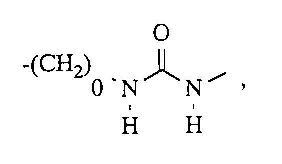
-(CH₂)₀-NH-SO₂-,
R¹ in der Bedeutung eines -COOR⁵-Esters. 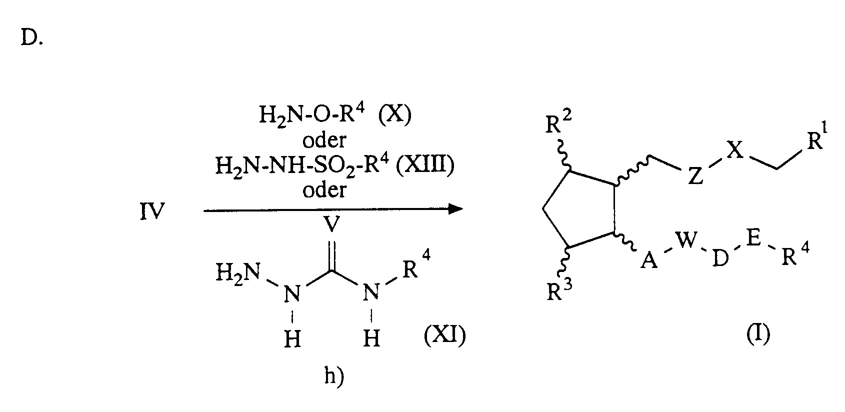
mit R², R³, R⁴, X, Z in den oben angegebenen Bedeutungen,
AW, E als Direktbindung,
R¹ in der Bedeutung eines -COOR⁵-Esters,
D in der Bedeutung 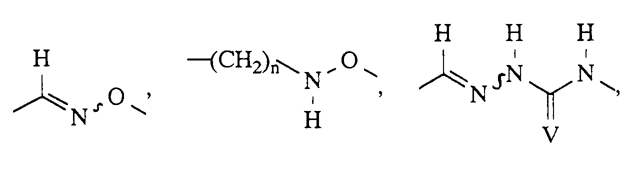 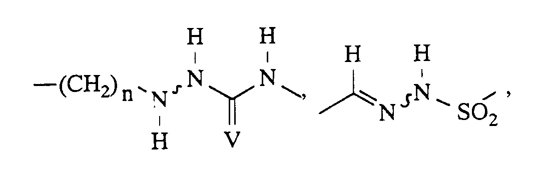
-(CH₂)ₙ-NH-NH-SO₂-. 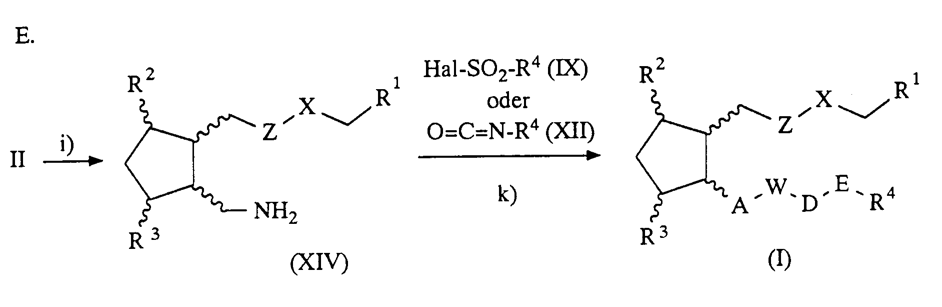
mit R², R³, R⁴, X, Z, Hal in den oben angegebenen Bedeutungen,
AW, E als Direktbindung,
D in der Bedeutung 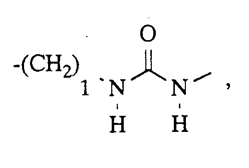
-(CH₂)₁-NH-SO₂-,
R¹ in der Bedeutung eines -COOR⁵-Esters.

Die Verbindungen der Formel I lassen sich nach Anspruch 3 entsprechend den oben beschriebenen Verfahrensalternativen herstellen. Die Ausgangsverbindungen der Formel II werden entsprechend den in den Beispielen 1g-1n, 24a-24g und 34a-34b angegebenen Vorschriften hergestellt.

Die Reaktionsbedingungen der nachfolgenden Verfahrensstufen sind:
a) II ⇒ I (Verfahren A)
   In Gegenwart von wässrigen Alkali- oder Erdalkalilösungen und unter Verwendung von Phasentransfer-Katalysatoren (wie z.B. Tetrabutylammoniumhydrogenphosphat oder -sulfat) werden Verbindungen der Formel II bei 20 bis 100°C in 1 bis 16 Std. mit dem Reaktanden III als organische Phase oder einer Lösung von III in einem inerten, nicht mit Wasser mischbaren organischen Lösungsmittel umgesetzt, gegebenenfalls, wie in Beispiel 1 beschrieben, ein Fluoratom für R² eingeführt bzw. eine Hydroxylgruppe, wie in den Beispielen 74 und 74a beschrieben, entfernt.
b) II ⇒ IV (Verfahren B)
   Die Oxidation von Verbindungen der Formel II erfolgt nach bekannten Verfahren, wie z.B. nach dem von Swern, Collins sowie unter Verwendung von Pyridiniumdichromat bzw. -chlorochromat in Lösungsmitteln wie Dichlormethan, Diethylether, Tetrahydrofuran, Benzol oder Toluol bei -80°C bis -50°C (Swern) bzw. bis +30°C (bei den übrigen Oxidationen) innerhalb von 10 Minuten bis 8 Stunden.
c) IV ⇒ VI (Verfahren B), d) VI ⇒ I (Verfahren B)
   Die Umsetzung der Verbindungen IV mit den Phosphonaten V sowie die sich anschließende Reduktion bzw. HBr-Eliminierung erfolgten analog den in der DE-OS 2845770 genannten Bedingungen.
e) II ⇒ VII (Verfahren C)
   Die Oxidation der Verbindungen der Formel II wird bevorzugt mit Jones-Reagenz oder Pyridiniumchlorochromat nach den dem Fachmann bekannten Reaktionsbedingungen durchgeführt. Anschließend wird unter üblichen Bedingungen mit Thionylchlorid zum Säurechlorid umgesetzt, unter Phasentransferkatalyse mit wässriger Natriumazidlösung zur Reaktion gebracht und wie in Beispiel 1c beschrieben zu Verbindungen der Formel VIII umgelagert, gegebenenfalls eine C-C-Mehrfachbindung in Z hydriert bzw. ein Fluoratom, wie in Beispiel 1 beschrieben, eingeführt.
f) VII ⇒ VIII (Verfahren C)
   Die Umsetzung der Verbindungen der Formel VII zu Verbindungen der Formel VIII erfolgt wie in Beispiel 1c beschrieben.
g) VIII ⇒ I (Verfahren C)
   Die Umsetzung der Verbindungen der Formel VIII mit den Verbindungen der Formel IX oder XII erfolgt wie in den dafür genannten Beispielen 1b und 78.
h) IV ⇒ I (Verfahren D)
   Die Umsetzung erfolgt in Analogie zu dem in WO 90/02740 (Verfahren C, S. 16) beschriebenen Verfahren.
i) II ⇒ XIV (Verfahren E)
   Die Umsetzung der Verbindungen der Formel II zu Verbindungen der Formel XIV erfolgt wie in den Beispielen 78a bis 78c Beschrieben.
k) XIV ⇒ I (Verfahren E)
   Die Umsetzung der Verbindungen der Formel XIV mit den Verbindungen der Formel IX oder XII erfolgt wie in den Beispielen 1b und 78 beschrieben.

Die Freisetzung der funktionell abgewandelten Hydroxygruppen R², R³, R⁴ und W erfolgt nach den dem Fachmann bekannten Methoden. Beispielsweise wird die Abspaltung von Etherschutzgruppen in einer wässrigen Lösung einer organischen Säure, wie z.B. Essigsäure, Propionsäure, Zitronensäure u.a. oder in einer wässrigen Lösung einer anorganischen Säure, wie z.B. Salzsäure, oder im Falle von Tetrahydropyranylethern unter Verwendung von Pyridinium-p-Toluolsulfonat, vorzugsweise in Alkoholen als Lösungsmittel oder unter Verwendung von wasserfreiem Magnesiumbromid, vorzugsweise in Diethylether als Lösungsmittel durchgeführt.
Zur Verbesserung der Löslichkeit wird bei Verwendung wässrig-saurer Reaktionsbedingungen zweckmäßigerweise ein mit Wasser mischbares inertes Lösungsmittel zugesetzt. Als geeignet erweisen sich z.B. Alkohole wie Methanol und Ethanol, Ether wie Dimethoxyethan, Dioxan und Tetrahydrofuran, wobei Tetrahydrofüran bevorzugt angewendet wird.

Die Abspaltung der Silyletherschutzgruppen erfolgt beispielsweise mit Tetrabutylammoniumfluorid nach den dem Fachmann bekannten Methoden. Als Lösungsmittel sind beispielsweise Tetrahydrofuran, Diethylether, Dioxan, Methylenchlorid etc. geeignet. Die Abspaltung wird vorzugsweise bei Temperaturen zwischen 20°C und 80°C durchgeführt.

Die Verseifung der Acylgruppen und ester in COOR⁵ der Cyclopentanderivate wird nach den dem Fachmann bekannten Methoden durchgeführt, wie beispielsweise mit basischen Katalysatoren wie z.B. mit Alkali- oder Erdalkali-carbonaten oder -hydroxiden in einem Alkohol oder der wässrigen Lösung eines Alkohols. Als Alkohole kommen aliphatische Alkohole wie z.B. Methanol, Ethanol, Butanol etc. in Betracht, vorzugsweise jedoch Methanol. Als Alkalicarbonate und -hydroxide seien Lithium-, Natrium- und Kaliumsalze genannt. Bevorzugt sind die Lithium- und Kaliumsalze. Als Erdalkalicarbonate und -hydroxide eignen sich beispielsweise Calciumcarbonat, Calciumhydroxid und Bariumcarbonat. Die Umsetzung erfolgt allgemein bei -10°C bis +70°C, vorzugsweise jedoch bei +25°C.

Die Einführung der Estergruppe CO₂R⁵ für R¹ bzw. CO₂R⁶ für Y, bei welcher R⁵ bzw. R⁶ eine Alkylgruppe mit 1-10-C-Atomen darstellt, erfolgt nach den dem Fachmann bekannten Methoden. Die Carboxyverbindungen (R⁵ = H bzw. R⁶ = H) werden beispielsweise mit Diazokohlenwasserstoffen in an sich bekannter Weise umgesetzt. Die Veresterung mit Diazokohlenwasserstoffen erfolgt z.B. dadurch, daß eine Lösung des Diazokohlenwasserstoffes in einem inerten Lösungsmittel, vorzugsweise in Diethylether, mit der Carboxyverbindung, gelöst in dem gleichen oder in einem anderen ebenfalls inerten Lösungsmittel, wie z.B. Methylenchlorid, vermischt wird. Nach beendeter Umsetzung in 1 bis 60 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389-394(1954)].

Die Einführung der Estergruppe CO₂R⁵ für R¹ bzw. CO₂R⁶ für Y, bei welcher R⁵ bzw. R⁶ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base wie z.B. Pyridin, DMAP, Triethylamin, in einem inerten Lösungsmittel wie z.B. Methylenchlorid, Ethylenchlorid, Chloroform, Essigsäureethylester, Tetrahydrofuran, vorzugsweise jedoch mit Chloroform umgesetzt. Die Reaktion wird bei Temperaturen zwischen -30°C und +50°C, vorzugsweise bei +10°C, durchgeführt.

Die Cyclopentanderivate der Formel I mit R⁵ bzw. R⁶ in der Bedeutung eines Wasserstoffatomes können mit geeigneten Mengen der entsprechenden anorganischen Basen unter Neutralisierung in Salze überführt werden. Beispielsweise erhält man beim Lösen der entsprechenden Säuren in Wasser, welches stöchiometrische Mengen der Base enthält, nach Abdampfen des Wassers oder nach Zugabe eines mit Wasser mischbaren Lösungsmittels, z.B. Alkohol oder Aceton, das feste anorganische Salz.

Die Herstellung der Aminsalze erfolgt in üblicher Weise. Dazu löst man die Säure in einem geeigneten Lösungsmittel, wie z.B. Ethanol, Aceton, Diethylether oder Benzol und setzt 1 bis 5 Äquivalente des jeweiligen Amins dieser Lösung zu. Dabei fällt das Salz gewöhnlich in fester Form an oder wird nach Verdampfen des Lösungsmittels in üblicher Weise isoliert.

Die funktionelle Abwandlung der freien Hydroxygruppen erfolgt nach den dem Fachmann bekannten Methoden. Zur Einführung der Etherschutzgruppen wird beispielsweise mit Dihydropyran oder Methylvinylether in Methylenchlorid oder Chloroform unter Verwendung katalytischer Mengen eines sauren Kondensationsmittels wie z.B. p-Toluolsulfonsäure, umgesetzt. Der jeweilige Enolether wird im Überschuß, vorzugsweise in der 1,2- bis 10-fachen Menge des theoretischen Bedarfs, zugesetzt. Die Umsetzung erfolgt normalerweise bei -10°C bis +30°C und ist nach 2 bis 45 Minuten beendet.

Zur Einführung von Silyletherschutzgruppen wird beispielsweise mit t-Butyl-diphenylchlorsilan oder t-Butyl-dimethylchlorsilan in Dimethylformamid unter Verwendung einer Base wie z.B. Imidazol, umgesetzt. Das jeweilige Silylchlorid wird im Überschuß, vorzugsweise in der 1,05- bis 4-fachen Menge des theoretischen Bedarfs, zugesetzt. Die Umsetzung erfolgt normalerweise bei 0°C bis 30°C und ist nach 1 bis 24 Stunden beendet.

Die Einführung der Acylschutzgruppen erfolgt, indem man eine Verbindung der Formel I in an sich bekannter Weise mit einem Carbonsäurederivat, wie z.B. Säurechlorid, Säureanhydrid etc., umsetzt.

Cyclodextrinclathrate werden analog der Vorschrift in WO 87/05294 erhalten.

Liposomen werden nach dem in "Pharmazie in unserer Zeit 11, 98 (1982)" beschriebenen Herstellungsverfahren hergestellt.

Alle stereoisomeren Formen gehören ebenfalls zum Gegenstand der Erfindung.

### Biologische Wirkung und Anwendungsbereich der neuen TXA₂-Antagonisten:

Die Verbindungen dieser Erfindung eignen sich zur Therapie von Erkrankungen des cardiovaskulären Systems, des Magens, des Pankreas, der Leber und der Niere. Sie wirken blutdrucksenkend und bronchodilatorisch. Sie sind hervorragend geeignet zur Hemmung der Thrombozytenaktivierung. Folglich stellen die neuen TXA₂-Antagonisten der Formel I wertvolle pharmazeutische Wirkstoffe dar. Darüber hinaus zeichnen sich die Verbindungen durch höhere Selektivität, eine wesentlich längere Wirksamkeit und eine größere Stabilität verglichen mit ähnlichen TXA₂-Antagonisten aus.
Die neuen TXA₂-Antagonisten besitzen die für diese Verbindungsklasse typischen Eigenschaften, wie z.B. Senkung des peripheren arteriellen, des koronaren und des pulmonalen Gefäßwiderstandes, Senkung des pulmonalen Blutdrucks, Senkung des systemischen Blutdrucks ohne zugleich Schlagvolumen und koronare Durchblutung zu senken, Förderung der Nierendurchblutung und der Durchblutung anderer peripherer Organe, Erhöhung der cerebralen Durchblutung, Inhibierung der Thrombozytenaktivierung und Auflösung von Thromben, Inhibierung der Bronchokonstriktion, Inhibierung der Magensäuresekretion, Zytoprotektion des Herzens, der Magen- und Darmschleimhaut, der Leber, Zytoprotektion im Pankreas und in der Niere sowie antiallergische Eigenschaften. Daher sind die neuen TXA₂-Antagonisten prinzipiell geeignet zur Behandlung des Schlaganfalles, der Prophylaxe und Therapie koronarer Herzerkrankungen, zum Beispiel der Koronarthrombose, zur Behandlung des Herzinfarktes, peripherer Arterienerkrankungen, zur Prophylaxe und Therapie bei anderen thromboembolischen Erkrankungen und bei Arteriosklerose, bei ischämischen Attacken des ZNS-Systems und anderer Durchblutungsstörungen des Gehirns wie z.B. der Migräne, zur Behandlung der Hypertonie und zur Behandlung von Krankheiten, die mit einer Erhöhung des pulmonalen Gefäßwiderstandes einhergehen wie z.B. der pulmonalen Hypertonie und zur Therapie des Schocks, des Asthmas und der allergischen Rhinitis. Sie können ferner eingesetzt werden, zur Inhibierung von Geburtswehen und zur Behandlung von Schwangerschaftstoxikosen.
Die neuen TXA₂-Antagonisten können außerdem Anwendung finden zur Verbesserung der Organfunktion nach Transplantation, zum Beispiel bei der Nierentransplantation, zur Verhinderung von Abstoßungsreaktionen, an Stelle von Heparin oder als Adjuvans bei der Dialyse oder Hämofiltration und bei der Konservierung von Blutplasmakonserven, zum Beispiel von Blutplättchenkonserven.
Die neuen TXA₂-Antagonisten besitzen eine antimetastatische Wirkung und antiproliferative Eigenschaften. Sie sind prinzipiell geeignet zur Behandlung von Neoplasien. Die neuen TXA₂-Antagonisten können in Kombination, zum Beispiel mit Carbacyclinen, Prostacyclin und seinen Analoga, 7-Oxo-prostacyclinen, Prostaglandinen und deren Derivate und 6-Oxo-PGE₁- und 6-Oxo-9-Fluor-Prostaglandin-Derivaten, mit TXA₂-Synthetase-Inhibitoren, mit Phosphodiesterase-Inhibitoren, mit Antagonisten und Rezeptorantagonisten verschiedener Thrombozytenstimulatoren (z.B. ADP, Thrombin, Collagen, PAF` Adrenalin, Serotonin, Fibrinogen), mit Calciumantagonisten, mit Fibrinolytika und Thrombolytika, z.B. t-PA, Streptokinase, mit Heparin und anderen Antikoagulanzien, mit Cyclooxygenasehemmern, z.B. Acetylsalicylsäure, mit Hemmstoffen der Lipoxy-genasen sowie Antagonisten von Lipoxygenaseprodukten, mit Vasodilatatoren wie z.B. Nitroverbindungen, mit Antihypertensiva wie z.B. β-Blockern oder mit Diuretika Verwendung finden.
Die Dosis der Verbindungen ist 0,1-1000 mg/Tag, bevorzugt 0,1-500 mg/Tag, auch in mehreren Teildosierungen, wenn sie am menschlichen Patienten verabreicht werden. Die Einheitsdosis für den pharmazeutisch akzeptablen Träger beträgt 0,1-100 mg. Für die parenterale Verabreichung werden sterile, injizierbare wässrige oder ölige Lösungen benutzt. Für die orale Applikation sind beispielsweise Tabletten, Dragees oder Kapseln geeignet.
Die Erfindung betrifft damit auch Arzneimittel auf Basis der Verbindungen der allgemeinen Formel I und üblicher Hilfs- und Trägerstoffe.
Die erfindungsgemäßen Wirkstoffe sollen in Verbindung mit den in der Galenik bekannten und üblichen Hilfsstoffen z.B. zur Herstellung von Blutdrucksenkern dienen. Der Einheitsdosisberich für die Ampulle ist 0,1-100 mg, für die Tablette 0,1-100 mg.

### Beispiel 1:

### 7-[(1R,2S,5R)-2-(4-Toluolsulfonylamino)-5-fluor-cyclopentyl]-5(Z)-heptensäuremethylester:

55 mg (139 »mol) des nach Beispiel 1a dargestellten Alkohols löst man in 2,5 ml wasserfreiem Toluol, versetzt mit 56 »l wasserfreiem Pyridin, kühlt unter einer Atmosphäre aus trockenem Argon auf -60°C und versetzt mit 42 »l Diethylaminoschwefeltrifluorid. Man läßt innerhalb 3,5 Stunden auf 0°C erwärmen, quencht durch Zugabe von 1 ml einer gesättigten Natriumhydrogenkarbonatlösung, verdünnt mit Wasser und extrahiert mehrfach mit Dichlormethan. Die vereinigten organischen Extrakte trocknet man über Magnesiumsulfat und reinigt den nach Lösungsmittelabzug erhaltenen Rückstand durch Chromatographie an 4 analytischen Dünnschichtplatten. Als Laufmittel dient ein Gemisch aus n-Hexan und Aceton, als Elutionsmittel Diethylether. Isoliert werden 20 mg (53 »mol, 38%) 7-[5(S)-(4-Toluolsulfonylamino)-cyclopent-1enyl]-5(Z)-heptensäuremethylester sowie 16 mg (40 »mol, 29%) der Titelverbindung jeweils als farbloses Öl. IR (KBr): 3430, 3310, 3050, 3020, 2940, 2860, 1735, 1600, 1450, 1320, 1240, 1160, 1095, 920, 815, 670, 580 und 550 cm⁻¹.

### Beispiel 1a:

### 7-[(1R,2S,5S)-2-(4-Toluolsulfonylamino)-5-hydroxy-cyclopentyl]-5(Z)-heptensäuremethylester:

Die Lösung von 140 mg (280 »mol) der nach Beispiel 1b dargestellten Verbindung in 1,5 ml Methanol versetzt man mit 20 mg fein pulverisiertem Kaliumkarbonat und erhitzt 1 Stunde auf 70°C. Nach dem erkalten wird mit gesättigter Zitronensäure angesäuert, mit Wasser verdünnt und mehrfach mit Dichlormethan extrahiert. Die vereinigten organischen Extrakte trocknet man über Magnesiumsulfat und reinigt den nach Lösungsmittelabzug erhaltenen Rückstand durch Chromatographie an 2 analytischen Dünnschichtplatten. Als Laufmittel dient ein Gemisch aus n-Hexan und Ethylacetat, als Elutionsmittel Diethylether. Isoliert werden 96 mg (243 »mol, 87%) der Titelverbindung als farbloses Öl. IR (Film): 3600-3200, 3270, 2940, 2870, 1735, 1600, 1450, 1330, 1270, 1160, 1110, 1090, 1025, 815 und 665 cm⁻¹.

### Beispiel 1b:

### 7-[(1R,2S,5S)-2-(4-Toluolsulfonylamino)-5-benzoyloxy-cyclopentyl]-5(Z)-heptensäuremethylester:

Die Lösung von 340 mg (984 »mol) des nach Beispiel 1c dargestellten Amins in 30 ml wasserfreiem Dichlormethan versetzt man mit 1,52 ml Triethylamin, 483 mg p-Toluolsulfonsäurechlorid und rührt 1,5 Stunden bei 23°C unter einer Atmosphäre aus trockenem Argon. Man gießt auf eine halbkonzentrierte Natriumchloridlösung und extrahiert mehrfach mit Dichlormethan. Die vereinigten organischen Extrakte trocknet man über Magnesiumsulfat und reinigt den nach Lösungsmittelabzug erhaltenen Rückstand durch Chromatographie an ca. 70 ml feinem Kieselgel mit einem Gradientensystem aus n-Hexan und Ethylacetat Isoliert werden 330 mg (660 »mol, 67%) der Titelverbindung als farbloses Öl. IR (Film): 3270, 2940, 2870, 1730, 1710, 1600, 1450, 1330, 1270, 1160, 1110, 1090, 1025, 905, 815, 710 und 665 cm⁻¹.

### Beispiel 1c:

### 7-[(1R,2S,5S)-2-Amino-5-benzoyloxy-cyclopentyl]-5(Z)-heptensäuremethylester (A) und 7-[(1R,2S,5S)-2-Trifluoracetamido-5-benzoyloxy-cyclopentyl]-5(Z)-heptensäuremethylester (B):

Die nach Beispiel 1d gewonnene Lösung versetzt man mit 1,47 ml Trifluoressigsäure und erhitzt 6 Stunden zum Rückfluß. Man läßt 14 Stunden bei 23°C stehen, engt ein und reinigt den Rückstand durch Chromatographie an ca. 250 ml feinem Kieselgel mit einem Gradientensystem aus Dichlormethan und Methanol. Isoliert werden 2,0 g (5,79 mmol, 43% bezogen auf Edukt in Beispiel 1e) der Titelverbindung A sowie 1,53 g (3,47 mmol, 26% bezogen auf Edukt in Beispiel 1e) der Titelverbindung B.
IR (CHCl₃) von A: 3500-2600, 2950, 2870, 1710, 1670, 1450, 1275, 1190, 1140 und 835 cm⁻¹.
IR (Film) von B: 3320, 2950, 2870, 1740-1690, 1600, 1550, 1450, 1435, 1270, 1210, 1180, 1110, 1070, 1025 und 710 cm⁻¹.

### Beispiel 1d:

### 7-[(1R,2S,5S)-2-Azidocarbonyl-5-benzoyloxy-cyclopentyl]-5(Z)-heptensäuremethylester:

Den nach Beispiel 1e gewonnenen Rückstand löst man in 20 ml Dichlormethan, kühlt auf 3°C, versetzt mit 10 mg Tetrabutylammoniumhydrogensulfat und der Lösung von 1,04 g Natriumazid in 3,5 ml Wasser. Man rührt 2,5 Stunden, verdünnt mit Dichlormethan, trennt die organische Phase ab und trocknet über frisch geglühtem Magnesiumsulfat. Die nach Filtration erhaltene Lösung wird sofort weiter umgesetzt.

### Beispiel 1e:

### 7-[(1R,2S,5S)-2-Chlorcarbonyl-5-benzoyloxy-cyclopentyl]-5(Z)-heptensäuremethylester:

Die Lösung von 5,0 g (13,4 mmol) der nach Beispiel 1f dargestellten Verbindung in 133 ml wasserfreiem Dichlormethan versetzt man unter Eiskühlung mit 2,13 ml frisch destilliertem Thionylchlorid und läßt 20 Stunden bei 23°C unter einer Atmosphäre aus trockenem Argon rühren. Man engt ein und setzt den erhaltenen Rückstand ohne Reinigung weiter um.

### Beispiel 1f:

### 7-[(1R,2S,5S)-2-Hydroxycarbonyl-5-benzoyloxy-cyclopentyl]-5(Z)-heptensäuremethylester:

Die Lösung von 30,2 g (83,9 mmol) des nach Beispiel 1g dargestellten Alkohols in 725 ml Aceton kühlt man auf -15°C, versetzt mit 44 ml einer standardisierten Chromschwefelsäurelösung (Jones-Reagenz), rührt 3 Stunden bei -10°C und zersetzt überschüssiges Oxidationsmittel durch Zugabe von 13 ml Isopropanol. Man verdünnt mit Wasser, extrahiert mehrfach mit Diethylether, wäscht die vereinigten organiaschen Extrakte mit Wasser und gesättigter Natriumchloridlösung und trocknet über Magnesiumsulfat. Den nach Filtration und Lösungsmittelabzug erhaltenen Rückstand reinigt man durch Chromatographie an ca. 1l groben Kieselgel mit einem Gradientensystem aus n-Hexan und Ethylacetat. Isoliert werden 29,3 g (78,3 mmol, 93%) der Titelverbindung als farbloses Öl.
IR (Film): 3600-2500, 3060, 2950, 2860, 1740-1690, 1600, 1580, 1450, 1435, 1360, 1310, 1270, 1170, 1110, 1070, 1025 und 710 cm⁻¹.

### Beispiel 1g:

### 7-[(1R,2S,5S)-2-Hydroxymethyl-5-benzoyloxy-cyclopentyl]-5(Z)-heptensäuremethylester:

Die Lösung von 57,9 g (96,7 mmol) der nach Beispiel 1h dargestellten Verbindung in 124 ml wasserfreiem Tetrahydrofuran versetzt man mit 155 ml einer 1M Lösung von Tetrabutylammoniumfluorid in Tetrahydrofuran und rührt 17 Stunden bei 23°C unter einer Atmosphäre aus trockenem Argon. Man versetzt mit Wasser, extrahiert mehrfach mit Diethylether, wäscht die vereinigten organiaschen Extrakte mit Wasser und gesättigter Natriumchloridlösung und trocknet über Magnesiumsulfat. Den nach Filtration und Lösungsmittelabzug erhaltenen Rückstand reinigt man durch Chromatographie an ca. 2l groben Kieselgel mit einem Gradientensystem aus n-Hexan und Ethylacetat. Isoliert werden 30,2 g (83,8 mmol, 87%) der Titelverbindung als farbloses Öl.
IR (Film): 3600-3200, 3060, 2940, 2860, 1735, 1710, 1600, 1580, 1360, 1310, 1275, 1170, 1110, 1070, 1025 und 715 cm⁻¹.

### Beispiel 1h:

### 7-[(1R,2S,5S)-2-(tert.-Butyldiphenylsilyloxymethyl)-5-benzoyloxy-cyclopentyl]-5(Z)-heptensäuremethylester:

Die Lösung von 47,9 g (96,7 mmol) der nach Beispiel 1i dargestellten Verbindung in 212 ml wasserfreiem Pyridin kühlt man unter einer Atmosphäre aus trockenem Argon auf 5°C, versetzt innerhalb 30 Minuten mit 29 ml Benzoylchlorid und rührt 1,5 Stunden bei 23°C. Man gießt auf 600 ml Eiswasser, extrahiert mehrfach mit Diethylether, wäscht die vereinigten organiaschen Extrakte mit 2n Salzsäure, Wasser und gesättigter Natriumchloridlösung und trocknet über Magnesiumsulfat. Den nach Filtration und Lösungsmittelabzug erhaltenen Rückstand reinigt man durch Chromatographie an ca. 2l feinem Kieselgel mit einem Gradientensystem aus n-Hexan und Ethylacetat. Isoliert werden 57,9 g (96,7 mmol, 100%) der Titelverbindung als farbloses Öl.
IR (Film): 3070, 3040, 3000, 2950, 2930, 2850, 1735, 1710, 1600, 1450, 1425, 1360, 1310, 1270, 1110, 820, 740 und 705 cm⁻¹.

### Beispiel 1i:

### 7-[(1R,2S,5S)-2-(tert.-Butyldiphenylsilyloxymethyl)-5-hydroxy-cyclopentyl]-5(Z)-heptensäuremethylester:

Die Lösung von 154 g des nach Beispiel 1j dargestellten Rohproduktes in 150 ml Aceton versetzt man mit 33,4 g Kaliumkarbonat, 41,2 g Methyliodid und erwärmt 6 Stunden auf 80°C. Man engt ein, nimmt in 400 ml Dichlormethan auf, wäscht mit Wasser und gesättigter Natriumchloridlösung und trocknet über Magnesiumsulfat. Den nach Filtration und Lösungsmittelabzug erhaltenen Rückstand reinigt man durch Chromatographie an ca. 2l groben Kieselgel mit einem Gradientensystem aus n-Hexan und Ethylacetat. Isoliert werden 47,9 g (96,8 mmol, 88% bezogen auf Edukt in Beispiel 1j) der Titelverbindung als farbloses Öl.
IR (Film): 3600-3200, 3070, 3040, 3000, 2940, 2920, 2850, 1735, 1585, 1460, 1425, 1240, 1165, 1110, 1005, 820, 740 und 700 cm⁻¹.

### Beispiel 1j:

### 7-[(1R,2S,5S)-2-(tert.-Butyldiphenylsilyloxymethyl)-5-hydroxy-cyclopentyl]-5(Z)-heptensäure:

Zu der Emulsion aus 99,7 g Carboxybutyltriphenylphosphoniumbromid in 256 ml wasserfreiem Tetrahydrofuran und 600 ml wasserfreiem Dimethylsulfoxid gibt man innerhalb einer Stunde portionsweise insgesamt 50g fein pulverisiertes Kalium-tert.-butanolat. Man rührt so lange, bis eine klare rote Lösung entsteht, tropft zügig die Lösung von 43,8 g (110 mmol) der nach Beispiel 1k dargestellten Verbindung in 130 ml wasserfreiem Tetrahydrofuran zu und läßt 2 Stunden bei 23°C unter einer Atmosphäre aus trockenem Argon reagieren. Man gießt in Eiswasser, stellt durch Zugabe einer gesättigten Zitronensäurelösung einen pH-Wert von 4-5 ein und extrahiert mehrfach mit Dichlormethan. Man wäscht mit Wasser und gesättigter Natriumchloridlösung, trocknet über Magnesiumsulfat, filtriert und engt ein. Den erhaltenen Rückstand setzt man ohne Reinigung weiter um.

### Beispiel 1k:

### (1S,3RS,5R,6S)-3-Hydroxy-6-(tert.-butyldiphenylsilyloxymethyl)-2-oxabicyclo[3.3.0]octan:

Die Lösung von 45,8 g (116 mmol) der nach Beispiel 1l dargestellten Verbindung in 1,4 l wasserfreiem Toluol kühlt man unter einer Atmosphäre aus trockenem Argon auf - 70°C` tropft innerhalb einer Stunde 202 ml einer 1,2M Diisobutylaluminiumhydridlösung in Toluol zu und rührt 1 Stunde. Überschüssiges Reduktionsmittel wird durch Zugabe von 13 ml Isopropanol zersetzt. Man läßt auf 0°C erwärmen, tropft 100 ml Wasser zu und rührt so lange bei 23°C, bis sich ein feinkörniger Niederschlag gebildet hat. Man saugt ab, wäscht mit Dichlormethan nach und isoliert nach Lösungsmittelabzug 43,8 g (110 mmol, 95%) der Titelverbindung als farbloses Öl.
IR (Film): 3600-3200, 3070, 3050, 2940, 2850, 1590, 1465, 1425, 1390, 1360, 1260, 1110, 1010, 940, 820, 740 und 700 cm⁻¹.

### Beispiel 1l:

### (1S,5R,6S)-3-Oxo-6-(tert.-butyldiphenylsilyloxymethyl)-2-oxabicyclo[3.3.0]octan:

Die Lösung von 67 g (119 mmol) des nach Beispiel 1m dargestellten Tosylates in 1,3 l Dimethoxyethan versetzt man mit 136 g Natriumiodid, 118 g Zinkstaub, 79 ml Wasser und erhitzt 16 Stunden unter Rückfluß. Nach dem Erkalten wird von ungelösten Rückständen abfiltriert, das Filtrat auf ca. 200 ml eingeengt, mit Wasser versetzt und mehrfach mit Diethylether extrahiert. Die vereinigten organiaschen Extrakte wäscht man mit 10%iger Natriumhydrogensulfatlösung, Wasser, gesättigter Natriumchloridlösung und trocknet über Magnesiumsulfat. Den nach Filtration und Lösungsmittelabzug erhaltenen Rückstand reinigt man durch Chromatographie an ca. 2l groben Kieselgel mit einem Gradientensystem aus n-Hexan und Ethylacetat. Isoliert werden 45,8 g (116 mmol, 98%) der Titelverbindung als farbloses Öl.
IR (Film): 3070, 3050, 2950, 2930, 2850, 1775, 1590, 1470, 1425, 1165, 1110, 1005, 820, 740 und 705 cm⁻¹.

### Beispiel 1m:

### (1S,5R,6S,7R)-3-Oxo-6-(tert.-butyldiphenylsilyloxymethyl)-7-toluolsulfonyloxy-2-oxabicyclo[3.3.0]octan:

Die Lösung von 67,3 g (164 mmol) des nach Beispiel 1n dargestellten Alkohols in 260 ml wassertreiem Pyridin versetzt man mit 62,8 g p-Toluolsulfonsäurechlorid und rührt 27 Stunden unter einer Atmosphäre aus trockenem Argon bei 50°C. Man engt ein, versetzt mit Wasser und extrahiert mehrfach mit Dichlormethan. Man wäscht mit Wasser und gesättigter Natriumchloridlösung und trocknet über Magnesiumsulfat. Den nach Filtration und Lösungsmittelabzug erhaltenen Rückstand reinigt man durch Chromatographie an ca. 2l groben Kieselgel mit einem Gradientensystem aus n-Hexan und Ethylacetat. Isoliert werden 67 g (119 mmol, 72%) der Titelverbindung als farbloses Öl.
IR (CHCl₃): 3070, 3030, 2960, 2930, 2860, 1770, 1600, 1470, 1425, 1365, 1190, 1175, 1110, 1040, 980, 960, 895, 875, 820 und 700 cm⁻¹.

### Beispiel 1n:

### (1S,5R,6S,7R)-3-Oxo-6-(tert.-butyldiphenylsilyloxymethyl)-7-hydroxy-2-oxabicyclo[3.3.0] octan:

Die Lösung von 129 g (251 mmol) (1S,5R,6S,7R)-3-Oxo-6-(tert.-butyldiphenylsilyloxymethyl)-7-benzoyloxy-2-oxabicyclo[3.3.0]octan in 1l Methanol versetzt man mit 14,9 g Kaliumkarbonat und rührt 3 Stunden bei 23°C unter einer Atmosphäre aus trockenem Argon. Man versetzt mit Wasser, neutralisiert durch vorsichtige Zugabe von 2n Salzsäure, engt ein und extrahiert mehrfach mit Dichlormethan. Man wäscht mit Wasser, gesättigter Natriumchloridlösung und trocknet über Magnesiumsulfat. Den nach Filtration und Lösungsmittelabzug erhaltenen Rückstand reinigt man durch Chromatographie an ca. 2l feinem Kieselgel mit einem Gradientensystem aus n-Hexan und Ethylacetat. Isoliert werden 97 g (236 mmol, 94%) der Titelverbindung als farbloses Öl.
IR (Film): 3600-3200, 3070, 3050, 2940, 2960, 1770, 1590, 1430, 1240, 1170, 1115, 1075, 825, 745, 705, 615 und 510 cm⁻¹.

### Beispiel 2:

### 7-[(1R,2S,5R)-2-(4-Toluolsulfonylamino)-5-fluor-cyclopentyl]-5(Z)-heptensäure:

Die Lösung von 16 mg (44 »mol) der nach Beispiel 1 dargestellten Verbindung in 1 ml Methanol versetzt man mit 0,5 ml einer 5%igen Lithiumhydroxidlösung und rührt 1,5 Stunden bei 23°C. Durch Zugabe von gesättigter Zitronensäure wird angesäuert, mit Wasser verdünnt und mehrfach mit Dichlormethan extrahiert. Man wäscht mit Wasser, gesättigter Natriumchloridlösung und trocknet über Magnesiumsulfat. Den nach Filtration und Lösungsmittelabzug erhaltenen Rückstand reinigt man durch Chromatographie an einer analytischen Dünnschichtplatte. Als Laufmittel dient ein Gemisch aus Dichlormethan und Methanol, als Elutionsmittel ein Gemisch aus Chloroform und Isopropanol. Isoliert werden 15,7 mg (41 »mol, 93%) der Titelverbindung als farblosen Feststoff.
IR (KBr): 3600-2400, 3430, 3310, 3050, 3020, 2950, 2930, 2860, 1715, 1600, 1450, 1320, 1240, 1160, 1095, 920, 815, 670, 580 und 550cm⁻¹.

### Beispiel 3:

### 7-[(1R,2S,5R)-2-(Benzolsulfonylamino)-5-fluor-cyclopentyl]-5(Z)-heptensäuremethylester:

80 mg (210 »mol) der nach Beispiel 3a dargestellten Verbindung setzt man in Analogie zu Beispiel 1 um und isoliert nach Aufarbeitung und Reinigung 23 mg (63 »mol, 30%) 7-[5(S)-(Benzolsulfonylamino)-cyclopent-1-enyl]-5(Z)-heptensäuremethylester sowie 26 mg (68 »mol, 32%) der Titelverbindung als farbloses Öl.
IR (Film): 3430, 3310, 3040, 3020, 2950, 2860, 1730, 1600, 1450, 1320, 1240, 1160, 1095, 930, 755, 720 und 690 cm⁻¹.

### Beispiel 3a:

### 7-[(1R,2S,5S)-2-Benzolsulfonylamino-5-hydroxy-cyclopentyl]-5(Z)-heptensäuremethylester:

270 mg (556 »mol) der nach Beispiel 3b dargestellten Verbindung setzt man in Analogie zu Beispiel 1a um und isoliert nach Aufarbeitung und Reinigung 212 mg (556 »mol, 100%) der Titelverbindung als farbloses Öl.
IR (Film): 3600-3200, 3260, 3060, 2930, 2850, 1725, 1445, 1320, 1265, 1160, 1090, 1020 und 735 cm⁻¹.

### Beispiel 3b:

### 7-[(1R,2S,5S)-2-Benzolsulfonylamino-5-benzoyloxy-cyclopentyl]-5(Z)-heptensäuremethylester:

250 mg (724 »mol) der nach Beispiel 1c dargestellten Verbindung setzt man in Analogie zu Beispiel 1b mit Benzolsulfonsäurechlorid um und isoliert nach Aufarbeitung und Reinigung 270 mg (556 »mol, 77%) der Titelverbindung als farbloses Öl.
IR (Film): 3270, 3060, 3000, 2950, 2860, 1730, 1715, 1600, 1585, 1450, 1330, 1315, 1270, 1160, 1110, 990, 1025, 910, 755, 715 und 690 cm⁻¹.

### Beispiel 4:

### 7-[(1R,2S,5R)-2-(Benzolsulfonylamino)-5-fluor-cyclopentyl]-5(Z)-heptensäure:

26 mg (68 »mol) der nach Beispiel 3 dargestellten Verbindung setzt man in Analogie zu Beispiel 2 um und isoliert nach Aufarbeitung und Reinigung 23,6 mg (64 »mol, 94%) der Titelverbindung als farbloses Öl.
¹H-NMR (CDCl₃): δ= 1,5-2,2(m, 11H), 2,34(t, 2H), 3,3-3,45(m, 1H), 4,7(m, 1H), 5,1(s, NH), 5,2-5,5(m, 2H), 7,45-7,65(m, 3H), 7,9(m, 2H).

### Beispiel 5:

### 7-[(1R,2S,5R)-2-(4-Fluorbenzolsulfonylamino)-5-fluor-cyclopentyl]-5(Z)-heptensäuremethylester:

84 mg (210 »mol) der nach Beispiel 5a dargestellten Verbindung setzt man in Analogie zu Beispiel 1 um und isoliert nach Aufarbeitung und Reinigung 25 mg (66 »mol, 31%) 7-[5(S)-(4-Fluorbenzylsulfonylamino)-cyclopent-1-enyl]-5(Z)-heptensäuremethylester sowie 26 mg (65 »mol, 31%) der Titelverbindung als farbloses Öl.
IR (Film): 3300, 3060, 3020, 2940, 2860, 1735, 1605, 1595, 1490, 1440, 1325, 1250, 1160, 835, 790, 735 und 700 cm⁻¹.

### Beispiel 5a:

### 7-[(1R,2S,5S)-2-(4-Fluorbenzolsulfonylamino)-5-hydroxy-cyclopentyl]-5(Z)-heptensäuremethylester:

240 mg (477 »mol) der nach Beispiel 5b dargestellten Verbindung setzt man in Analogie zu Beispiel 1b um und isoliert nach Aufarbeitung und Reinigung 175 mg (438 »mol, 92%) der Titelverbindung als farbloses Öl.
IR (Film): 3600-3200, 3310, 3060, 3020, 2940, 2860, 1730, 1610, 1595, 1490, 1440, 1325, 1250, 1160, 835, 790, 740 und 700 cm⁻¹.

### Beispiel 5b:

### 7-[(1R,2S,5S)-2-(4-Fluorbenzolsulfonylamino)-5-benzoyloxy-cyclopentyl]-5(Z)-heptensäuremethylester:

250 mg (724 »mol) der nach Beispiel 1c dargestellten Verbindung setzt man in Analogie zu Beispiel 1b mit 4-Fluorbenzolsulfonsäurechlorid um und isoliert nach Aufarbeitung und Reinigung 243 mg (483 »mol, 67%) der Titelverbindung als farbloses Öl.
IR (Film): 3270, 3060, 3000, 2950, 2870, 1730, 1710, 1590, 1570, 1490, 1450, 1335, 1270, 1165, 1150, 1090, 1025, 910, 715 und 670 cm⁻¹.

### Beispiel 6:

### 7-[(1R,2S,5R)-2-(4-Fluorbenzolsulfonylamino)-5-fluor-cyclopentyl]-5(Z)-heptensäure:

26 mg (65 »mol) der nach Beispiel 5 dargestellten Verbindung setzt man in Analogie zu Beispiel 2 um und isoliert nach Aufarbeitung und Reinigung 22,5 mg (58 »mol, 89%) der Titelverbindung als farbloses Öl.
¹H-NMR (CDCl₃): δ= 1,5-2,2(m, 11H), 2,35(t, 2H), 3,3-3,45(m, 1H), 4,7(m, 1H), 5,15(s, NH), 5,25-5,5(m, 2H), 7,1-7,3(m, 2H), 7,9(m, 2H).

### Beispiel 7:

### 7-[(1R,2S,5R)-2-(Chinon-8-ylsulfonylamino)-5-fluor-cyclopentyl]-5(Z)-heptensäuremethylester:

74 mg (171 »mol) der nach Beispiel 7a dargestellten Verbindung setzt man in Analogie zu Beispiel 1 um und isoliert nach Aufarbeitung und Reinigung 29 mg (70 »mol, 41%) 7-[5(S)-(Chinon-8-ylsulfonylamino)-cyclopent-1-enyl]-5(Z)-heptensäuremethylester sowie 19 mg (44 »mol, 25%) der Titelverbindung als farbloses Öl.
IR (Film): 3270, 3060, 2950, 2860, 1730, 1610, 1595, 1365, 1490, 1435, 1330, 1265, 1160, 1145, 835, 790, 735 und 700 cm⁻¹.

### Beispiel 7a:

### 7-[(1R,2S,5S)-2-(Chinon-8-ylsulfonylamino)-5-hydroxy-cyclopentyl]-5(Z)-heptensäuremethylester:

339 mg (632 »mol) der nach Beispiel 7b dargestellten Verbindung setzt man in Analogie zu Beispiel 1a um und isoliert nach Aufarbeitung und Reinigung 259 mg (596 »mol, 94%) der Titelverbindung als farbloses Öl.
IR (Film): 3600-3200, 3280, 3060, 3000, 2940, 2860, 1730, 1665, 1610, 1595, 1565, 1490, 1435, 1325, 1215, 1165, 1145, 1090, 835 und 790 cm⁻¹.

### Beispiel 7b:

### 7-[(IR,2S,5S)-2-(Chinon-8-ylsulfonylamino)-5-benzoyloxy-cyclopentyl]-5(Z)-heptensäuremethylester:

250 mg (724 »mol) der nach Beispiel 1c dargestellten Verbindung setzt man in Analogie zu Beispiel 1b mit Chinon-8-ylsulfonsäurechlorid um und isoliert nach Aufarbeitung und Reinigung 339 mg (632 »mol, 87%) der Titelverbindung als farbloses Öl.
IR (Film): 3270, 3060, 2950, 2860, 1730, 1710, 1675, 1600, 1565, 1490, 1450, 1435, 1330, 1270, 1245, 1165, 1145, 1110, 1070, 1045, 1025, 900, 835, 790 und 715 cm⁻¹.

### Beispiel 8:

### 7-[(1R,2S,5R)-2-(Chinon-8-ylsulfonylamino)-5-fluor-cyclopentyl]-5(Z)-heptensäure:

19 mg (44 »mol) der nach Beispiel 7 dargestellten Verbindung setzt man in Analogie zu Beispiel 2 um und isoliert nach Aufarbeitung und Reinigung 14 mg (33 »mol, 77%) der Titelverbindung als farbloses Öl.
IR (Film): 3600-2400, 3290, 3060, 2950, 2860, 1710, 1610, 1590, 1360, 1490, 1435, 1330, 1265, 1160, 1145, 835, 790, 735 und 700 cm⁻¹.

### Beispiel 9:

### 7-[(1R,2S,5R)-2-(Benzolsulfonylamino)-5-fluor-cyclopentyl]-heptansäuremethylester:

109 mg (284 »mol) der nach Beispiel 18 dargestellten Verbindung setzt man in Analogie zu Beispiel 1 um und isoliert nach Aufarbeitung und Reinigung 42 mg (115 »mol, 40%) 7-[5(S)-(Benzolsulfonylamino)-cyclopent-1-enyl]-heptansäuremethylester sowie 34 mg (88 »mol, 31%) der Titelverbindung als farbloses Öl.
IR (Film): 3370, 3060, 2930, 2860, 1725, 1440, 1325, 1160, 1090, 1070, 930, 755, 720 und 690 cm⁻¹.

### Beispiel 10:

### 7-[(1R,2S,5R)-2-(Benzolsulfonylamino)-5-fluor-cyclopentyl]-heptansäure:

34 mg (88 »mol) der nach Beispiel 9 dargestellten Verbindung setzt man in Analogie zu Beispiel 2 um und isoliert nach Aufarbeitung und Reinigung 29 mg (78 »mol, 89%) der Titelverbindung als farbloses Öl.
¹H-NMR (CDCl₃): δ= 1,0-1,35(m, 7H), 1,5-2,05(m, 8H), 2,35(t, 2H), 3,3-3,45(m, 1H), 4,7(m, 1H), 4,93(d, NH), 7,45-7,65(m, 3H), 7,88(m, 2H).

### Beispiel 11:

### 7-[(1R,2S,5R)-2-(4-Fluorbenzolsulfonylamino)-5-fluor-cyclopentyl]-heptansäuremethylester:

120 mg (299 »mol) der nach Beispiel 19 dargestellten Verbindung setzt man in Analogie zu Beispiel 1 um und isoliert nach Aufarbeitung und Reinigung 44 mg (115 »mol, 38%) 7-[5(S)-(4-Fluorbenzylsulfonylamino)-cyclopent-1-enyl]-heptansäuremethylester sowie 33 mg (82 »mol, 27%) der Titelverbindung als farbloses Öl.
IR (Film): 3370, 3070, 2930, 2860, 1730, 1665, 1595, 1490, 1450, 1435, 1330, 1290, 1230, 1160, 1090, 1115, 950, 925, 840 und 820 cm⁻¹.

### Beispiel 12:

### 7-[(1R,2S,5R)-2-(4-Fluorbenzolsulfonylamino)-5-fluor-cyclopentyl]-heptansäure:

33 mg (82 »mol) der nach Beispiel 11 dargestellten Verbindung setzt man in Analogie zu Beispiel 2 um und isoliert nach Aufarbeitung und Reinigung 31 mg (80 »mol, 97%) der Titelverbindung als farbloses Öl.
¹H-NMR (CDCl₃): δ= 1,05-1,35(m, 8H), 1,5-2,05(m, 7H), 2,35(t, 2H), 3,38(m, 1H), 4,7(m, 1H), 4,95(d, NH), 7,2(t, 2H), 7,9(m, 2H).

### Beispiel 13:

### 7-[(1R,2S,5R)-2-(Chinon-8-ylsulfonylamino)-5-fluor-cyclopentyl]-heptansäuremethylester:

78 mg (179 »mol) der nach Beispiel 20 dargestellten Verbindung setzt man in Analogie zu Beispiel 1 um und isoliert nach Aufarbeitung und Reinigung 24 mg (57 »mol, 32%) 7-[5(S)-(Chinon-8-ylsulfonylamino)-cyclopent-1-enyl]-heptansäuremethylester sowie 19 mg (43 »mol, 24%) der Titelverbindung als farbloses Öl.
IR (Film): 3270, 3060, 2940, 2850, 1730, 1610, 1595, 1565, 1490, 1435, 1330, 1160, 1140, 835, 795, 735 und 680 cm⁻¹.

### Beispiel 14:

### 7-[(1R,2S,5R)-2-(Chinon-8-ylsulfonylamino)-5-fluor-cyclopentyl]-heptansäure:

32 mg (73 »mol) der nach Beispiel 13 dargestellten Verbindung setzt man in Analogie zu Beispiel 2 um und isoliert nach Aufarbeitung und Reinigung 26 mg (62 »mol, 84%) der Titelverbindung als farbloses Öl.
IR (Film): 3600-2500, 3400, 3280, 2920, 2850, 1705, 1600, 1510, 1460, 1290, 1085, 1055, 1030 und 735 cm⁻¹.

### Beispiel 15:

### 7-[(1R,2S,5R)-2-(4-Toluolsulfonylamino)-5-hydroxy-cyclopentyl]-5(Z)-heptensäuremethylester:

Die Lösung von 241 mg (612 »mol) der nach Beispiel 15a dargestellten Verbindung löst man in 8 ml wasserfreiem Methanol, kühlt unter einer Atmosphäre aus trockenem Aregon auf -30°C und versetzt portionsweise mit insgesamt 69 mg Natriumborhydrid. Man laßt noch 30 Minuten reagieren, zersetzt überschüssiges Reduktionsmittel durch Zugabe von 120 »l Essigsäure, versetzt mit Wasser und extrahiert mehrfach mit Dichlomethan. Man wäscht mit Wasser, gesättigter Natriumchloridlösung und trocknet über Magnesiumsulfat. Den nach Filtration und Lösungsmittelabzug erhaltenen Rückstand reinigt man durch Chromatographie an 12 analytischen Dünnschichtplatten. Als Laufmittel dient ein Gemisch aus n-Hexan und Ethylacetat, als Elutionsmittel Diethylether. Isoliert werden 108 mg (273 »mol, 45%) der Titelverbindung als polarere Komponente, sowie 48 mg (121 »mol, 20%) der Titelverbindung aus Beispiel 1a als unpolarere Komponente.
IR (Film): 3600-3200, 2950, 2920, 2850, 1730, 1600, 1450, 1325, 1155, 1090, 895, 815, 735 und 670 cm⁻¹.

### Beispiel 15a:

### 7-[(1R,2S)-2-(4-Toluolsulfonylamino)-5-oxo-cyclopentyl]-5(Z)-heptensäuremethylester:

245 mg (619 »mol) der nach Beispiel 1a dargestellten Verbindung oxidiert man in Analogie zu Beispiel 1f und isoliert nach Aufarbeitung 241 mg (612 »mol, 99%) der Titelverbindung als blass gelbes Öl.
IR (Film): 3360, 2950, 2920, 2850, 1735, 1600, 1450, 1325, 1155, 1090, 900, 815, 735 und 665 cm⁻¹.

### Beispiel 16:

### 7-[(1R,2S,5S)-2-(4-Toluolsulfonylamino)-5-fluor-cyclopentyl]-5(Z)-heptensäuremethylester:

108 mg (273 »mol) der nach Beispiel 15 dargestellten Verbindung setzt man in Analogie zu Beispiel 1 um und isoliert nach Aufarbeitung und Reinigung 9 mg (24 »mol, 9%) 7-[5(S)-(4-Toluolsulfonylamino)-cyclopent-1-enyl]-5(Z)-heptensäuremethylester sowie 23 mg (60 »mol, 22%) der Titelverbindung jeweils als farbloses Öl.
IR (Film): 3250, 3010, 2960, 2930, 2870, 1730, 1600, 1450, 1380, 1325, 1305, 1290, 1240, 1160, 1095, 925, 910, 815 und 665 cm⁻¹.

### Beispiel 17:

### 7-[(1R,2S,5S)-2-(4-Toluolsulfonylamino)-5-fluor-cyclopentyl]-5(Z)-heptensäure:

30 mg (76 »mol) der nach Beispiel 16 dargestellten Verbindung verseifte man in Analogie zu Beispiel 2 und isolierte nach Aufarbeitung und Reinigung 14 mg (37 »mol, 48%) der Titelverbindung als farbloses Öl.
IR (Film): 3700-2400, 3260, 3010, 2960, 2930, 2870, 1710, 1600, 1450, 1405, 1380, 1325, 1305, 1290, 1240, 1160, 1095, 925, 910, 815, 665, 620 und 550 cm⁻¹.

### Beispiel 18:

### 7-[(1R,2S,5S)-2-(Benzolsulfonylamino)-5-hydroxy-cyclopentyl]-heptansäuremethylester:

132 mg (346 »mol) der nach Beispiel 3a dargestellten Verbindung löst man in 5 ml Ethylacetat, versetzt mit 50 mg Pd/C (5%ig) und hyriert bei 1 atm, bis die theoretisch berechnete Menge Wasserstoff aufgenommen ist. Man filtriert, wäscht nach und engt ein. Isoliert werden 109 mg (284 »mol, 82%) der Titelverbindung als farbloses Öl.
IR (Film): 3600-3200, 3260, 2930, 2850, 1725, 1445, 1320, 1265, 1160, 1095, 1020 und 735 cm⁻¹.

### Beispiel 19:

### 7-[(1R,2S,5S)-2-(4-Fluorbenzolsulfonylamino)-5-hydroxy-cyclopentyl]-heptensäuremethyl ester:

141 mg (355 »mol) der nach Beispiel 5a dargestellten Verbindung hydriert man in Analogie zu Beispiel 18 und isoliert nach Aufarbeitung 120 mg (300 »mol, 84%) der Titelverbindung als farbloses Öl.
IR (Film): 3270, 2930, 2850, 1730, 1660, 1600, 1445, 1320, 1260, 1160, 1095, 1020, 925, 840 und 820 cm⁻¹.

### Beispiel 20:

### 7-[(1R,2S,5S)-2-(Chinon-8-ylsulfonylamino)-5-hydroxy-cyclopentyl]-heptansäuremethylester:

149 mg (344 »mol) der nach Beispiel 7a dargestellten Verbindung hydriert man in Analogie zu Beispiel 18 und isoliert nach Aufarbeitung 142 mg (327 »mol, 95%) der Titelverbindung als farbloses Öl.
IR (Film): 3600-3200, 3520, 3410, 3280, 2940, 2850, 1725, 1665, 1595, 1490, 1455, 1430, 1320, 1160, 1140, 1020, 890, 840, 790, 735 und 675 cm⁻¹.

### Beispiel 21:

### 7-[(1R,2S,5S)-2-(Chinon-8-ylsulfonylamino)-5-hydroxy-cyclopentyl]-heptansäure:

30 mg (69 »mol) der nach Beispiel 20 dargestellten Verbindung verseift man in Analogie zu Beispiel 2 und isoliert nach Aufarbeitung und Reinigung 25 mg (59 »mol, 86%) der Titelverbindung als farbloses Öl.
¹H-NMR (CDCl₃): δ= 1,0-1,9(m, 16H), 2,32(t, 2H), 3,4(m, 1H), 4,12(m, 1H), 6,27(d, NH), 7,57(dt, 1H), 7,66(t, 1H), 8,06(dd, 1H), 8,3(dd, 1H), 8,43(dd, 1H), 9,03(dd, 1H).

### Beispiel 22:

### 7-[(1R,2S,5S)-2-(4-Toluolsulfonylamino)-5-hydroxy-cyclopentyl]-5(Z)-heptensäure:

47 mg (119 »mol) der nach Beispiel 1b dargestellten Verbindung verseift man in Analogie zu Beispiel 2 und isoliert nach Aufarbeitung und Reinigung 41 mg (107 »mol, 90%) der Titelverbindung als farbloses Öl.
IR (Film): 3600-2400, 3470, 3260, 3000, 2940, 2870, 1710, 1600, 1445, 1320, 1160, 1095, 910, 810, 670, 570 und 550 cm⁻¹.

### Beispiel 23:

### 7-[(1R,2S,5S)-2-(Chinon-8-ylsulfonylamino)-5-hydroxy-cyclopentyl]-5(Z)-heptensäure:

19 mg (44 »mol) der nach Beispiel 7b dargestellten Verbindung verseift man in Analogie zu Beispiel 2 und isoliert nach Aufarbeitung und Reinigung 16 mg (38 »mol, 85%) der Titelverbindung als wachsartigen Feststoff.
¹H-NMR (CDCl₃): δ= 1,0-2,35(m, 14H), 3,43(m, 1H), 4,1(m, 1H), 5,15-5,35(m, 2H), 6,47(d, NH), 7,52(dd, 1H), 7,66(t, 1H), 8,04(dd, 1H), 8,27(dd, 1H), 8,43(dd, 1H), 9,03(dd, 1H).

### Beispiel 24:

### 7-[(1R,2S,3RS,5R)-2-Benzyloxymethyl-3-phenyl-5-fluor-cyclopentyl]-5(E/Z)-heptensäure:

33 mg (99 »mol) der nach Beispiel 24a dargestellten Verbindung versetzt man mit 0,4 ml einer 50%igen KOH-Lösung, 257 »l Benzylchlorid, 4 mg Tetrabutylammoniumhydrogensulfat und rührt intensiv 18 Stunden bei 23°C. Man gießt in Eiswasser, extahiert mehrfach mit Dichlormethan, trocknet die organische Phase über Magnesiumsulfat und entfernt überschüssiges Benzylchlorid durch Chromatographie an 4 analytischen Dünnschichtplatten. Als Laufmittel dient ein Gemisch aus n-Hexan und Ethylacetat, als Elutionsmittel Chloroform. Den erhaltenen Rückstand verseift man in Analogie zu Beispiel 2 und isoliert nach Aufarbeitung und Reinigung 17 mg (41 »mol, 42%) der Titelverbindung als farbloses Öl.
IR (Film): 3600-2400, 3060, 3030, 2930, 2850, 1710, 1600, 1495, 1455, 1360, 1240, 1210, 1100, 970, 755, 735 und 700 cm⁻¹.

### Beispiel 24a:

### 7-[(1R,2S,3RS,5R)-2-Hydroxymethyl-3-phenyl-5-fluor-cyclopentyl]-5(E/Z)-heptensäuremethylester:

305 mg (532 »mol) der nach Beispiel 24b dargestellten Verbindung setzt man in Analogie zu Beispiel 1g um und isoliert nach Aufarbeitung und Reinigung 176 mg (526 »mol, 99%) der Titelverbindung als farbloses Öl.
IR (Film): 3600-3200, 3030, 2920, 2850, 1730, 1450, 1435, 1370, 1150, 1045, 755 und 700 cm⁻¹.

### Beispiel 24b:

### 7-[(1R,2S,3RS,5R)-2-(tert.-Butyldiphenylsilyloxymethyl)-3-phenyl-5-fluor-cyclopentyl]-5(E/Z)-heptensäuremethylester:

742 mg (1,30 mmol) der nach Beispiel 24c dargestellten Verbindung setzt man in Analogie zu Beispiel 1 um und isoliert nach Aufarbeitung und Reinigung 294 mg (532 »mol, 41%) 7-[(4RS,5S)-4-Phenyl-5-(tert.-butyldiphenylsilyloxymethyl)-cyclopent-1-enyl]-5(E/Z)-heptensäuremethylester sowie 337 mg (588 »mol, 45%) der Titelverbindung als farbloses Öl.
IR (Film): 3070, 3030, 2960, 2930, 2860, 1735, 1600, 1590, 1425, 1110, 820, 740 und 700 cm⁻¹.

### Beispiel 24c:

### 7-[(1R,2S,3RS,5S)-2-(tert.-Butyldiphenylsilyloxymethyl)-3-phenyl-5-hydroxy-cyclopentyl] -5(E/Z)-heptensäuremethylester (A) und 7-[(1R,2S,3RS,5R)-2-(tert.-Butyldiphenylsilyloxymethyl)-3-phenyl-5-hydroxy-cyclopentyl]-5(E/Z)-heptensäuremethylester (B):

1,97 g (3,47 mmol) der nach Beispiel 24d dargestellten Verbindung setzt man in Analogie zu Beispiel 15 um und isoliert nach Aufarbeitung und Reinigung 742 mg (1,30 mmol, 37%) der Titelverbindung A sowie 925 mg (1,62 mmol, 47%) der Titelverbindung B jeweils als farbloses Öl.
IR (Film) von A: 3600-3200, 3070, 3020, 2950, 2920, 2850, 1735, 1600, 1585, 1450, 1425, 1110, 820, 760, 740 und 700 cm⁻¹.
IR (Film) von B: 3600-3200, 3060, 3020, 2950, 2920, 2850, 1730, 1600, 1590, 1450, 1425, 1265, 1110, 1060, 820, 740 und 700 cm⁻¹.

### Beispiel 24d:

### 7-[(1R,2S,3RS)-2-(tert.-Butyldiphenylsilyloxymethyl)-3-phenyl-5-oxo-cyclopentyl]-5(E/Z)-heptensäuremethylester:

Die Lösung von 11,82 g (24,0 mmol) der nach Beispiel 24e dargestellten Verbindung in 120 ml wasserfreiem Tetrahydrofuran versetzt man mit 500 mg Cupfer(II)acetat, und kühlt unter einer Atmosphäre aus trockenem Argon auf -78°C. Man gibt 3,04 ml Trimethylchlorsilan und anschließend 12,8 ml einer 3M Lösung von Phenylmagnesiumbromid in Diethylether zu. Nach 45 Minuten gießt man in gesättigte Ammoniumchloridlösung und extrahiert mehrfach mit Dichlormethan. Die vereinigten organischen Extrakte trocknet man über Magnesiumsulfat und reinigt den nach Lösungsmittelabzug erhaltenen Rückstand durch Chromatographie an ca.200 ml feinem Kieselgel. Als Laufmittel dient ein Gradientensystem aus n-Hexan und Ethylacetat. Isoliert werden 9,78 g (17,2 mmol, 72%) der Titelverbindung als farbloses Öl.
IR (Film): 3070, 3030, 2950, 2930, 2850, 1735, 1600, 1465, 1450, 1425, 1110, 1055, 820, 780, 740 und 700 cm⁻¹.

### Beispiel 24e:

### 7-[(1R,2S)-2-(tert.-Butyldiphenylsilyloxymethyl)-5-oxo-cyclopent-3-enyl]-5(E/Z)-heptensäuremethylester:

Die Lösung von 5,84 g (11,5 mmol) der nach Beispiel 24f dargestellten Verbindung in 52 ml wasserfreiem Pyridin kühlt man unter einer Atmosphäre aus trockenem Argon auf 3°C, tropft 2,17 ml Methansulfonsäurechlorid zu und rührt noch 2 Stunden bei 3°C. Man versetzt mit Eis, verdünnt mit Wasser und extrahiert mehrfach mit Dichlormethan. Die vereinigten organischen Extrakte trocknet man über Magnesiumsulfat und reinigt den nach Lösungsmittelabzug erhaltenen Rückstand durch Chromatographie an ca. 200 ml feinen Kieselgel. Als Laufmittel dient ein Gradientensystem aus n-Hexan und Ethylacetat. Isoliert werden 4,93 g (10,0 mmol, 87%) der Titelverbindung als farbloses Öl.
IR (Film): 3070, 3040, 2950, 2930, 2850, 1735, 1705, 1585, 1425, 1110, 820, 740 und 700 cm⁻¹.

### Beispiel 24f:

### 7-[(1R,2S,3R)-2-(tert.-Butyldiphenylsilyloxymethyl)-3-(tetrahydropyran-2-yloxy)-5-oxo-cyclopentyl]-5(E/Z)-heptensäuremethylester:

11,9 g (20,0 mmol) der nach Beispiel 24g dargestellten Verbindung setzt man in Analogie zu Beispiel 1f um und isoliert nach Aufarbeitung und Reinigung 9,39 g (15,8 mmol, 79%) der Titelverbindung als farbloses Öl.
IR (Film): 3070, 3040, 2940, 2850, 1740, 1590, 1425, 1110, 1075, 1030, 970, 820, 740 und 700 cm⁻¹.

### Beispiel 24g:

### 7-[(1R,2S,3R,5S)-2-(tert.-Butyldiphenylsilyloxymethyl)-3-(tetrahydropyran-2-yloxy)-5-hydroxy-cyclopentyl]-5(E/Z)-heptensäuremethylester:

22,4 g (47,8 mmol) (1S,3RS,5R,6S,7R)-7-(Tetrahydropyran-2-yloxy)-6-(tert.-butyldiphenylsilyloxymethyl)-2-oxabicyclo[3.3.0]octan-3-ol setzt man in Analogie zu Beispiel 1j unter Verwendung von KOH-haltigem Kalium-tert.-butanolat um und isoliert nach Aufarbeitung und Veresterung mit einer etherischen Lösung von Diazomethan nach chromatographischer Reinigung an ca. 1,3 l feinem Kieselgel mit einem Gradientensystem aus n-Hexan und Ethylacetat 21,6 g (36,3 mmol, 76%) der Titelverbindung als farbloses Öl.
IR (Film): 3600-3200, 3070, 3040, 2940, 2850, 1730, 1595, 1425, 1110, 1075, 1025, 970, 820, 740 und 700 cm⁻¹.

### Beispiel 25:

### 7-[(1R,2S,3RS,5R)-2-(4-Cyanobenzyloxymethyl)-3-phenyl-5-fluor-cyclopentyl]-5(E/Z)-heptensäure:

28 mg (84 »mol) der nach Beispiel 24a dargestellten Verbindung setzt man in Analogie zu Beispiel 24 unter Verwendung von 4-Cyanobenzylbromid um und isoliert nach Aufarbeitung und Reinigung 14 mg (32 »mol, 38%) der Titelverbindung als farbloses Öl.
IR (Film): 3600-2400, 3060, 3015, 1930, 2860, 2230, 1705, 1600, 1455, 1435, 1360, 1290, 1240, 1150, 1110, 1090, 970, 795, 755, 700 und 685 cm⁻¹.

### Beispiel 26:

### 7-[(1R,2S,3RS,5R)-2-(3-Methylbenzyloxymethyl)-3-phenyl-5-fluor-cyclopentyl]-5(E/Z)-heptensäure:

27 mg (81 »mol) der nach Beispiel 24a dargestellten Verbindung setzt man in Analogie zu Beispiel 24 unter Verwendung von 3-Methylbenzylbromid um und isoliert nach Aufarbeitung und Reinigung 23 mg (54 »mol, 67%) der Titelverbindung als farbloses Öl.
IR (Film): 3600-2400, 3060, 3030, 2930, 2860, 1705, 1600, 1450, 1360, 1245, 1160, 1105, 1090, 970, 780, 755 und 700 cm⁻¹.

### Beispiel 27:

### 7-[(1R,2S,3RS,5R)-2-(3,5-Bis-trifluormethylbenzyloxymethyl)-3-phenyl-5-fluor-cyclopentyl]-5(E/Z)-heptensäure:

29 mg (87 »mol) der nach Beispiel 24a dargestellten Verbindung setzt man in Analogie zu Beispiel 24 unter Verwendung von 3,5-Bis(trifluormethyl)benzylbromid um und isoliert nach Aufarbeitung und Reinigung 28 mg (51 »mol, 59%) der Titelverbindung als farbloses Öl.
IR (Film): 3600-2400, 3060, 3030, 2930, 2870, 1710, 1625, 1600, 1455, 1380, 1355, 1280, 1175, 1135, 970, 885, 840, 760, 700 und 680 cm⁻¹.

### Beispiel 28:

### 7-[(1R,2S,3RS,5R)-2-(1-Naphthylmethoxymethyl)-3-phenyl-5-fluor-cyclopentyl]-5(E/Z)-heptensäure:

29 mg (87 »mol) der nach Beispiel 24a dargestellten Verbindung setzt man in Analogie zu Beispiel 24 unter Verwendung von 1-Brommethylnaphtalin um und isoliert nach Aufarbeitung und Reinigung 26 mg (56 »mol, 65%) der Titelverbindung als farbloses Öl.
IR (Film): 3600-2400, 3060, 3030, 3010, 2930, 2860, 1705, 1600, 1455, 1240, 1165, 1100, 970, 800, 790, 775, 755 und 700 cm⁻¹.

### Beispiel 29:

### 7-[(1S,2R,3RS,5S)-2-[2-(4-Fluorphenoxy)-ethoxymethyl]-3-phenyl-5-fluor-cyclopentyl]-5-(Z)-heptensäure:

43 mg (129 »mol) der nach Beispiel 29a dargestellten Verbindung setzt man in Analogie zu Beispiel 24 unter Verwendung von 1-Brom-2-(4-fluorphenoxy)-ethan um und isoliert nach Aufarbeitung und Reinigung 12 mg (26 »mol, 20%) der Titelverbindung als farbloses Öl.
IR (Film): 3600-2400, 3060, 3030, 3010, 2930, 2870, 1710, 1600, 1505, 1455, 1250, 1210, 1130, 100, 1050, 830, 760, 745 und 700 cm⁻¹.

### Beispiel 29a:

### 7-[(1S,2R,3RS,5S)-2-hydroxymethyl-3-phenyl-5-fluor-cyclopentyl]-5-(Z)-heptensäure:

2,36 g (4,12 mmol) der nach Beispiel 29b dargestellten Verbindung setzt man in Analogie zu Beispiel 1g um und isoliert nach Aufarbeitung und Reinigung 1,24 g (3,71 mmol, 90%) der Titelverbindung als farbloses Öl.
IR (Film): 3600-3200, 3020, 2920, 2850, 1730, 1600, 1450, 1435, 1330, 1150, 1045, 965, 755 und 700 cm⁻¹.

### Beispiel 29b:

### 7-[(1S,2R,3RS,5S)-2-(tert.-Butyldiphenylsilyloxymethyl)-3-phenyl-5-fluor-cyclopentyl]-5-(Z)-heptensäure:

Ausgehend von (1R,3RS,5S,6R,7S)-7-(Tetrahydropyran-2-yloxy)-6-(tert.-butyldiphenylsilyloxymethyl)-2-oxabicyclo[3.3.0]octan-3-ol stellt man in Analogie zu den beschriebenen Beispielen 24b bis 24g 7-[(4RS,5R)-4-Phenyl-5-(tert.-butyldiphenylsilyloxymethyl)-cyclopent-1-enyl]-5(Z)-heptensäuremethylester sowie die Titelverbindung her. Durch Verwendung von frisch sublimiertem Kalium-tert.-butanolat (vgl. Beispiel 24g) wird bezüglich der Doppelbindung in 5 jeweils nur das Z-Isomere erhalten.

### Beispiel 30:

### 7-[(1S,2R,3RS,5S)-2-Benzyloxymethyl-3-phenyl-5-fluor-cyclo-pentyl]-5(Z)-heptensäure:

46 mg (129 »mol) der nach Beispiel 29a dargestellten Verbindung setzt man in Analogie zu Beispiel 24 um und isoliert nach Aufarbeitung und Reinigung 25 mg (61 »mol, 47%) der Titelverbindung als farbloses Öl.
IR (Film): 3600-2400, 3060, 3030, 3010, 2930, 2860, 1710, 1600, 1495, 1455, 1360, 1240, 1210, 1100, 755, 735 und 700 cm⁻¹.

### Beispiel 31:

### 7-[(1S,2R,3RS,5S)-2-(4-Cyanobenzyloxymethyl)-3-phenyl-5-fluor-cyclo-pentyl]-5(Z)-heptensäure:

43 mg (129 »mol) der nach Beispiel 29a dargestellten Verbindung setzt man in Analogie zu Beispiel 24 unter Verwendung von 4-Cyanobenzylbromid um und isoliert nach Aufarbeitung und Reinigung 31 mg (71 »mol, 55%) der Titelverbindung als farbloses Öl.
IR (Film): 3600-2400, 3060, 3030, 2940, 2860, 2230, 1710, 1610, 1450, 1235, 1220, 1130, 1100, 820, 760 und 700 cm⁻¹.

### Beispiel 32:

### 7-[(1S,2R,3RS,5S)-2-(3-Methylbenzyloxymethyl)-3-phenyl-5-fluor-cyclo-pentyl]-5(Z)-heptensäure:

43 mg (129 »mol) der nach Beispiel 29a dargestellten Verbindung setzt man in Analogie zu Beispiel 24 unter Verwendung von 3-Methylbenzylbromid um und isoliert nach Aufarbeitung und Reinigung 26 mg (61 »mol, 47%) der Titelverbindung als farbloses Öl.
IR (Film): 3600-2400, 3060, 3030, 3010, 2940, 2860, 1710, 1600, 1495, 1455, 1360, 1250, 1160, 1105, 1090, 780, 760 und 700 cm⁻¹.

### Beispiel 33:

### 7-[(1S,2R,3RS,5S)-2-(3-Cyanobenzyloxymethyl)-3-phenyl-5-fluor-cyclo-pentyl]-5(Z)-heptensäure:

43 mg (129 »mol) der nach Beispiel 29a dargestellten Verbindung setzt man in Analogie zu Beispiel 24 unter Verwendung von 3-Cyanobenzylbromid um und isoliert nach Aufarbeitung und Reinigung 36 mg (83 »mol, 64%) der Titelverbindung als farbloses Öl.
IR (Film): 3600-2400, 3060, 3030, 3010, 2930, 2860, 2230, 1710, 1600, 1495, 1455, 1435, 1360, 1240, 1150, 1105, 1090, 795, 760, 700 und 685 cm⁻¹.

### Beispiel 34:

### 7-[(1R,2S,3RS,5S)-2-[2-(4-Fluorphenoxy)-ethoxymethyl]-3-phenyl-5-fluor-cyclopentyl]-5-(E/Z)-heptensäure:

33 mg (99 »mol) der nach Beispiel 34b dargestellten Verbindung setzt man in Analogie zu Beispiel 24 unter Verwendung von 1-Brom-2-(4-fluorphenoxy)-ethan um und isoliert nach Aufarbeitung und Reinigung 17 mg (37 »mol, 37%) der Titelverbindung als farbloses Öl.
IR (Film): 3600-2400, 3060, 3030, 3000, 2930, 2870, 2850, 1710, 1600, 1505, 1455, 1250, 1210, 1140, 995, 1050, 825, 760, 745 und 700 cm⁻¹.

### Beispiel 34a:

### 7-[(1R,2S,3RS,5S)-2-hydroxymethyl-3-phenyl-5-fluor-cyclopentyl]-5-(E/Z)-heptensäuremethylester:

586 mg (1,02 mmol) der nach Beispiel 34b dargestellten Verbindung setzt man in Analogie zu Beispiel 1g um und isoliert nach Aufarbeitung und Reinigung 335 mg (1,00 mmol, 98%) der Titelverbindung als farbloses Öl.
IR (Film): 3600-3200, 3060, 3030, 3000, 2940, 2870, 1735, 1600, 1490, 1450, 1435, 1245, 1175, 1065, 1045, 1030, 945, 865, 760 und 700 cm⁻¹.

### Beispiel 34b:

### 7-[(1R,2S,3RS,5S)-2-(tert.-Butyldiphenylsilyloxymethyl)-3-phenyl-5-fluor-cyclopentyl]-5-(E/Z)-heptensäuremethylester:

925 mg (1,62 mmol) der nach Beispiel 24c dargestellten polareren Verbindung B setzt man in Analogie zu Beispiel 1 um und isoliert nach Aufarbeitung und Reinigung 98 mg (165 »mol, 10%) 7-[4RS,5S)-4-Phenyl-5-(tert.-butyldiphenylsilyloxymethyl)-cyclopent-1-enyl]-5(E/Z)-heptensäuremethylester sowie 586 mg (1,04 mmol, 64%) der Titelverbindung als farbloses Öl.
IR (Film): 3070, 3020, 3000, 2950, 2930, 2850, 1735, 1600, 1585, 1490, 1465, 1450, 1425, 1360, 1110, 1005, 965, 870, 820, 760, 740 und 700 cm⁻¹.

### Beispiel 35:

### 7-[(1R,2S,3RS,5S)-2-Benzyloxymethyl-3-phenyl-5-fluor-cyclopentyl]-5(E/Z)-heptensäure:

31 mg (93 »mol) der nach Beispiel 34a dargestellten Verbindung setzt man in Analogie zu Beispiel 24 unter Verwendung von Benzylchlorid um und isoliert nach Aufarbeitung und Reinigung 14 mg (34 »mol, 37%) der Titelverbindung als farbloses Öl.
IR (Film): 3600-2400, 3060, 3020, 2930, 2850, 1705, 1600, 1490, 1465, 1100, 1070, 1030, 950, 760, 735 und 700 cm⁻¹.

### Beispiel 36:

### 7-[(1R,2S,3RS,5S)-2-(3,5-Bis-trifluormethylbenzyloxymethyl)-3-phenyl-5-fluor-cyclopentyl]-5(E/Z)-heptensäure:

25 mg (75 »mol) der nach Beispiel 34a dargestellten Verbindung setzt man in Analogie zu Beispiel 24 unter Verwendung von 3,5-Bis-(trifluormethyl)-benzylbromid um und isoliert nach Aufarbeitung und Reinigung 27 mg (49 »mol, 66%) der Titelverbindung als farbloses Öl.
IR (Film): 3600-2400, 3070, 3030, 2940, 2860, 1710, 1610, 1600, 1360, 1280, 1180, 1140, 700 und 680 cm⁻¹.

### Beispiel 37:

### 7-[(1R,2S,3RS,5S)-2-(1-Naphthylmethoxymethyl)-3-phenyl-5-fluor-cyclopentyl]-5(E/Z)-heptensäure:

23 mg (69 »mol) der nach Beispiel 34a dargestellten Verbindung setzt man in Analogie zu Beispiel 24 unter Verwendung von 1-Brommethylnaphtalin um und isoliert nach Aufarbeitung und Reinigung 13 mg (28 »mol, 41%) der Titelverbindung als farbloses Öl.
IR (Film): 3600-2400, 3060, 3020, 3000, 2930, 2850, 1710, 1600, 1505, 1455, 1250, 1220, 1100, 950, 800, 790, 780, 760 und 700 cm⁻¹.

### Beispiel 38:

### 7-[(1R,2S,3RS,5S)-2-(4-Cyanobenzyloxymethyl)-3-phenyl-5-fluor-cyclopentyl]-5(E/Z)-heptensäure:

20 mg (60 »mol) der nach Beispiel 34a dargestellten Verbindung setzt man in Analogie zu Beispiel 24 unter Verwendung von 4-Cyanobenzylbromid um und isoliert nach Aufarbeitung und Reinigung 10 mg (24 »mol, 44%) der Titelverbindung als farbloses Öl.
IR (Film): 3600-2400, 3060, 3030, 2930, 2850, 2220, 1710, 1605, 1505, 1495, 1455, 1415, 1130, 1100, 950, 820, 760 und 700 cm⁻¹.

### Beispiel 39:

### 7-[(1R,2S,3RS,5S)-2-(3-Methylbenzyloxymethyl)-3-phenyl-5-fluor-cyclopentyl]-5(E/Z)-heptensäure:

20 mg (60 »mol) der nach Beispiel 34a dargestellten Verbindung setzt man in Analogie zu Beispiel 24 unter Verwendung von 3-Methylbenzylbromid um und isoliert nach Aufarbeitung und Reinigung 16 mg (38 »mol, 63%) der Titelverbindung als farbloses Öl.
IR (Film): 3600-2400, 3060, 3030, 2930, 2850, 1710, 1600, 1495, 1455, 1435, 1240, 1155, 1100, 1090, 950, 760 und 700 cm⁻¹.

### Beispiel 40:

### 7-[(1R,2S,3RS,5S)-2-(3-Cyanobenzyloxymethyl)-3-phenyl-5-fluor-cyclopentyl]-5(E/Z)-heptensäure:

20 mg (60 »mol) der nach Beispiel 34a dargestellten Verbindung setzt man in Analogie zu Beispiel 24 unter Verwendung von 3-Cyanobenzylbromid um und isoliert nach Aufarbeitung und Reinigung 17 mg (39 »mol, 65%) der Titelverbindung als farbloses Öl.
IR (Film): 3600-2400, 3060, 3030, 2930, 2860, 2230, 1710, 1600, 1585, 1455, 1435, 1355, 1285, 1200, 1150, 1105, 1085, 795, 760, 700 und 685 cm⁻¹.

### Beispiel 41:

### 7-[(1S,2R,3RS,5R)-2-[2-(4-Fluorphenoxy)-ethoxymethyl]-3-phenyl-5-fluor-cyclopentyl]-5-(Z)-heptensäure:

43 mg (128 »mol) der nach Beispiel 41a dargestellten Verbindung setzt man in Analogie zu Beispiel 24 unter Verwendung von 4-Fluorphenoxyethylbromid um und isoliert nach Aufarbeitung und Reinigung 12 mg (26 »mol, 20%) der Titelverbindung als farbloses Öl.
IR (Film): 3600-2400, 3070, 3030, 2930, 2870, 1710, 1600, 1505, 1455, 1250, 1210, 1140, 1100, 1055, 830, 760, 750 und 700 cm⁻¹.

### Beispiel 41a:

### 7-[(1S,2R,3RS,5R)-2-hydroxymethyl-3-phenyl-5-fluor-cyclopentyl ]-5-(Z)-heptensäure:

3,63 g (6,34 mmol) der nach Beispiel 41b dargestellten Verbindung setzt man in Analogie zu Beispiel 1g um und isoliert nach Aufarbeitung und Reinigung 2,05 g (2,04 mmol, 95%) der Titelverbindung als farbloses Öl.
IR (Film): 3600-3200, 3060, 3030, 3000, 2940, 2870, 1735, 1600, 1490, 1450, 1435, 1245, 1220, 1170, 1150, 1070, 1045, 945, 870, 760 und 700 cm⁻¹.

### Beispiel 41b:

### 7-[(1S,2R,3RS,5R)-2-(tert.-Butyldiphenylsilyloxymethyl)-3-phenyl-5-fluor-cyclopentyl]-5-(Z)-heptensäure:

6,15 g (10,8 mmol) 7-[(1S,2R,3RS,5S)-2-(tert.-Butyldiphenylsilyloxymethyl)-3-phenyl-5-hydroxy-cyclopentyl]-5(Z)-heptensäuremethylester der im Verlauf der unter Beispiel 29b beschriebenen Synthese in Analogie zu Beispiel 24c gewonnen wird, setzt man in Analogie zu Beispiel 1 um und isoliert nach Aufarbeitung und Reinigung 610 mg (1,1 mmol, 10%) 7-[(4RS,5R)-4-Phenyl-5-(tert.-butyldiphenylsilyloxymethyl)-cyclo-pent-1-enyl]-5(Z)-heptensäuremethylester sowie 3,63 g (6,34 mmol, 59%) der Titelverbindung als farbloses Öl.
IR (Film): 3070, 3020, 2950, 2930, 2850, 1735, 1600, 1585, 1425, 1110, 820, 760, 740 und 700 cm⁻¹.

### Beispiel 42:

### 7-[(1S,2R,3RS,5R)-2-Benzyloxymethyl-3-phenyl-5-fluor-cyclopentyl]-5(Z)-heptensäure:

43 mg (129 »mol) der nach Beispiel 41a dargestellten Verbindung setzt man in Analogie zu Beispiel 24 unter Verwendung von Benzylchlorid um und isoliert nach Aufarbeitung und Reinigung 23 mg (56 »mol, 43%) der Titelverbindung als farbloses Öl.
IR (Film): 3600-2400, 3060, 3030, 2940, 2860, 1710, 1600, 1495, 1450, 1100, 760, 740 und 700 cm⁻¹.

### Beispiel 43:

### 7-[(1S,2R,3RS,5R)-2-(4-Cyanobenzyloxymethyl)-3-phenyl-5-fluor-cyclopentyl]-5(Z)-heptensäure:

43 mg (129 »mol) der nach Beispiel 41a dargestellten Verbindung setzt man in Analogie zu Beispiel 24 unter Verwendung von 4-Cyanobenzylbromid um und isoliert nach Aufarbeitung und Reinigung 21 mg (48 »mol, 37%) der Titelverbindung als farbloses Öl.
IR (Film): 3600-2400, 3060, 3030, 2940, 2860, 2230, 1710, 1610, 1455, 1275, 1130, 1100, 820, 780 und 700 cm⁻¹.

### Beispiel 44:

### 7-[(1S,2R,3RS,5R)-2-(3-Methylbenzyloxymethyl)-3-phenyl-5-fluor-cyclopentyl]-5(Z)-heptensäure:

43 mg (129 »mol) der nach Beispiel 41a dargestellten Verbindung setzt man in Analogie zu Beispiel 24 unter Verwendung von 3-Methylbenzylbromid um und isoliert nach Aufarbeitung und Reinigung 21 mg (49 »mol, 38%) der Titelverbindung als farbloses Öl.
IR (Film): 3600-2400, 3060, 3030, 2930, 2850, 1710, 1600, 1455, 1240, 1160, 1100, 950, 780, 760 und 700 cm⁻¹.

### Beispiel 45:

### 7-[(1S,2R,3RS,5R)-2-(3-Cyanobenzyloxymethyl)-3-phenyl-5-fluor-cyclopentyl]-5(Z)-heptensäure:

43 mg (129 »mol) der nach Beispiel 41a dargestellten Verbindung setzt man in Analogie zu Beispiel 24 unter Verwendung von 3-Cyanobenzylbromid um und isoliert nach Aufarbeitung und Reinigung 41 mg (94 »mol, 73%) der Titelverbindung als farbloses Öl.
IR (Film): 3600-2400, 3060, 3030, 2940, 2860, 2230, 1710, 1600, 1580, 1450, 1430, 1360, 1240, 1150, 1110, 1090, 950, 795, 760, 700 und 690 cm⁻¹.

### Beispiel 46:

### 7-[(1R,2S,3RS,5S)-2-(2,4-Bis-trifluormethylbenzyloxymethyl)-3-phenyl-5-fluor-cyclopentyl]-5(E/Z)-heptensäure:

20 mg (60 »mol) der nach Beispiel 34a dargestellten Verbindung setzt man in Analogie zu Beispiel 24 unter Verwendung von 2,4-Bis-(trifluormethyl)-benzylbromid um und isoliert nach Aufarbeitung und Reinigung 20 mg (37 »mol, 61%) der Titelverbindung als farbloses Öl.
IR (Film): 3600-2400, 3030, 3000, 2920, 2850, 1710 1630, 1600, 1455, 1345, 1275, 1170, 1130, 1055, 910, 860, 840, 760 und 700 cm⁻¹.

### Beispiel 47:

### 7-[(1R,2S,3RS,5S)-2-(4-Methylbenzyloxymethyl)-3-pheny-5-fluor-cyclopentyl]-5(E/Z)-heptensäure:

20 mg (60 »mol) der nach Beispiel 34a dargestellten Verbindung setzt man in Analogie zu Beispiel 24 unter Verwendung von 4-Methylbenzylbromid um und isoliert nach Aufarbeitung und Reinigung 15 mg (35 »mol, 59%) der Titelverbindung als farbloses Öl.
IR (Film): 3600-2400, 3020, 3000, 2920, 2850, 1705, 1600, 1455, 1100, 1085, 1070, 950, 800, 760 und 700 cm⁻¹.

### Beispiel 48:

### 7-[(1R,2S,3RS,5S)-2-(2-Naphthylmethoxymethyl)-3-phenyl-5-fluor-cyclopentyl]-5(Z)-heptensäure (A) und 7-[(1R,2S,3RS,5S)-2-(2-Naphthylmethoxymethyl)-3-phenyl-5-fluor -cyclopentyl]-5(E)-heptensäure (B):

20 mg (60 »mol) der nach Beispiel 34a dargestellten Verbindung setzt man in Analogie zu Beispiel 24 unter Verwendung von 2-Brommethylnaphtalin um und isoliert nach Aufarbeitung und Reinigung 15 mg (33 »mol, 50%) der Titelverbindung A sowie 7 mg (14 »mol, 21%) der Titelverbindung B jeweils als farbloses Öl.
IR (Film) von A: 3600-2400, 3060, 3020, 2930, 2850, 1705, 1600, 1490, 1455, 1435, 1410, 1345, 1270, 1240, 1125, 1100, 950, 855, 820, 755 und 700 cm⁻¹.
IR (Film) von B: 3600-2400, 3060, 3030, 2930, 2850, 1705, 1600, 1455, 1435, 1410, 1345, 1125, 1100, 1090, 1070, 970, 950, 855, 815, 755 und 700 cm⁻¹.

### Beispiel 49:

### 7-[(1R,2S,3RS,5R)-2-[2-(4-Fluorphenoxy)-ethoxymethyl]-3-phenyl-5-fluor-cyclopentyl]-5(E/Z)-heptensäure:

31 mg (93 »mol) der nach Beispiel 24a dargestellten Verbindung setzt man in Analogie zu Beispiel 24 unter Verwendung von 1-Brom-2-(4-fluorphenoxy)-ethan um und isoliert nach Aufarbeitung und Reinigung 15 mg (32 »mol, 35%) der Titelverbindung als farbloses Öl.
IR (Film): 3600-2400, 3060, 3030, 2930, 2870, 1710, 1600, 1505, 1455, 1250, 1210, 1130, 825, 760, 740 und 700 cm⁻¹.

### Beispiel 50:

### 7-[(1R,2S,3RS,5S)-2-[(3RS,4S)-3-hydroxy-4-methyl-non-1(E)-en-6-inyl]-3-phenyl-5-fluor-cyclopentyl]-5(Z)-heptensäuremethylester (A) und 7-[(1R,2S,3RS,5S)-2-[(3RS,4S)-3-hydroxy-4-methyl-non-1(E)-en-6-inyl]-3-phenyl-5-fluor-cyclopentyl]-5(E)-heptensäuremethylester (B):

41 mg (91 »mol) der nach Beispiel 50a dargestellten Verbindung setzt man in Analogie zu Beispiel 15 um und isoliert nach Aufarbeitung und Reinigung 21 mg (46 »mol, 51%) der Titelverbindung A sowie 15 mg (33 »mol, 36%) der Titelverbindung B, jeweils als farbloses Öl.
IR (Film): 3600-3200, 3060, 3030, 2970, 2930, 2850, 1735, 1600, 1495, 1450, 1240, 1125, 970, 760 und 700 cm⁻¹.

### Beispiel 50a:

### 7-[(1R,2S,3RS,5S)-2-[3-oxo-4S-methyl-non-1(E)-en-6-inyl]-3-phenyl-5-fluor-cyclopentyl]-5(E/Z)-heptensäuremethylester:

Zu der Aufschlämmung von 8 mg Natriumhydrid-Dispersion (55%ig) in 410 »l wasserfreiem Tetrahydrofuran tropft man unter einer Atmosphäre aus trockenem Argon die Lösung von 45 mg Dimethyl-(2-oxo-3S-methyl-oct-5-inyl)-phosphonat in 330 »l Tetrahydrofuran und rührt 20 Minuten bei 23°C. Man kühlt auf -30°C, troft die Lösung von 70 mg (164 »mol) des nach Beispiel 50b dargestellten Aldehyds in 490 »l Tetrahydrofuran zu und rührt 14 Stunden bei -15°C. Man versetzt mit Essigsäure, und extrahiert mehrfach mit Diethylether. Die vereinigten organischen Extrakte wäscht man mit verdünnter Natriumhydrogenkarbonatlösung, trocknet über Magnesiumsulfat und reinigt den nach Lösungsmittelabzug erhaltenen Rückstand durch Chromatographie an 5 analytischen Dünnschichtplatten. Als Laufmittel dient ein Gemisch aus n-Hexan und Ethylacetat, als Elutionsmittel Diethylether. Isoliert werden 41 mg (91 »mol, 55%) der Titelverbindung als farbloses Öl.
IR (Film): 3030, 3000, 2970, 2930, 2870, 1735, 1690, 1665, 1625, 1450, 1435, 1170, 1040, 980, 760 und 700 cm⁻¹.

### Beispiel 50b:

### 7-[(1R,2S,3RS,5S)-2-formyl-3-phenyl-5-fluor-cyclopentyl]-5-(E/Z)-heptensäuremethylester:

Zu der unter einer Atmosphäre aus trockenem Argon bei -60°C vorgelegten Lösung von 34 »l frisch destilliertem Oxalylchlorid 690 »l wasserfreiem Dichlormethan tropft man die Lösung von 63 »l Dimethylsulfoxid in 280 »l Dichlormethan, laßt 15 Minuten reagieren und versetzt anschließend mit der Lösung von 114 mg (341 »mol) des nach Beispiel 34a dargestellten Alkohols in 690 »l Dichlormethan. Man läßt 4 Stunden reagieren, versetzt mit 107 »l Triethylamin, gießt in Eiswasser und extrahiert mehrfach mit Dichlormethan. Die vereinigte organischen Extrakte trocknet man über Magnesiumsulfat. Den nach Filtration und Lösungsmittelabzug erhaltenen Rückstand setzt man ohne Reinigung weiter um.

### Beispiel 51:

### 7-[(1R,2S,3RS,5S)-2-[(3R oder 3S,4S)-3-hydroxy-4-methyl-non-1(E)-en-6-inyl]-3-phenyl-5-fluor-cyclopentyl]-5(E)-heptensäure (A) und 7-[(1R,2S,3RS,5S)-2-[(3S oder 3R,4S)-3-hydroxy-4-methyl-non-1(E)-en-6-inyl]-3-phenyl-5-fluor-cyclopentyl]-5(E)-heptensäuremethylester (B):

15 mg (33 »mol) der nach Beispiel 50 dargestellten Verbindung B setzt man in Analogie zu Beispiel 2 um und isoliert nach Aufarbeitung und Reinigung 5 mg (11 »mol, 33%) einer unpolareren Komponente, der man die Struktur der Titelverbindung A zuordnete sowie 5,3 mg (12 »mol, 36%) einer polareren Komponente, der man die Struktur der Titelverbindung B zuordnete jeweils als farbloses Öl.
IR (Film) von A: 3600-2400, 3060, 3030, 2970, 2930, 2850, 1710, 1600, 1495, 1455, 1435, 1240, 1130, 970, 760 und 700 cm⁻¹.
IR (Film) von B: 3600-2400, 3060, 3030, 2970, 2930, 1710, 1600, 1495, 1455, 1435, 1385, 1240, 1070, 1030, 1015, 970, 760 und 700 cm⁻¹.

### Beispiel 52:

### 7-[(1R,2S,3RS,5S)-2-[(3RS,4S)-3-hydroxy-4-methyl-non-1(E)-en-6-inyl]-3-phenyl-5-fluor-cyclopentyl]-5(Z)-heptensäure:

21 mg (46 »mol) der nach Beispiel 50 dargestellten Verbindung A setzt man in Analogie zu Beispiel 2 um und isoliert nach Aufarbeitung und Reinigung 17 mg (39 »mol, 84%) der Titelverbindung als farbloses Öl.
IR (Film): 3600-2400, 3080, 3060, 3030, 3000, 2970, 2930, 2870, 1710, 1600, 1495, 1455, 1435, 1405, 1345, 1320, 1240, 1035, 1010, 970, 760 und 700 cm⁻¹.

### Beispiel 53:

### 7-[(1R,2S,3RS,5S)-2-[3(RS)-hydroxy-oct-1(E)-enyl]-3-phenyl-5-fluor-cyclo-pentyl]-5(E)-heptensäuremethylester (A) und 7-[(1R,2S,3RS,5S)-2-[3(RS)-hydroxy-oct-1(E)-enyl]-3-phenyl-5-fluor-cyclo-pentyl]-5(Z)-heptensäuremethylester (B):

61 mg (142 »mol) der nach Beispiel 53a dargestellten Verbindung setzt man in Analogie zu Beispiel 15 um und isoliert nach Aufarbeitung und Reinigung 47 mg (109 »mol, 77%) der Titelverbindung A sowie 12 mg (28 »mol, 20%) der Titelverbindung B jeweils als farbloses Öl.
IR (Film): 3600-3200, 3030, 3000, 2950, 2930, 2850, 1735, 1600, 1450, 1435, 1245, 1170, 1070, 1030, 970, 755 und 700 cm⁻¹.

### Beispiel 53a:

### 7-[(1R,2S,3RS,5S)-2-[3-oxo-oct-1(E)-enyl]-3-phenyl-5-fluor-cyclo-pentyl]-5(E/Z)-heptensäuremethylester:

70 mg (168 »mol) der nach Beispiel 50b dargestellten Verbindung setzt man in Analogie zu Beispiel 50a unter Verwendung von Dimethyl-(2-oxo-heptyl)-phosphonat um und isoliert nach Aufarbeitung und Reinigung 61 mg (142 »mol, 77%) der Titelverbindung als farbloses Öl.
IR (Film): 3030, 2950, 2930, 2850, 1730, 1690, 1670, 1625, 1450, 1430, 1165, 1070, 980, 760 und 700 cm⁻¹.

### Beispiel 54:

### 7-[(1R,2S,3RS,5S)-2-[(3S oder 3R)-3-hydroxy-oct-1(E)-enyl]-3-phenyl-5-fluor-cyclopentyl]-5(E)-heptensäure (A) und 7-[(1R,2S,3RS,5S)-2-[(3R oder 3S)-3-hydroxy-oct-1(E)-enyl]-3-phenyl-5-fluor-cyclopentyl]-5(E)-heptensäure (B):

12 mg (27 »mol) der nach Beispiel 53 dargestellten Verbindung A setzt man in Analogie zu Beispiel 2 um und isoliert nach Aufarbeitung und Reinigung 5,3 mg (13 »mol, 47%) einer unpolareren Komponente, der man die Struktur der Titelverbindung A zuordnete sowie 4,9 mg (12 »mol, 44%) einer polareren Komponente, der man die Struktur der Titelverbindung B zuordnete jeweils als farbloses Öl.
IR (Film) von A: 3600-2400, 3080, 3060, 3030, 2950, 2930, 2860, 1690, 1600, 1495, 1455, 1305, 1260, 1035, 965, 755 und 700 cm⁻¹.
IR (Film) von B: 3600-2400, 3080, 3060, 3030, 2950, 2930, 2860, 1700, 1600, 1495, 1455, 1340, 1325, 1260, 1135, 1070, 1015, 965, 755 und 700 cm⁻¹.

### Beispiel 55:

### 7-[(1R,2S,3RS,5S)-2-[3(RS)-hydroxy-oct-1(E)-enyl]-3-phenyl-5-fluor-cyclopentyl]-5(Z)-heptensäure:

33 mg (77 »mol) der nach Beispiel 53 dargestellten Verbindung B setzt man in Analogie zu Beispiel 2 um und isoliert nach Aufarbeitung und Reinigung 17 mg (41 »mol, 53%) der Titelverbindung als farbloses Öl.
IR (Film): 3600-2400, 3080, 3060, 3030, 3000, 2950, 2930, 2860, 1710, 1600, 1495, 1465, 1405, 1345, 1240, 1035, 970, 760 und 700 cm⁻¹.

### Beispiel 56:

### 7-[(1S,2R,3RS,5R)-2-[(3RS,4S)-3-hydroxy-4-methyl-non-1(E)-en-6-inyl]-3-phenyl-5-fluor-cyclopentyl]-5(Z)-heptensäuremethylester:

183 mg (404 »mol) der nach Beispiel 56a dargestellten Verbindung setzt man in Analogie zu Beispiel 15 um und isoliert nach Aufarbeitung und Reinigung 184 mg (404 »mol, 100%) der Titelverbindung als farbloses Öl.
IR (Film): 3600-3200, 3030, 2960, 2920, 2870, 1735, 1450, 1435, 1245, 1170, 1145, 1025, 970, 760 und 700 cm⁻¹.

### Beispiel 56a:

### 7-[(1S,2R,3RS,5R)-2-[3-oxo-4S-methyl-non-1(E)-en-6-inyl]-3-phenyl-5-fluor-cyclopentyl]-5(Z)-heptensäuremethylester:

274 mg (561 »mol) der nach Beispiel 56b dargestellten Verbindung setzt man in Analogie zu Beispiel 50a um und isoliert nach Aufarbeitung und Reinigung 183 mg (404 »mol, 72%) der Titelverbindung als farbloses Öl.
IR (Film): 3060, 3030, 3000, 2970, 2930, 2870, 1735, 1690, 1670, 1625, 1450, 1430, 1370, 1170, 1040, 970, 760 und 700 cm⁻¹.

### Beispiel 56b:

### 7-[(1S,2R,3RS,5R)-2-formyl-3-phenyl-5-fluor-cyclopentyl]-5-(Z)-heptensäuremethylester:

500 mg (1,5 mmol) der nach Beispiel 41a dargestellten Verbindung setzt man in Analogie zu Beispiel 50b um und isoliert nach Aufarbeitung 500 mg (1,5 mmol, 100%) der Titelverbindung als farbloses Öl, das man ohne Reinigung weiter umsetzt.

### Beispiel 57:

### 7-[(1S,2R,3RS,5R)-2-[(3RS,4S)-3-hydroxy-4-methyl-non-1(E)-en-6-inyl]-3-phenyl-5-fluor-cyclopentyl]-5(Z)-heptensäure:

184 mg (404 »mol) der nach Beispiel 56 dargestellten Verbindung verseift man in Analogie zu Beispiel 2 und isoliert nach Aufarbeitung und Reinigung 132 mg (300 »mol, 74%) der Titelverbindung als farbloses Öl.
IR (Film): 3600-2400, 3060, 3030, 2970, 2950, 1710, 1600, 1455, 1240, 970, 760 und 700 cm⁻¹.

### Beispiel 58:

### 7-[(1S,2R,3RS,5R)-2-[3(RS)-hydroxy-oct-1(E)-enyl]-3-phenyl-5-fluor-cyclopentyl]-5(Z)-heptensäuremethylester:

298 mg (695 »mol) der nach Beispiel 58a dargestellten Verbindung setzt man in Analogie zu Beispiel 15 um und isoliert nach Aufarbeitung und Reinigung 260 mg (603 »mol, 87%) der Titelverbindung als farbloses Öl.
IR (Film): 3600-3200, 3060, 3030, 3000, 2950, 2930, 2850, 1735, 1600, 1450, 1435, 1245, 1170, 1070, 1030, 970, 760 und 700 cm⁻¹.

### Beispiel 58a:

### 7-[(1S,2R,3RS,5R)-2-[3-oxo-oct -1(E)-enyl]-3-phenyl-5-fluor-cyclopentyl]-5(Z)-heptensäuremethylester:

270 mg (748 »mol) der nach Beispiel 56b dargestellten Verbindung setzt man in Analogie zu Beispiel 56a unter Verwendung von Dimethyl-(2-oxo-heptyl)-phosphonat um und isoliert nach Aufarbeitung und Reinigung 298 mg (695 »mol, 93%) der Titelverbindung als farbloses Öl.
IR (Film): 3030, 2950, 2920, 2850, 1730, 1690, 1670, 1625, 1450, 1430, 1240, 1165, 980, 760 und 700 cm⁻¹.

### Beispiel 59:

### 7-[(1S,2R,3RS,5R)-2-[3(RS)-hydroxy-oct-1(E)-enyl]-3-phenyl-5-fluor-cyclopentyl]-5(Z)-heptensäure:

260 mg (604 »mol) der nach Beispiel 58 dargestellten Verbindung verseift man in Analogie zu Beispiel 2 und isoliert nach Aufarbeitung und Reinigung 235 mg (564 »mol, 93%) der Titelverbindung als farbloses Öl.
IR (Film): 3600-2400, 3060, 3030, 2960, 2920, 2850, 1710, 1600, 1450, 1270, 970, 760 und 700 cm⁻¹.

### Beispiel 60:

### 7-[(1S,2R,3RS,5R)-2-[(3RS,4RS)-3-hydroxy-4-phenyl-pent-1(E)-enyl]-3-phenyl-5-fluor-cyclopentyl]-5(Z)-heptensäuremethylester:

172 mg (372 »mol) der nach Beispiel 60a dargestellten Verbindung setzt man in Analogie zu Beispiel 15 um und isoliert nach Aufarbeitung und Reinigung 170 mg (366 »mol, 98%) der Titelverbindung als farbloses Öl.
IR (Film): 3600-3200, 3060, 3030, 2960, 2940, 1730, 1600, 1495, 1450, 1240, 1020, 970, 760 und 700 cm⁻¹.

### Beispiel 60a:

### 7-[(1S,2R,3RS,5R)-2-[3-oxo-4RS-phenyl-pent-1(E)-enyl]-3-phenyl-5-fluor-cyclopentyl]-5(Z)-heptensäuremethylester:

274 mg (561 »mol) der nach Beispiel 56b dargestellten Verbindung setzt man in Analogie zu Beispiel 56a unter Verwendung von Dimethyl-(2-oxo-3-phenyl-butyl)-phosphonat um und isoliert nach Aufarbeitung und Reinigung 172 mg (372 »mol, 66%) der Titelverbindung als farbloses Öl.
IR (Film): 3060, 3030, 2950, 2930, 2850, 1735, 1690, 1670, 1625, 1490, 1450, 1430, 1370, 1245, 1165, 1070, 1030, 980, 760 und 700 cm⁻¹.

### Beispiel 61:

### 7-[(1S,2R,3RS,5R)-2-[(3R oder 3S,4RS)-3-hydroxy-4-phenyl-pent-1(E)-enyl]-3-phenyl-5-fluor-cyclopentyl]-5(Z)-heptensäure (A) und 7-[(1S,2R,3RS,5R)-2-[(3S oder 3R,4RS)-3-hydroxy-4-phenyl-pent-1(E)-enyl]-3-phenyl-5-fluor-cyclopentyl]-5(Z)-heptensäuremethylester (B):

170 mg (366 »mol) der nach Beispiel 60 dargestellten Verbindung verseift man in Analogie zu Beispiel 2 und isoliert nach Aufarbeitung und Reinigung 24 mg (53 »mol, 15%) einer unpolaren Komponente, der man die Struktur der Titelverbindung A zuordnet, sowie 45 mg (100 »mol, 27%) einer polaren Komponente, der man die Struktur der Titelverbindung B zuordnet, jeweils als farbloses Öl.
IR (Film) von A: 3600-2400, 3060, 3030, 2960, 2940, 1710, 1600, 1500, 1450, 1240, 1020, 970, 760 und 700 cm⁻¹.
IR (Film) von B: 3600-2400, 3060, 3030, 2960, 2950, 1710, 1600, 1495, 1455, 1240, 1030, 1010, 980, 760 und 700 cm⁻¹.

### Beispiel 62:

### 7-[(IS,2R,3R,5R)-2-[(E/X)-Diphenylmethoxyiminomethyl]-3,5-dihydroxy-cyclopentyl]-5(Z)-heptensäure:

108 mg (207 »mol) der nach Beispiel 62a dargestellten Verbindung versetzt man mit 1,2 ml eines Gemisches aus Essigsäure, Wasser und Tetrahydrofuran (65:35:10) und rührt 15 Stunden bei 23°C. Man engt ein, entfernt restliche Essigsäure durch wiederholte azeotrope Destillation mit Toluol und reinigt den Rückstand durch Chromatographie an ca. 20 ml feinem Kieselgel. Isoliert werden 75 mg (171 »mol, 83%) der Titelverbindung als farbloses Öl.
IR (Film): 3700-2400, 3090, 3060, 3030, 3010, 2940, 1710, 1490, 1450, 1245, 1035, 1020, 940, 745 und 700 cm⁻¹.

### Beispiel 62a:

### 7-[(1S,2R,3R,5R)-2-[(E/Z)-Diphenylmethoxyiminomethyl]-3-(tetrahydropyran-2-yloxy)-5-hydroxy-cyclopentyl]-5(Z)-heptensäure:

1,18 g (1,84 mmol) der nach Beispiel 62b dargestellten Verbindung verseift man in Analogie zu Beispiel 2 und isoliert nach Aufarbeitung und Reinigung 943 mg (1,81 mmol, 98%) der Titelverbindung als farbloses Öl.
IR (Film): 3600-2400, 3090, 3060, 3030, 3010, 2940, 2870, 1740, 1710, 1600, 1495, 1450, 1240, 1130, 1115, 1035, 1020, 935, 920, 870, 815, 745 und 705 cm⁻¹.

### Beispiel 62b:

### 7-[(1S,2R,3R,5R)-2-[(E/X)-Diphenylmethoxyiminomethyl]-3-(tetrahydropyran-2-yloxy)-5-benzoyloxy-cyclopentyl]-5(Z)-heptensäuremethylester:

Die farblose Lösung von 1,0 g (2,18 mmol) der nach Beispiel 62c dargestellten Verbindung in 16 ml wasserfreiem Ethanol versetzt man mit 0,4 ml wasserfreiem Pyridin, 588 mg Diphenylmethoxyamin und erhitzt 3,5 Stunden unter einer Atmosphäre aus trockenem Argon auf 50°C. Man engt ein, nimmt den Rückstand in Dichlormethan auf, wäscht mit Wasser und gesättigter Natriumchloridlösung und reinigt den nach Trocknen über Magnesiumsulfat, Filtration und Einengen erhaltenen Rückstand durch Chromatographie an ca. 50 ml Kieselgel mit einem n-Hexan/Ethylacetat-Gemisch. Isoliert werden 1,18 g (1,84 mmol, 85%) der Titelverbindung als farbloses Öl.
IR (Film): 3070, 3030, 3010, 2950, 2870, 1740, 1715, 1605, 1585, 1495, 1450, 1360, 1275, 1115, 1025, 940, 920, 870, 815, 745, 715 und 705 cm⁻¹.

### Beispiel 62c:

### 7-[(1S,2R,3R,5R)-2-Formyl-3-(tetrahydropyran-2-yloxy)-5-benzoyloxy-cyclopentyl]-5(Z)-heptensäuremethylester:

9 g (19,5 mmol) der nach Beispiel 62d dargestellten Verbindung setzt man in Analogie zu Beispiel 50b um und isoliert nach Aufarbeitung 9 g (19,5 mmol, 100%) der Titelverbindung, die man ohne Reinigung weiter umsetzt.

### Beispiel 62d:

### 7-[(1S,2R,3R,5R)-2-Hydroxymethyl-3-(tetrahydropyran-2-yloxy)-5-benzoyloxy-cyclopentyl]-5(Z)-heptensäuremethylester:

22,4 g (32 mmol) der nach Beispiel 62e dargestellten Verbindung setzt man in Analogie zu Beispiel 1g um und isoliert nach Aufarbeitung und Reinigung 13,7 g (29,7 mmol, 93%) der Titelverbindung als farbloses Öl.
IR (Film): 3600-3200, 3070, 2950, 2870, 1740, 1720, 1605, 1585, 1450, 1370, 1315, 1275, 1245, 1115, 1075, 1030, 870, 810 und 715 cm⁻¹.

### Beispiel 62e:

### 7-[(1S,2R,3R,5R)-2-(tert.-Butyldiphenylsilyloxymethyl)-3-(tetrahydropyran-2-yloxy)-5-benzoyloxy-cyclopentyl]-5(Z)-heptensäuremethylester:

19,8 g (33,4 mmol) der nach Beispiel 62f dargestellten Verbindung setzt man in Analogie zu Beispiel 1h um und isoliert nach Aufarbeitung und Reinigung 22,4 g (32 mmol, 96%) der Titelverbindung als farbloses Öl.
IR (Film): 3070, 3050, 3010, 2940, 2860, 1740, 1720, 1605, 1590, 1455, 1430, 1275, 1115, 1070, 1025, 825, 740 und 710 cm⁻¹.

### Beispiel 62f:

### 7-[(1S,2R,3R,5R)-2-(tert.-Butyldiphenylsilyloxymethyl)-3-(tetrahydropyran-2-yloxy)-5-hydroxy-cyclopentyl]-5(Z)-heptensäuremethylester:

25,8 g (44,4 mmol) der nach Beispiel 62g dargestellten Verbindung setzt man in Analogie zu Beispiel 1i um und isoliert nach Aufarbeitung und Reinigung 19,9 g (33,4 mmol, 75%) der Titelverbindung als farbloses Öl.
IR (Film): 3600-3200, 3070, 3050, 3010, 2940, 2860, 1740, 1590, 1430, 1200, 1110, 1075, 1020, 1000, 870, 825, 740 und 705 cm⁻¹.

### Beispiel 62g:

### 7-[(1S,2R,3R,5R)-2-(tert.-Butyldiphenylsilyloxymethyl)-3-(tetrahydropyran-2-yloxy)-5-hydroxy-cyclopentyl]-5(Z)-heptensäure:

23,8 g (47,9 mmol) der nach Beispiel 62h dargestellten Verbindung setzt man in Analogie zu Beispiel 1j um und isoliert nach Aufarbeitung und Reinigung 25,8 g (44,4 mmol, 93%) der Titelverbindung als farbloses Öl.
IR (Film): 3700-2400, 3070, 3050, 2940, 2860, 1710, 1590, 1430, 1390, 1260, 1200, 1110, 1075, 1045, 1020, 995, 880, 825, 740 und 705 cm⁻¹.

### Beispiel 62h:

### (1R,3RS,5S,6R,7R)-3-Hydroxy-6-(tert.-butyldiphenylsilyloxymethyl)-7-(tetrahydropyran-2-yloxy)-2-oxabicyclo[3.3.0]octan:

23,8 g (48,1 mmol) der nach Beispiel 62i dargestellten Verbindung setzt man in Analogie zu Beispiel 1k um und isoliert nach Aufarbeitung und Reinigung 23,8 g (47,9 mmol, 99%) der Titelverbindung als farbloses Öl.
IR (Film): 3600-3200, 3070, 3050, 2940, 2860, 1740, 1590, 1430, 1390, 1375, 1355, 1240, 1110, 1075, 1020, 870, 820, 740 und 705 cm⁻¹.

### Beispiel 62i:

### (1R,5S,6R,7R)-3-Oxo-6-(tert.-butyldiphenylsilyloxymethyl)-7-(tetrahydropyran-2-yloxy)-2-oxabicyclo[3.3.0]octan:

Die Lösung von 20,7 g (50,4 mmol) der nach Beispiel 62j dargestellten Verbindung in 400 ml wasserfreiem Dichlormethan versetzt man bei 0°C mit 6,83 ml Dihydropyran, 135 mg p-Toluolsulfonsäure und rührt 1,5 Stunden unter einer Atmosphäre aus trockenem Argon. Man verdünnt mit Dichlormethan, wäscht mit einer gesättigten Natriumhydrogenkarbonatlösung, einer gesättigten Natriumchloridlösung und trocknet über Magnesiumsulfat. Den nach Filtration und Lösungsmittelabzug erhaltenen Rückstand reinigt man durch Chromatographie an ca. 300 ml feinem Kieselgel. Isoliert werden 23,8 g (48,1 mmol, 95%) der Titelverbindung als farbloses Öl.
IR (Film): 3070, 3050, 2940, 2860, 1780, 1740, 1590, 1470, 1430, 1390, 1375, 1355, 1245, 1170, 1110, 1075, 1035, 870, 825, 745 und 705 cm⁻¹.

### Beispiel 62j:

### (1R,5S,6R,7R)-3-Oxo-6-(tert.-butyldiphenylsilyloxymethyl)-7-hydroxy-2-oxabicyclo[3.3.0] octan:

Die Lösung von 1,5 g (2,66 mmol) der nach Beispiel 62k dargestellten Verbindung in 74 ml wasserfreiem Dimethylformamid versetzt man mit 3,32 g Kaliumnitrit und erhitzt unter einer Atmosphäre aus trockenem Argon 16 Stunden auf 85°C. Man engt ein, verdünnt mit Wasser und extrahiert mehrfach mit Dichlormethan. Die vereinigten organischen Extrakte wäscht man mit gesättigter Natriumchloridlösung, trocknet über Magnesiumsulfat und reinigt den nach Lösungsmittelabzug erhaltenen Rückstand durch Chromatographie an ca. 100 ml feinem Kieselgel. Isoliert werden 700 mg (1,70 mmol, 64%) der Titelverbindung als farbloses Öl.
IR (Film): 3600-3200, 3070, 3050, 2940, 2960, 1770, 1590, 1430, 1240, 1170, 1115, 1075, 825, 745, 705, 615 und 510 cm⁻¹.

### Beispiel 62k:

### (IR,5S,6R,7S)-3-Oxo-6-(tert.-butyldiphenylsilyloxymethyl)-7-toluolsulfonyloxy-2-oxabicyclo[3.3.0]octan:

2,37 g (5,77 mmol) (1R,5S,6R,7S)-3-Oxo-6-(tert.-butyldiphenylsilyloxymethyl)-7-hydroxy-2-oxabicyclo[3.3.0]octan setzt man in Analogie zu Beispiel 1m um und isoliert nach Aufarbeitung und Reinigung 2,9 g (5,17 mmol, 90%) der Titelverbindung als farbloses Öl.
IR (CHCl₃): 3070, 3050, 3030, 3000, 2960, 2930, 2860, 1770, 1600, 1425, 1365, 1190, 1175, 1110, 1040, 980, 960, 895, 875, 820 und 700 cm⁻¹.

### Beispiel 63:

### 7-[(1S,2R,3R,5R)-2-[(E/Z)-Phenylureidoiminomethyl]-3,5-dihydroxy-cyclopentyl]-5(Z)-heptensäure:

99,5 mg (210 »mol) der nach Beispiel 63a dargestellten Verbindung setzt man in Analogie zu Beispiel 62 um und isoliert nach Aufarbeitung und Reinigung 77 mg (198 »mol, 94%) der Titelverbindung als farbloses Öl.
IR (Film): 3600-2400, 3380, 3260, 3010, 2950, 2860, 1710, 1670, 1600, 1590, 1500, 1450, 1240, 750 und 690 cm⁻¹.

### Beispiel 63a:

### 7-[(1S,2R,3R,5R)-2-[(E/Z)-Phenylureidoiminomethyl]-3-(tetrahydropyran-2-yloxy)-5-hydroxy-cyclopentyl]-5(Z)-heptensäure:

345 mg (583 »mol) der nach Beispiel 63b dargestellten Verbindung verseift man in Analogie zu Beispiel 2 und isoliert nach Aufarbeitung und Reinigung 248 mg (418 »mol, 72%) der Titelverbindung als farbloses Öl.
IR (Film): 3600-2400, 3380, 3220, 3100, 3010, 2940, 2870, 1710, 1690, 1590, 1535, 1450, 1320, 1230, 1115, 1030, 1020, 985, 870, 810, 755 und 690 cm⁻¹.

### Beispiel 63b:

### 7-[(1S,2R,3R,5R)-2-[(E/Z)-Phenylureidoiminomethyl]-3-(tetrahydropyran-2-yloxy)-5-benzoyloxy-cyclopentyl]-5(Z)-heptensäuremethylester:

600 mg (1,31 mmol) der nach Beispiel 62c dargestellten Verbindung setzt man in Analogie zu Beispiel 62b unter Verwendung von 4-Phenylsemicarbazid um und isoliert nach Aufarbeitung und Reinigung 775 mg (1,31 mmol, 100%) der Titelverbindung als farbloses Öl.
IR (Film): 3380, 3200, 3100, 2950, 2870, 1715, 1690, 1595, 1535, 1450, 1275, 1225, 1115, 1035, 1025, 980, 870, 815, 760, 715 und 695 cm⁻¹.

### Beispiel 64:

### 7-[1S,2R,3R,5R)-2-(2-Fluorbenzyloxymethyl)-3,5-dihydroxy-cyclopentyl]-5(Z)-heptensäure:

50,6 mg (112 »mol) der nach Beispiel 64a dargestellten Verbindung setzt man in Analogie zu Beispiel 62 um und isoliert nach Aufarbeitung und Reinigung 34,9 mg (95 »mol, 85%) der Titelverbindung als farbloses Öl.
IR (Film): 3600-2400, 3010, 2930, 2870, 1710, 1620, 1595, 1495, 1455, 1230, 1110, 1085, 1020, 995 und 760 cm⁻¹.

### Beispiel 64a:

### 7-[(1S,2R,3R,5R)-2-(2-Fluorbenzyloxymethyl)-3-(tetrahydropyran-2-yloxy)-5-hydroxy-cyclopentyl]-5(Z)-heptensäure:

127 mg (224 »mol) der nach Beispiel 64b dargestellten Verbindung verseift man in Analogie zu Beispiel 2 und isoliert nach Aufarbeitung und Reinigung 69 mg (153 »mol, 69%) der Titelverbindung als farbloses Öl.
IR (Film): 3600-2400, 3010, 2930, 2870, 1710, 1620, 1590, 1495, 1455, 1235, 1115, 1085, 1020, 995, 870, 810 und 760 cm⁻¹.

### Beispiel 64b:

### 7-[(1S,2R,3R,5R)-2-(2-Fluorbenzyloxymethyl)-3-(tetrahydropyran-2-yloxy)-5-benzoyloxy-cyclopentyl]-5(Z)-heptensäuremethylester:

Die Lösung von 500 mg (1,09 mmol) der nach Beispiel 62d dargestellten Verbindung in 8,5 ml Toluol versetzt man mit 3,5 ml einer 25%igen wässrigen Natriumhydroxidlösung, 50 mg Tetrabutylammoniumhydrogensulfat, 1,03 g 2-Fluorbenzylbromid und rührt das 2-Phasen-System 5 Tage bei 23°C. Man verdünnt mit Wasser, stellt durch Zugebe von gesättigter Zitronensäure einen pH-Wert von 5 ein und extrahiert mehrfach mit Diethylether. Die vereinigten organischen Extrakte trocknet man über Magnesiumsulfat und reinigt den nach Lösungsmittelabzug erhaltenen Rückstand durch Chromatographie an ca. 100 ml Kieselgel mit einem Gemisch aus n-Hexan und Ethylacetat. Isoliert werden 137 mg (241 »mol, 22%) der Titelverbindung als farbloses Öl.
IR (Film): 3070, 3010, 2950, 2870, 1740, 1720, 1620, 1605, 1590, 1495, 1455, 1365, 1315, 1275, 1115, 1025, 870, 815, 760 und 715 cm⁻¹.

### Beispiel 65:

### 7-[(1S,2R,3R,5R)-2-Benzyloxymethyl-3,5-dihydroxy-cyclopentyl]-5(Z)-heptensäure:

57,5 mg (133 »mol) der nach Beispiel 65a dargestellten Verbindung setzt man in Analogie zu Beispiel 62 um und isoliert nach Aufarbeitung und Reinigung 40 mg (115 »mol, 86%) der Titelverbindung als farbloses Öl.
IR (Film): 3700-2400, 3070, 3030, 3010, 2930, 2870, 1710, 1450, 1240, 1100, 1070, 740 und 700 cm⁻¹.

### Beispiel 65a:

### 7-[(1S,2R,3R,5R)-2-Benzyloxymethyl-3-(tetrahydropyran-2-yloxy)-5-hydroxycyclopentyl]-5(Z)-heptensäure:

126 mg (229 »mol) der nach Beispiel 65b dargestellten Verbindung verseift man in Analogie zu Beispiel 2 und isoliert nach Aufarbeitung und Reinigung 77 mg (178 »mol, 78%) der Titelverbindung als farbloses Öl.
IR (Film): 3600-2400, 3060, 3030, 3010, 2940, 2870, 1710, 1455, 1365, 1260, 1240, 1200, 1115, 1075, 1020, 995, 870, 810, 740 und 700 cm⁻¹.

### Beispiel 65b:

### 7-[(1S,2R,3R,5R)-2-Benzyloxymethyl-3-(tetrahydropyran-2-yloxy)-5-benzoyloxy-cyclopentyl]-5(Z)-heptensäuremethylester:

500 mg (1.09 »mol) der nach Beispiel 62d dargestellten Verbindung setzt man in Analogie zu Beispiel 64b unter Verwendung von Benzylbromid um und isoliert nach Aufarbeitung und Reinigung 134 mg (243 »mol, 22%) der Titelverbindung als farbloses Öl.
IR (Film): 3070, 3030, 3010, 2940, 2860, 1735, 1720, 1605, 1585, 1450, 1365, 1275, 1115, 1075, 1025, 870, 815, 740, 715 und 700 cm⁻¹.

### Beispiel 66:

### 7-[(1S,2R,3R,5R)-2-(3-Methylbenzyloxymethyl)-3,5-dihydroxy-cyclopentyl]-5(Z)-heptensäure:

36,3 mg (81 »mol) der nach Beispiel 66a dargestellten Verbindung setzt man in Analogie zu Beispiel 62 um und isoliert nach Aufarbeitung und Reinigung 23 mg (63 »mol, 78%) der Titelverbindung als farbloses Öl.
IR (Film): 3600-2400, 3010, 2930, 2870, 1710, 1610, 1410, 1245, 1155, 1085, 885, 785, 745 und 700 cm⁻¹.

### Beispiel 66a:

### 7-[(1S,2R,3R,5R)-2-(3-Methylbenzyloxymethyl)-3-(tetrahydropyran-2-yloxy)-5-hydroxy-cyclopentyl]-5(Z)-heptensäure:

68,7 mg (122 »mol) der nach Beispiel 66b dargestellten Verbindung verseift man in Analogie zu Beispiel 2 und isoliert nach Aufarbeitung und Reinigung 41,4 mg (93 »mol, 76%) der Titelverbindung als farbloses Öl.
IR (Film): 3600-2400, 3010, 2940, 2870, 1710, 1610, 1455, 1440, 1355, 1200, 1160, 1115, 1075, 1020, 995, 870, 780, 740 und 695 cm⁻¹.

### Beispiel 66b:

### 7-[(1S,2R,3R,5R)-2-(3-Methylenzyloxymethyl)-3-(tetrahydropyran-2-yloxy)-5-benzoyloxy-cyclopentyl]-5(Z)-heptensäuremethylester:

1,3 g (2,82 mmol) der nach Beispiel 62d dargestellten Verbindung setzt man in Analogie zu Beispiel 64b unter Verwendung von 3-Methylbenzylbromid um und isoliert nach Aufarbeitung und Reinigung 424 mg (751 »mol, 27%) der Titelverbindung als farbloses Öl.
IR (Film): 3060, 3010, 2940, 2860, 1740, 1720, 1605, 1585, 1450, 1360, 1315, 1275, 1115, 1075, 1025, 780 und 715 cm⁻¹.

### Beispiel 67:

### 7-[(1S,2R,3R,5R)-2-(4-Fluorbenzyloxymethyl)-3,5-dihydroxy-cyclopentyl]-5(Z)-heptensäure:

66,8 mg (148 »mol) der nach Beispiel 67a dargestellten Verbindung setzt man in Analogie zu Beispiel 62 um und isoliert nach Aufarbeitung und Reinigung 39,2 mg (107 »mol, 72%) der Titelverbindung als farbloses Öl.
IR (Film): 3600-2400, 3010, 2940, 2870, 1710, 1605, 1510, 1225, 1090, 855 und 825 cm⁻¹.

### Beispiel 67a:

### 7-[(1S,2R,3R,5R)-2-(4-Fluorbenzyloxymethyl)-3-(tetrahydropyran-2-yloxy)-5-hydroxy-cyclopentyl]-5(Z)-heptensäure:

136 mg (238 »mol) der nach Beispiel 67b dargestellten Verbindung verseift man in Analogie zu Beispiel 2 und isoliert nach Aufarbeitung und Reinigung 87,5 mg (194 »mol, 81%) der Titelverbindung als farbloses Öl.
IR (Film): 3600-2400, 3010, 2940, 2870, 1710, 1605, 1510, 1225, 1115, 1075, 1020, 995, 855 und 825 cm⁻¹.

### Beispiel 67b:

### 7-[(1S,2R,3R,5R)-2-(4-Fluorbenzyloxymethyl)-3-(tetrahydropyran-2-yloxy)-5-benzoyloxy-cyclopentyl]-5(Z)-heptensäuremethylester:

500 mg (1,09 mmol) der nach Beispiel 62d dargestellten Verbindung setzt man in Analogie zu Beispiel 64b unter Verwendung von 4-Fluorbenzylbromid um und isoliert nach Aufarbeitung und Reinigung 144 mg (253 »mol, 23%) der Titelverbindung als farbloses Öl.
IR (Film): 3070, 3010, 2950, 2870, 1740, 1715, 1605, 1510, 1450, 1365, 1315, 1275, 1225, 1115, 1025, 1000, 825 und 715 cm⁻¹.

### Beispiel 68:

### 7-[(1S,2R,3S,5S)-2-[(E/Z)-Diphenylmethoxyiminomethyl]-3-hydroxy-5-fluor-cyclopentyl]-5(Z)-heptensäuremethylester:

72 mg (134 »mol) der nach Beispiel 68 dargestellten Verbindung A setzt man in Analogie zu Beispiel 62 um und isoliert nach Aufarbeitung und Reinigung 46 mg (101 »mol, 76%) der Titelverbindung als farbloses Öl.
IR (Film): 3600-3200, 3060, 3030, 3010, 2940, 2870, 1730, 1600, 1495, 1455, 1240, 1020, 935, 745 und 700 cm⁻¹.

### Beispiel 68a:

### 7-[(1S,2R,3S,5S)-2-[(E/Z)-Diphenylmethoxyiminomethyl]-3-(tetrahydropyran-2-yloxy)-5-fluor-cyclopentyl]-5(Z)-heptensäuremethylester (A) und 7-[(5R,4S)-5-[(E/Z)-Diphenylmethoxyiminomethyl]-4-(tetrahydropyran-2-yloxy)-cyclopent-1-enyl]-5(Z)-heptensäuremethylester:

252 mg (471 »mol) der nach Beispiel 68b dargestellten Verbindung setzt man in Analogie zu Beispiel 1 um und isoliert nach Aufarbeitung und Reinigung 72 mg (134 »mol, 28%) der Titelverbindung A sowie 58 mg (112 »mol, 24%) der Titelverbindung B jeweils als farbloses Öl.
IR (Film): 3600-3200, 3060, 3030, 3010, 2940, 2870, 1730, 1600, 1455, 1245, 1020, 935, 870, 820, 745 und 700 cm⁻¹.

### Beispiel 68b:

### 7-[(1S,2R,3S,5R)-2-[(E/Z)-Diphenylmethoxyiminomethyl]-3-(tetrahydropyran-2-yloxy)-5-hydroxy-cyclopentyl]-5(Z)-heptensäuremethylester:

Die Lösung von 257 mg (493 »mol) der nach Beispiel 68c dargestellten Verbindung in 10 ml Dichlormethan versetzt man mit der etherischen Lösung von Diazomethan und engt nach beendeter Umsetzung ein. Isoliert werden 262 mg (489 »mol, 99%) der Titelverbindung als farbloses Öl.
IR (Film): 3600-3200, 3070, 3030, 3010, 2950, 2870, 1740, 1455, 1440, 1350, 1200, 1130, 1080, 1025, 975, 910, 870, 815, 745 und 705 cm⁻¹.

### Beispiel 68c:

### 7-[(1S,2R,3S,5R)-2-[(E/Z)-Diphenylmethoxyiminomethyl]-3-(tetrahydropyran-2-yloxy)-5-hydroxy-cyclopentyl]-5(Z)-heptensäure:

569 mg (889 »mol) der nach Beispiel 68d dargestellten Verbindung verseift man in Analogie zu Beispiel 2 und isoliert nach Aufarbeitung und Reinigung 399 mg (765 »mol, 86%) der Titelverbindung als farbloses Öl.
IR (Film): 3600-2400, 3070, 3030, 3010, 2940, 270, 1730, 1710, 1605, 1495, 1445, 1345, 1245, 1205, 1185, 1130, 1080, 1020, 975, 920, 870, 810, 745 und 705 cm⁻¹.

### Beispiel 68d:

### 7-[(1S,2R,3S,5R)-2-[(E/Z)-Diphenylmethoxyiminomethyl]-3-(tetrahydropyran-2-yloxy)-5-benzoyloxy-cyclopentyl]-5(Z)-heptensäuremethylester:

300 mg (654 »mol) der nach Beispiel 68e dargestellten Verbindung setzt man in Analogie zu Beispiel 62b um und isoliert nach Aufarbeitung und Reinigung 268 mg (453 »mol, 69%) der Titelverbindung als farbloses Öl.
IR (Film): 3380, 3210, 3100, 3010, 2950, 2870, 1735, 1715, 1690, 1600, 1535, 1450, 1360, 1320, 1275, 1115, 1070, 1030, 970, 870, 815, 760, 715 und 695 cm⁻¹.

### Beispiel 68e:

### 7-[(1S,2R,3S,5R)-2-Formyl-3-(tetrahydropyran-2-yloxy)-5-benzoyloxy-cyclopentyl]-5(Z)-heptensäuremethylester:

1,2 g (2,61 mmol) der nach Beispiel 68f dargestellten Verbindung setzt man in Analogie zu Beispiel 50b um und isoliert nach Aufarbeitung 1,2 g (2,61 mmol, 100%) der Titelverbindung, die man ohne Reinigung weiter umsetzt.

### Beispiel 68f:

### 7-[(1S,2R,3S,5R)-2-Hydroxymethyl-3-(tetrahydropyran-2-yloxy)-5-benzoyloxy-cyclopentyl]-5(Z)-heptensäuremethylester:

2,16 g (3,09 mmol) der nach Beispiel 68g dargestellten Verbindung setzt man in Analogie zu Beispiel 1g um und isoliert nach Aufarbeitung und Reinigung 1,2 g (2,61 mmol, 85%) der Titelverbindung als farbloses Öl.
IR (Film): 3600-3200, 3060, 3000, 2940, 2860, 1735, 1710, 1600, 1585, 1450, 1355, 1310, 1270, 1110, 1070, 1025, 865, 810 und 710 cm⁻¹.

### Beispiel 68g:

### 7-[(1S,2R,3S,5R)-2-(tert.-Butyldiphenylsilyloxymethyl)-3-(tetrahydropyran-2-yloxy)-5-benzoyloxy-cyclo-pentyl]-5(Z)-heptensäuremethylester:

59,4 g (99,9 mmol) der in Analogie zu Beispiel 29b dargestellten Verbindung 7-[(1S,2R,3S,5R)-2-(tert.-Butyldiphenylsilyloxymethyl)-3-(tetrahydropyran-2-yloxy)-5-hydroxy-cyclopentyl]-5(Z)-heptensäuremethylester setzt man in Analogie zu Beispiel 1h um und isoliert nach Aufarbeitung und Reinigung 62,5 g (89,4 mmol, 90%) der Titelverbindung als farbloses Öl.
IR (Film): 3070, 3050, 3010, 2950, 2860, 1740, 1715, 1600, 1585, 1450, 1425, 1275, 1110, 1025, 970, 870, 825, 790, 710, 690, 610 und 500 cm⁻¹.

### Beispiel 69:

### 7-[(1S,2R,3S,5S)-2-[(E/Z)-Diphenylmethoxyiminomethyl]-3-hydroxy-5-fluor-cyclopentyl]-5(Z)-heptensäure:

46 mg (101 »mol) der nach Beispiel 68 dargestellten Verbindung verseift man in Analogie zu Beispiel 2 und isoliert nach Aufarbeitung und Reinigung 29 mg (66 »mol, 65%) der Titelverbindung als farbloses Öl.
IR (Film): 3600-2400, 3060, 3030, 3010, 2940, 2870, 1710, 1600, 1495, 1455, 1240, 1020, 935, 745 und 700 cm⁻¹.

### Beispiel 70:

### 7-[(1S,2R,3S,5R)-2-[(E/Z)-Diphenylmethoxyiminomethyl]-3,5-dihydroxy-cyclopentyl]-5(Z)-heptensäure:

128 mg (244 »mol) der nach Beispiel 68c dargestellten Verbindung setzt man in Analogie zu Beispiel 62 um und isoliert nach Aufarbeitung und Reinigung 84 mg (192 »mol, 78%) der Titelverbindung als farbloses Öl.
IR (Film): 3600-2400, 3060, 3030, 3010, 2940, 1710, 1605, 1495, 1450, 1375, 1245, 1045, 1020, 935, 920, 745 und 700 cm⁻¹.

### Beispiel 71:

### 7-[(1S,2R,3R,5S)-2-[(E/Z)-Phenylureidoiminomethyl]-3,5-dihydroxy-cyclopentyl]-5(Z)-heptensäure:

137,6 mg (271 »mol) der nach Beispiel 71a dargestellten Verbindung verseift man in Analogie zu Beispiel 2 und isoliert nach Aufarbeitung und Reinigung 93,8 mg (241 »mol, 89%) der Titelverbindung als farbloses Öl.
IR (Film): 3600-2400, 3370, 3010, 2940, 1730-1680, 1600, 1540, 1450, 1375, 1240, 1115, 1045, 760 und 695 cm⁻¹.

### Beispiel 71a:

### 7-[(1S,2R,3R,5S)-2-[(E/Z)-Phenylureidoiminomethyl]-3-hydroxy-5-benzoyloxy-cyclopentyl]-5(Z)-heptensäuremethylester:

248 mg (419 »mol) der nach Beispiel 71b dargestellten Verbindung setzt man in Analogie zu Beispiel 62 um und isoliert nach Aufarbeitung und Reinigung 149 mg (294 »mol, 70%) der Titelverbindung als farbloses Öl.
IR (Film): 3600-2400, 3380, 3070, 3010, 2950, 2870, 1740-1680, 1600, 1540, 1450, 1360, 1315, 1275, 1175, 1115, 1070, 1030, 940, 760, 715 und 695 cm⁻¹.

### Beispiel 71b:

### 7-[(1S,2R,3R,5S)-2-[(E/Z)-Phenylureidoiminomethyl]-3-(tetrahydropyran-2-yloxy)-5-benzoyloxy-cyclopentyl]-5(Z)-heptensäuremethylester:

300 mg (654 »mol) der nach Beispiel 68e dargestellten Verbindung setzt man in Analogie zu Beispiel 62b um und isoliert nach Aufarbeitung und Reinigung 268 mg (453 »mol, 69%) der Titelverbindung als farbloses Öl.
IR (Film): 3380, 3210, 3100, 3010, 2950, 2860, 1735, 1715, 1690, 1595, 1530, 1450, 1315, 1275, 1115, 1025, 970, 870, 810, 755, 715 und 695 cm⁻¹.

### Beispiel 72:

### 7-[(1S,2R,3S,5S)-2-(3-Methylbenzyloxymethyl)-3-hydroxy-5-fluor-cyclopentyl]-5(Z)-heptensäuremethylester:

201 mg (435 »mol) der nach Beispiel 72a dargestellten Verbindung setzt man in Analogie zu Beispiel 62 um und isoliert nach Aufarbeitung und Reinigung 89 mg (235 »mol, 54%) der Titelverbindung als farbloses Öl.
IR (Film): 3600-3200, 3010, 2940, 2860, 1735, 1610, 1440, 1360, 1250, 1220, 1155, 1100, 1090, 780, 745 und 695 cm⁻¹.

### Beispiel 72a:

### 7-[(1S,2R,3S,5S)-2-(3-Methylbenzyloxymethyl)-3-(tetrahydropyran-2-yloxy)-5-fluor-cyclopentyl]-5(Z)-heptensäuremethylester (A) und 7-[(5R,4S)-5-(3-Methylbenzyloxymethyl)-4-(tetrahydropyran-2-yloxy)-cyclopent- 1-enyl]-5(Z)-heptensäuremethylester (B):

800 mg (1,74 mmol) der nach Beispiel 72b dargestellten Verbindung setzt man in Analogie zu Beispiel 1 um und isoliert nach Aufarbeitung und Reinigung 201 mg (436 »mol, 25%) der Titelverbindung A sowie 212 mg (480 »mol, 28%) der Titelverbindung B jeweils als farbloses Öl.
IR (Film): 3600-3200, 3010, 2940, 2850, 1730, 1610, 1440, 1360, 1250, 1225, 1155, 1095, 870, 815, 780, 745 und 695 cm⁻¹.

### Beispiel 72b:

### 7-[(1S,2R,3S,5R)-2-(3-Methylbenzyloxymethyl)-3-(tetrahydropyran-2-yloxy)-5-hydroxy-cyclopentyl]-5(Z)-heptensäuremethylester:

1,23 g (2,75 mmol) der nach Beispiel 68f dargestellten Verbindung setzt man in Analogie zu Beispiel 64b um und isoliert nach Aufarbeitung und Reinigung 1,18 g (2,56 mmol, 93%) der Titelverbindung als farbloses Öl.
IR (Film): 3600-3300, 3010, 2940, 2860, 1740, 1610, 1595, 1440, 1355, 1250, 1200, 1155, 1115, 1075, 1020, 975, 905, 870, 815, 780, 745 und 695 cm⁻¹.

### Beispiel 73:

### 7-[(1S,2R,3S,5S)-2-(3-Methylbenzyloxymethyl)-3-hydroxy-5-fluor-cyclopentyl]-5(Z)-heptensäure:

30,7 mg (81 »mol) der nach Beispiel 72 dargestellten Verbindung verseift man in Analogie zu Beispiel 2 und isoliert nach Aufarbeitung und Reinigung 19,8 mg (54 »mol, 67%) der Titelverbindung als farbloses Öl.
IR (Film): 3600-2400, 3010, 2930, 2860, 1710, 1610, 1590, 1455, 1440, 1360, 1240, 1155, 1100, 1085, 780, 745 und 695 cm⁻¹.

### Beispiel 74:

### 7-[(1S,2R,5S)-2-(3-Methylbenzyloxymethyl)-5-fluor-cyclopentyl]-5(Z)-heptensäuremethylester:

76 mg (148 »mol) der nach Beispiel 74a dargestellten Verbindung setzt man in Analogie zu Beispiel 11 um und isoliert nach Aufarbeitung und Reinigung 36,4 mg (106 »mol, 72%) der Titelverbindung als farbloses Öl.
IR (Film): 3010, 2930, 2850, 1730, 1610, 1590, 1455, 1410, 1355, 1240, 1160, 1100, 1090, 780, 755 und 695 cm⁻¹.

### Beispiel 74a:

### 7-[(1S,2R,3S,5S)-2-(3-Methylbenzyloxymethyl)-3-toluolsulfonylo xy-5-fluor-cyclopentyl]-5(Z)-heptensäuremethylester:

58 mg (162 »mol) der nach Beispiel 72 dargestellten Verbindung setzt man in Analogie zu Beispiel 1m um und isoliert nach Aufarbeitung und Reinigung 76 mg (148 »mol, 92%) der Titelverbindung als farbloses Öl.
IR (Film): 3050, 3010, 2950, 2920, 2860, 1735, 1600, 1435, 1360, 1190, 1175, 1095, 975, 945, 885, 815, 780, 745 und 665 cm⁻¹.

### Beispiel 75:

### 7-[(1S,2R,5S)-2-(3-Methylbenzyloxymethyl)-5-fluor-cyclopentyl]-5(Z)-heptensäure:

36 mg (106 »mol) der nach Beispiel 74 dargestellten Verbindung verseift man in Analogie zu Beispiel 2 und isoliert nach Aufarbeitung und Reinigung 8,6 mg (26 »mol, 25%) der Titelverbindung als farbloses Öl.
IR (Film): 3600-2400, 3010, 2920, 2850, 1705, 1610, 1590, 1455, 1410, 1355, 1240, 1155, 1105, 1085, 780, 755 und 695 cm⁻¹.

### Beispiel 76:

### 7-[(1S,2R,3S,5R)-2-(3-Methylbenzyloxymethyl)-3,5-dihydroxy-cyclopentyl]-5(Z)-heptensäuremethylester:

87,2 mg (189 »mol) der nach Beispiel 72b dargestellten Verbindung setzt man in Analogie zu Beispiel 62 um und isoliert nach Aufarbeitung und Reinigung 63,4 mg (168 »mol, 89%) der Titelverbindung als farbloses Öl.
IR (Film): 3600-3100, 3010, 2950, 2930, 2860, 1735, 1610, 1440, 1360, 1245, 1160, 1100, 780, 745 und 695 cm⁻¹.

### Beispiel 77:

### 7-[(1S,2R,3S,5R)-2-(3-Methylbenzyloxymethyl)-3,5-dihydroxy-cyclopentyl]-5(Z)-heptensäure:

53 mg (141 »mol) der nach Beispiel 76 dargestellten Verbindung verseift man in Analogie zu Beispiel 2 und isoliert nach Aufarbeitung und Reinigung 40 mg (110 »mol, 79%) der Titelverbindung als farbloses Öl.
IR (Film): 3600-2400, 3010, 2930, 2860, 1710, 1610, 1590, 1455, 1430, 1405, 1355, 1245, 1155, 1100, 780, 745 und 695 cm⁻¹.

### Beispiel 78:

### 6-[(1R,2S,5R)-2-[3-(4-Fluorphenyl)-ureidomethyl]-5-fluor-cyclopentyl]-4(Z)-hexansäuremethylester:

Die Lösung von 80 mg (max. 136 »mol) des nach Beispiel 78a dargestellten Amins in 1 ml Dichlormethan versetzt man mit 21 »l 4-Fluorphenylisocyanat und rührt 2,5 Stunden bei 23°C. Man reinigt durch Chromatographie an drei analytischen Dünnschichtplatten. Als Laufmittel dient ein Gemisch aus n-Hexan und Ethylacetat, als Elutionsmittel Ethylacetat. Isoliert werden 25 mg (66 »mol, 48%) der Titelverbindung als farbloses Öl.
IR (Film): 3250-3450, 3050, 2950, 2850, 1730, 1640, 1595, 1555, 1515, 1490, 1430, 1325, 1235, 835, 735 und 705 cm⁻¹.

### Beispiel 78a:

### 6-[(1R,2S,5R)-2-Aminomethyl-5-fluor-cyclopentyl]-4(Z)-hexensäuremethylester:

Die Lösung von 747 mg (2,77 mmol) der nach Beispiel 78b dargestellten Verbindung in 24 ml Tetrahydrofuran versetzt man mit 874 mg Triphenylphosphin und erwärmt 3,5 Stunden unter einer Atmosphäre aus trockenem Argon auf 50°C. Anschließend versetzt man mit 3 mi Wasser und erhitzt 1 Stunde zum Rückfluß. Man engt ein, nimmt mit Dichlormethan auf, trocknet über Magnesiumsulfat und isoliert nach Filtration und Lösungsmittelabzug 1,62 g (max. 2,77 mmol) mit Triphenylphosphin und Triphenylphosphinoxid verunreinigtes Amin, das man ohne Reinigung weiter umsetzt.

### Beispiel 78b:

### 6-[(1R,2S,5R)-2-Azidomethyl-5-fluor-cyclopentyl]-4(Z)-hexensäuremethylester:

Die Lösung von 934 mg (3,04 mmol) des nach Beispiel 78c dargestellten Bromides in 67 ml Dimethylformamid versetzt man mit 839 mg Natriumazid und erwärmt 4 Stunden unter einer Atmosphäre aus trockenem Argon auf 60°C. Man gießt in Eiswasser, extrahiert mehrfach mit Diethylether, trocknet die organische Phase über Magnesiumsulfat und reinigt den nach Filtration und Lösungsmittelabzug erhaltenen Rückstand durch Chromatographie an ca. 200 ml feinem Kieselgel unter Verwendung eines Gradientensystems aus n-Hexan und Ethylacetat. Isoliert werden 747 mg (2,77 mmol, 91%) der Titelverbindung als farbloses Öl.
IR (Film): 2950, 2870, 2100, 1735, 1435, 1360, 1245 und 1160 cm⁻¹.

### Beispiel 78c:

### 6-[(1R,2S,5R)-2-Brommethyl-5-fluor-cyclopentyl]-4(Z)-hexensäuremethylester:

Die Lösung von 743 mg (3,04 mmol) des nach Beispiel 78d dargestellten Alkohols in 22 ml Acetonitril versetzt man mit 2,05 ml Collidin, 5,05 g Tetrabrommethan, 4,0 g Triphenylphosphin und rührt 17 Stunden bei 23°C unter einer Atmosphäre aus trockenem Argon. Man engt ein und reinigt den erhaltenen Rückstand durch Chromatographie an ca. 200 ml feinem Kieselgel unter Verwendung eines Gradientensystems aus n-Hexan und Ethylacetat. Isoliert werden 934 mg (3,04 mmol, 100%) der Titelverbindung als farbloses Öl.
IR (Film): 2950, 2860, 1735, 1435, 1360, 1245, 1220, 1160 und 950 cm⁻¹.

### Beispiel 78d:

### 6-[(1R,2S,5R)-2-Hydroxymethyl-5-fluor-cyclopentyl]-4(Z)-hexensäuremethylester (A) und 6-[(5S)-5-hydroxy-cyclopent-1-enyl]-4(Z)-hexensäuremethylester (B):

3,69 g des nach Beispiel 78e dargestellten Substanzgemisches setzt man in Analogie zu Beispiel 1g um und isoliert nach Aufarbeitung und Reinigung 943 mg (4,20 mmol 40%) der Titelverbindung B als unpolare Komponente, sowie 758 mg (3,10 mmol, 30%) der Titelverbindung A als polare Komponente, jeweils als farbloses Öl.
IR (Film) von A: 3200-3600, 3050, 2950, 2870, 1735, 1435, 1360, 1165, 1040, 945 und 875 cm⁻¹.
IR (Film) von B: 3200-3600, 3050, 2940, 2850, 1735, 1435, 1360, 1200, 1160 und 1025 cm⁻¹.

### Beispiel 78e:

### 6-[(1R,2S,5R)-2-tert.-Butyldiphenylsilyloxymethyl-5-fluor-cyclopentyl]-4(Z)-hexensäuremethylester und 6-[(5S)-5-(tert.-Butyldiphenylsilyloxymethyl)-cyclopent-1-enyl]-4(Z)-hexensäuremethylester:

5,0 g (10,4 mmol) der nach Beispiel 78f dargestellten Verbindung setzt man in Analogie zu Beispiel 1 um und isoliert nach Aufarbeitung und Reinigung 3,81 g eines Gemisches der beiden Titelverbindungen als farbloses Öl.

### Beispiel 78f:

### 6-[(1R,2S,5S)-2-tert.-Butyldiphenylsilyloxymethyl-5-hydroxy-cyclopentyl)-4(Z)-hexensäuremethylester:

62,6 g (max. 58,3mol) der nach Beispiel 78g dargestellten Verbindung setzt man in Analogie zu Beispiel 68b um und isoliert nach Aufarbeitung und Reinigung 26,8 g (55,7 mmol, 96%) der Titelverbindung als farbloses Öl.
IR (Film): 3600-3200, 3070, 3040, 2950, 2930, 2850, 1735, 1585, 1460, 1425, 1240, 1160, 1110, 1005, 820, 740 und 700 cm⁻¹.

### Beispiel 78g:

### 6-[(1R,2S,5S)-2-tert.-Butyldiphenylsilyloxymethyl-5-hydroxy-cyclopentyl]-4(Z)-hexensäure:

23,1 g (58,3 mmol) der nach Beispiel 1k dargestellten Verbindung setzt man in Analogie zu Beispiel 1j unter Verwendung von Carboxypropyltriphenylphosphoniumbromid um und isoliert nach Aufarbeitung 62,6 g der Titelverbindung als Rohprodukt, das ohne Reinigung weiter umgesetzt wird.

### Beispiel 79:

### 6-[(1R,2S,5R)-2-[3-(4-Fluorphenyl)-ureidomethyl]-5-fluor-cyclopentyl]-4(Z)-hexensäure:

25 mg (66 »mol) der nach Beispiel 78 dargestellten Verbindung verseift man in Analogie zu Beispiel 2 und isoliert nach Aufarbeitung und Reinigung 21 mg (57 »mol, 87%) der Titelverbindung als farbloses Öl.
¹H-NMR (CD₃OD): δ= 1,4-2,4(m,12H), 3,12-3,35(m,2H), 4,74(m,1H), 5,4-5,55(m,2H), 6,92-7,04(m,2H), 28-7,38(m,2H).

### Beispiel 80:

### 6-[(1R,2S,5R)-2-[3-(4-Phenylphenyl)-ureidomethyl]-5-fluor-cyclopentyl]-4(Z)-hexensäuremethylester:

80 mg (max. 136 »mol) der nach Beispiel 78a dargestellten Verbindung setzt man in Analogie zu Beispiel 78 unter Verwendung von 4-Phenyl-phenylisocyanat um und isoliert nach Aufarbeitung und Reinigung 25 mg (57 »mol, 42%) der Titelverbindung als farbloses Öl.
IR (Film): 32003450, 3030, 2950, 2860, 1735, 1645, 1590, 1550, 1485, 1310, 1265, 1230, 835, 765, 735 und 700 cm⁻¹.

### Beispiel 81:

### 6-[(1R,2S,5R)-2-[3-(4-Phenylphenyl)-ureidomethyl]-5-fluor-cyclopentyl]-4(Z)-hexansäure:

25 mg (57 »mol) der nach Beispiel 80 dargestellten Verbindung verseift man in Analogie zu Beispiel 2 und isoliert nach Aufarbeitung und Reinigung 14 mg (33 »mol, 58%) der Titelverbindung als farbloses Öl.
¹H-NMR (CD₃OD): δ= 1,5-2,4(m,12H), 3,17-3,35(m,2H), 4,78(m,1H), 5,4-5,55(m,2H), 7,22-7,6(m,9H).

### Beispiel 82:

### 6-[(1R,2S,5R)-2-[3-(4-Methylphenyl)-ureidomethyl]-5-fluor-cyclopentyl]-4(Z)-hexensäuremethylester:

80 mg (max. 136 »mol) der nach Beispiel 78a dargestellten Verbindung setzt man in Analogie zu Beispiel 78 unter Verwendung von 4-Methylphenylisocyanat um und isoliert nach Aufarbeitung und Reinigung 28 mg (74 »mol, 55%) der Titelverbindung als farbloses Öl.
IR (Film): 3250-3450, 3050, 2950, 2860, 1730, 1640, 1600, 1555, 1515, 1260, 815, 735 und 705 cm⁻¹.

### Beispiel 83:

### 6-[(1R,2S,5R)-2-[3-(4-Methylphenyl)-ureidomethyl]-5-fluor-cyclopentyl]-4(Z)-hexensäure:

28 mg (74 »mol) der nach Beispiel 82 dargestellten Verbindung verseift man in Analogie zu Beispiel 2 und isoliert nach Aufarbeitung und Reinigung 14 mg (39 »mol, 52%) der Titelverbindung als farbloses Öl.
¹H-NMR (CD₃OD): δ= 1,45-2,4(m,15H), 3,12-3,34(m,2H), 4,76(m,1H), 5,4-5,55(m,2H), 7,06(d,2H), 7,21(d,2H).

### Beispiel 84:

### 6-[(1R,2S,5R)-2-[3-(3-Chlorphenyl)-ureidomethyl]-5-fluor-cyclopentyl]-4(Z)-hexensäuremethylester:

80 mg (max. 136 »mol) der nach Beispiel 78a dargestellten Verbindung setzt man in Analogie zu Beispiel 78 unter Verwendung von 3-Chlorphenylisocyanat um und isoliert nach Aufarbeitung und Reinigung 25 mg (63 »mol, 46%) der Titelverbindung als farbloses Öl.
IR (Film): 3200-3450, 3040, 2950, 2860, 1730, 1650, 1590, 1550, 1480, 1435, 1420, 1300, 1265, 1230, 1165, 775, 735, 700 und 680 cm⁻¹.

### Beispiel 85:

### 6-[(1R,2S,5R)-2-[3-(3-Chlorphenyl)-ureidomethyl]-5-fluor-cyclopentyl]-4(Z)-hexensäure:

25 mg (63 »mol) der nach Beispiel 84 dargestellten Verbindung verseift man in Analogie zu Beispiel 2 und isoliert nach Aufarbeitung und Reinigung 10 mg (26 »mol, 41%) der Titelverbindung als farbloses Öl.
¹H-NMR (CD₃OD): δ= 1,45-2,4(m,12H), 3,15-3,35(m,2H), 4,76(m,1H), 5,4-5,57(m,2H), 6,9-6,98(m,1H), 7,15-7,24(m,2H), 7,57(s,1H).

### Beispiel 86:

### 6-[(1R,2S,5R)-2-[3-Phenyl-ureidomethyl]-5-fluor-cyclopentyl]-4(Z)-hexensäuremethylester:

80 mg (max. 136 »mol) der nach Beispiel 78a dargestellten Verbindung setzt man in Analogie zu Beispiel 78 unter Verwendung von Phenylisocyanat um und isoliert nach Aufarbeitung und Reinigung 24 mg (66 »mol, 49%) der Titelverbindung als farbloses Öl.
IR (Film): 3200-3450, 3050, 2950, 2860, 1730, 1645, 1595, 1550, 1495, 1435, 1310, 1230, 1170, 750, 735 und 695 cm⁻¹.

### Beispiel 87:

### 6-[(1R,2S,5R)-2-[3-Phenyl-ureidomethyl]-5-fluor-cyclopentyl]-4(Z)-hexensäure:

24 mg (66 »mol) der nach Beispiel 86 dargestellten Verbindung verseift man in Analogie zu Beispiel 2 und isoliert nach Aufarbeitung und Reinigung 16 mg (46 »mol, 70%) der Titelverbindung als farbloses Öl.
¹H-NMR (CD₃OD): δ= 1,45-2,4(m,12H), 3,12-3,35(m,2H), 4,75(m,1H), 5,4-5,55(m,2H), 6,96(t,1H), 7,2-7,38(m,4H).

### Beispiel 88:

### 6-[(1R,2S,5R)-2-[3-(3,4-Dichlorphenyl)-ureidomethyl]-5-fluor-cyclopentyl]-4(Z)-hexensäuremethylester:

80 mg (max. 136 »mol) der nach Beispiel 78a dargestellten Verbindung setzt man in Analogie zu Beispiel 78 unter Verwendung von 3,4-Dichlorphenylisocyanat um und isoliert nach Aufarbeitung und Reinigung 26 mg (60 »mol, 44%) der Titelverbindung als farbloses Öl.
IR (Film): 3500, 3200-3450, 3010, 2950, 2860, 1730, 1650, 1585, 1540, 1470, 1375, 1265, 1230, 1130, 1025, 815, 735 und 700 cm⁻¹.

### Beispiel 89:

### 6-[(1R,2S,5R)-2-[3-(3,4-Dichlorphenyl)-ureidomethyl]-5-fluor-cyclopentyl]-4(Z)-hexensäure:

26 mg (60 »mol) der nach Beispiel 88 dargestellten Verbindung verseift man in Analogie zu Beispiel 2 und isoliert nach Aufarbeitung und Reinigung 15 mg (36 »mol, 60%) der Titelverbindung als farbloses Öl.
¹H-NMR (CD₃OD): δ= 1,5-2,4(m,12H), 3,16-3,35(m,2H), 4,75(m,1H), 5,4-5,55(m,2H), 7,21(dd,1H), 7,35(d,1H), 7,71(d,1H).

### Beispiel 90:

### 6-[(1R,2S,5R)-2-[3-(4-Nitrophenyl)-ureidomethyl]-5-fluor-cyclopentyl]-4(Z)-hexensäuremethylester:

100 mg (max. 170 »mol) der nach Beispiel 78a dargestellten Verbindung setzt man in Analogie zu Beispiel 78 unter Verwendung von 4-Nitrophenylisocyanat um und isoliert nach Aufarbeitung und Reinigung 32 mg (79 »mol, 46%) der Titelverbindung als gelbes Öl.
IR (Film): 3200-3450, 3010, 2950, 2870, 1730, 1700, 1670, 1610, 1600, 1550, 1500, 1330, 1300, 1230, 1175, 1110, 850, 735 und 700 cm⁻¹.

### Beispiel 91:

### 6-[(1R,2S,5R)-2-[3-(4-Nitrophenyl)-ureidomethyl]-5-fluor-cyclopentyl]-4(Z)-hexensäure:

32 mg (79 »mol) der nach Beispiel 90 dargestellten Verbindung verseift man in Analogie zu Beispiel 2 und isoliert nach Aufarbeitung und Reinigung 31 mg (79 »mol, 100%) der Titelverbindung als gelbes Öl.
¹H-NMR (CD₃OD): δ= 1,5-2,4(m,12H), 3,18-3,35(m,2H), 4,76(m,1H), 5,4-5,55(m,2H), 7,58(d,2H), 8,14(d,2H).

### Beispiel 92:

### 6-[(1R,2S,5R)-2-[3-(4-Chlorphenyl)-ureidomethyl]-5-fluor-cyclopentyl]-4(Z)-hexensäuremethylester:

100 mg (max. 170 »mol) der nach Beispiel 78a dargestellten Verbindung setzt man in Analogie zu Beispiel 78 unter Verwendung von 4-Chlorphenylisocyanat um und isoliert nach Aufarbeitung und Reinigung 38 mg (101 »mol, 59%) der Titelverbindung als farblosen Feststoff.
IR (KBr): 3200-3450, 3030, 2960, 2850, 1735, 1650, 1590, 1545, 1470, 1420, 1375, 1265, 1230, 1160, 780, 735 und 700 cm⁻¹.

### Beispiel 93:

### 6-[(1R,2S,5R)-2-[3-(4-Chlorphenyl)-ureidomethyl]-5-fluor-cyclopentyl]-4(Z)-hexensäure:

38 mg (101 »mol) der nach Beispiel 92 dargestellten Verbindung verseift man in Analogie zu Beispiel 2 und isoliert nach Aufarbeitung und Reinigung 33 mg (86 »mol, 85%) der Titelverbindung als farbloses Öl.
¹H-NMR (CD₃OD): δ= 1,45-2,4(m,12H), 3,15-3,35(m,2H), 4,76(m,1H), 5,4-5,55(m,2H), 7,21(dd,2H), 7,35(dd,2H).

### Beispiel 94:

### 6-[(1R,2S,5R)-2-[3-(4-Aminophenyl)-ureidomethyl]-5-fluor-cyclopentyl]-hexansäure:

Die Lösung von 15 mg (38 mmol) der nach Beispiel 91 dargestellten Verbindung in 1 ml Ethylacetat versetzt man mit 5 mg Palladium auf Kohle (10%ig) und hydriert bei 1 at Wasserstoff. Nach Aufnahme der theoretischen Menge Wasserstoff wird filtriert, eingeengt und der Rückstand an einer analytischen Dünnschichtplatte chromatographisch gereinigt. Als Laufmittel dient ein Gemisch aus Dichlormethan und Ethanol, als Elutionsmittel ein Gemisch aus Trichlormethan und Isopropanol. Isoliert werden 13 mg (36 »mol, 94%) der Titelverbindung als wachsartigen Feststoff.
¹H-NMR (CD₃OD): δ= 1,2-2,0(m,14H), 2,26(t,2H), 3,1-3,3(m,2H), 4,76(m,1H), 6,71(d,2H), 7,08(d,2H).

### Beispiel 95:

### 6-[(1R,2S,5R)-2-[3-(4-Chlorphenyl)-ureidomethyl]-5-fluor-cyclopentyl]-hexansäure:

16 mg (44 »mol) der nach Beispiel 93 dargestellten Verbindung setzt man in Analogie zu Beispiel 94 um und isoliert nach Aufarbeitung und Reinigung 16 mg (42 »mol, 94%) der Titelverbindung als farblosen Feststoff.
¹H-NMR (Aceton d₆): δ= 1,25-1,9(m, 14H), 2,28(t,2H), 2,5-3,5(s,1H), 3,18(m,1H), 3,32(m,1H), 4,77(m,1H), 5,99(t,1H), 7,21(d,2H), 7,5(d,2H), 8,08(s,1H).

### Beispiel 96:

### 6-[(1R,2S,5R)-2-[(2-Nitrophenyl)-sulfonylaminomethyl]-5-fluor-cyclopentyl]-4(Z)-hexansäuremethylester:

147 mg (max. 250 »mol) der nach Beispiel 78a dargestellten Verbindung setzt man in Analogie zu Beispiel 1b unter Verwendung von 2-Nitrobenzolsulfonsäurechlorid um und isoliert nach Aufarbeitung und Reinigung 54 mg (126 »mol, 50%) der Titelverbindung als farbloses Öl.
IR (Film): 3200-3400, 3100, 3010, 2950, 2920, 2860, 1730, 1590, 1540, 1435, 1415, 1360, 1165, 1070, 855, 780, 740 und 655 cm⁻¹.

### Beispiel 97:

### 6-[(1R,2S,5R)-2-[(2-Nitrophenyl)-sulfonylaminomethyl]-5-fluor-cyclopentyl]-4(Z)-hexensäure:

54 mg (126 »mol) der nach Beispiel 96 dargestellten Verbindung verseift man in Analogie zu Beispiel 2 und isoliert nach Aufarbeitung und Reinigung 48 mg (116 »mol, 92%) der Titelverbindung als farbloses Öl.
¹H-NMR (CD₃OD): δ= 1,4-2,4(m,12H), 2,99(dd,1H), 3,15(dd,1H), 4,7(m,1H), 5,33-5,5-(m,2H), 7,38-7,48(m,3H), 8,03-8,1(m,1H).

### Beispiel 98:

### 6-[(1R,2S,5R)-2-[(4-Methylphenyl)-sulfonylaminomethyl]-5-fluor-cyclopentyl]-4(Z)-hexensäuremethylester:

147 mg (max. 250 »mol) der nach Beispiel 78a dargestellten Verbindung setzt man in Analogie zu Beispiel 1b unter Verwendung von Tosylchlorid um und isoliert nach Aufarbeitung und Reinigung 83 mg (209 »mol, 84%) der Titelverbindung als farbloses Öl.
IR (Film): 3200-3400, 3010, 2950, 2870, 1730, 1600, 1435, 1330, 1285, 1160, 1090, 1075, 815 und 665 cm⁻¹.

### Beispiel 99:

### 6-[(1R,2S,5R)-2-[(4-Methylphenyl)-sulfonylaminomethyl]-5-fluor-cyclopentyl]-4(Z)-hexensäure:

83 mg (209 »mol) der nach Beispiel 98 dargestellten Verbindung verseift man in Analogie zu Beispiel 2 und isoliert nach Aufarbeitung und Reinigung 69 mg (180 »mol, 86%) der Titelverbindung als farblosen Feststoff.
IR (Film): (CD₃OD): δ= 1,4-1,55(m,1H), 1,63-2,38(m,11H), 2,42(s,3H), 2,77(m,1H), 3,92(m,1H), 4,7(m,1H), 5,33-5,5(m,2H), 7,38(d,2H), 7,72(d,2H).

### Beispiel 100:

### 6-[(1R,2S,5R)-2-[(3,4-Dichlorphenyl)-sulfonylaminomethyl]-5-fluor-cyclopentyl]-4(Z)-hexensäuremethylester:

147 mg (max. 250 »mol) der nach Beispiel 78a dargestellten Verbindung setzt man in Analogie zu Beispiel 1b unter Verwendung von 3,4-Dichlorbenzolsulfonsäurechlorid um und isoliert nach Aufarbeitung und Reinigung 81 mg (179 »mol, 72%) der Titelverbindung als farbloses Öl.
IR (Film): 3090, 3010, 2950, 2850, 1730, 1565, 1450, 1380, 1170, 1140, 1095, 1030, 885, 825, 780, 735, 710, 675 und 625 cm⁻¹.

### Beispiel 101:

### 6-[(1R,2S,5R)-2-[(3,4-Dichlorphenyl)-sulfonylaminomethyl)-5-fluor-cyclopentyl]-4(Z)-hexensäure:

81 mg (179 »mol) der nach Beispiel 100 dargestellten Verbindung verseift man in Analogie zu Beispiel 2 und isoliert nach Aufarbeitung und Reinigung 68 mg (155 »mol, 87%) der Titelverbindung als farbloses Öl.
¹H-NMR (CD₃OD): δ= 1,4-2,4(m,12H), 2,83(dd,1H), 2,97(dd,1H), 4,7(m,1H), 5,35-5,5(m,2H), 7,73(m,2H), 7,98(d,1H).

### Beispiel 102:

### 6-[(1R,2S,5R)-2-[(4-Fluorphenyl)-sulfonylaminomethyl]-5-fluor-cyclopentyl]-4(Z)-hexensäuremethylester:

147 mg (max. 250 »mol) der nach Beispiel 78a dargestellten Verbindung setzt man in Analogie zu Beispiel 1b unter Verwendung von 4-Fluorbenzolsulfonsäurechlorid um und isoliert nach Aufarbeitung und Reinigung 56 mg (139 »mol, 56%) der Titelverbindung als farbloses Öl.
IR (Film): 3200-3400, 3010, 2950, 2860, 1730, 1600, 1490, 1430, 1330, 1235, 1160, 1090, 840 und 670 cm⁻¹.

### Beispiel 103:

### 6-[(1R,2S,5R)-2-[(4-Fluorphenyl)-sulfonylaminomethyl]-5-fluor-cyclopentyl]-4(Z)-hexensäure:

56 mg (139 »mol) der nach Beispiel 102 dargestellten Verbindung verseift man in Analogie zu Beispiel 2 und isoliert nach Aufarbeitung und Reinigung 51 mg (132 »mol, 95%) der Titelverbindung als farbloses Öl.
¹H-NMR (CD₃OD): δ= 1,4-2,36(m,12H), 2,8(dd,1H), 2,95(dd,1H), 4,7(m,1H), 5,33-5,5(m,2H), 7,3(m,2H), 7,9(m,2H).

### Beispiel 104:

### 6-[(1R,2S,5R)-2-[Phenylsulfonylaminomethyl)-5-fluor-cyclopentyl]-4(Z)-hexensäuremethylester:

147 mg (max. 250 »mol) der nach Beispiel 78a dargestellten Verbindung setzt man in Analogie zu Beispiel 1b unter Verwendung von Benzolsulfonsäurechlorid um und isoliert nach Aufarbeitung und Reinigung 51 mg (133 »mol, 53%) der Titelverbindung als farbloses Öl.
IR (Film): 3200-3400, 3060, 3010, 2950, 2860, 1730, 1445, 1325, 1160, 1095, 755, 720 und 690 cm⁻¹.

### Beispiel 105:

### 6-[(1R,2S,5R)-2-[Phenylsulfonylaminomethyl]-5-fluor-cyclopentyl]-4(Z)-hexensäure:

51 mg (133 »mol) der nach Beispiel 104 dargestellten Verbindung verseift man in Analogie zu Beispiel 2 und isoliert nach Aufarbeitung und Reinigung 43 mg (116 »mol, 88%) der Titelverbindung als farbloses Öl.
¹H-NMR (CD₃OD): δ= 1,4-2,4(m,12H), 2,78(dd,1H), 2,93(dd,1H), 4,7(m,1H), 5,33-5,5(m,2H), 7,52-7,65(m,3H), 7,86(m,2H).

### Beispiel 106:

### 6-[(1R,2S,5R)-2-[(4-Chlorphenyl)-sulfonylaminomethyl]-5-fluor-cyclopentyl]-4(Z)-hexensäuremethylester:

147 mg (max. 250 »mol) der nach Beispiel 78a dargestellten Verbindung setzt man in Analogie zu Beispiel 1b unter Verwendung von 4-Chlorbenzolsulfonsäurechlorid um und isoliert nach Aufarbeitung und Reinigung 60 mg (143 »mol, 57%) der Titelverbindung als farbloses Öl.
IR (Film): 3200-3400, 3090, 3010, 2950, 2870, 1730, 1585, 1470, 1435, 1330, 1160, 1090, 1010, 830, 750 und 620 cm⁻¹.

### Beispiel 107:

### 6-[(1R,2S,5R)-2-[(4-Chlorphenyl)-sulfonylaminomethyl]-5-fluor-cyclopentyl]-4(Z)-hexensäure:

60 mg (143 »mol) der nach Beispiel 106 dargestellten Verbindung verseift man in Analogie zu Beispiel 2 und isoliert nach Aufarbeitung und Reinigung 45 mg (111 »mol, 78%) der Titelverbindung als farbloses Öl.
¹H-NMR (CD₃OD): δ= 1,4-2,4(m,12H), 2,8(dd,1H), 2,95(dd,1H), 4,7(m,1H), 5,33-5,5(m,2H), 7,58(d,2H), 7,82(d,2H).

### Beispiel 108:

### 6-[(1R,2S,5R)-2-[(4-Methylphenyl)-sulfonylaminomethyl]-5-fluor-cyclopentyl]-hexansäure:

20 mg (52 »mol) der nach Beispiel 99 dargestellten Verbindung setzt man in Analogie zu Beispiel 94 um und isoliert nach Aufarbeitung und Reinigung 20 mg (52 »mol, 99%) der Titelverbindung als farbloses Öl.
¹H-NMR (CD₃OD): δ= 1,2-1,9(m,14H), 2,28(t,2H), 2,42(d,3H) 2,76(dd,1H), 2,92(dd,1H), 4,68(m,1H), 7,48(d,2H), 7,72(d,2H).

### Beispiel 109:

### 6-[(1R,2S,5R)-2-[Phenyl-sulfonylaminomethyl]-5-fluor-cyclopentyl]-hexansäure:

19 mg (51 »mol) der nach Beispiel 105 dargestellten Verbindung setzt man in Analogie zu Beispiel 94 um und isoliert nach Aufarbeitung und Reinigung 19 mg (51 »mol, 100%) der Titelverbindung als farbloses Öl.
¹H-NMR (CD₃OD): δ= 1,2-1,9(m,14H), 2,28(t,2H), 2,77(dd,1H), 2,92(dd,1H), 4,7(m,1H), 7,52-7,66(m,3H), 7,85(m,2H).

### Beispiel 110:

### 6-[(1R,2S,5R)-2-[(4-Chlorphenyl)-sulfonylaminomethyl]-5-fluor-cyclopentyl]-hexansäure:

19 mg (47 »mol) der nach Beispiel 107 dargestellten Verbindung setzt man in Analogie zu Beispiel 94 um und isoliert nach Aufarbeitung und Reinigung 17 mg (42 »mol, 89%) der Titelverbindung als farbloses Öl.
¹H-NMR (CD₃OD): δ= 1,2-1,9(m,14H), 2,29(t,2H), 2,81(dd,1H), 2,95(dd,1H), 4,72(m,1H), 7,58(d,2H), 7,82(d,2H).

### Beispiel 111:

### 6-[(1R,2S,5R)-2-[(4-Fluorphenyl)-sulfonylaminomethyl]-5-fluor-cyclopentyl]-hexansäure:

19 mg (49 »mol) der nach Beispiel 103 dargestellten Verbindung setzt man in Analogie zu Beispiel 94 um und isoliert nach Aufarbeitung und Reinigung 19 mg (49 »mol, 100%) der Titelverbindung als farbloses Öl.
¹H-NMR (CD₃OD): δ= 1,2-1,96(m,14H), 2,28(t,2H), 2,78(dd,1H), 2,93(dd,1H), 4,72(m,1H), 7,3(m,2H), 7,9(m,2H).

### Beispiel 112:

### 6-[(1R,2S,5R)-2-[3-(4-Fluorphenyl)-ureidomethyl]-5-fluor-cyclopentyl]-hexansäure:

10 mg (27 »mol) der nach Beispiel 79 dargestellten Verbindung setzt man in Analogie zu Beispiel 94 um und isoliert nach Aufarbeitung und Reinigung 10 mg (27 »mol, 100%) der Titelverbindung als farbloses Öl.
¹H-NMR (CD₃OD): δ= 1,25-2,0(m,14H), 2,26(t,2H), 3,1-3,32(m,2H), 4,76(m,1H), 6,97(m,2H), 7,31(m,2H).

### Beispiel 113:

### 7-[(1R,2S,5R)-2-[(4-Chlorphenyl)-sulfonylaminomethyl]-5-fluor-cyclopentyl]-5(Z)-heptensäuremethylester:

129 mg (max. 245 »mol) der nach Beispiel 113a dargestellten Verbindung setzt man in Analogie zu Beispiel 1b unter Verwendung von 4-Chlorbenzolsulfonsäurechlorid um und isoliert nach Aufarbeitung und Reinigung 49 mg (113 »mol, 46%) der Titelverbindung als farbloses Öl.
IR (Film): 3200-3400, 3010, 2950, 2860, 1730, 1585, 1435, 1330, 1160, 1090, 830, 750, 735, 700 und 620 cm⁻¹.

### Beispiel 113a:

### 7-[(1R,2S,5R)-2-Aminomethyl-5-fluor-cyclopentyl]-5(Z)-heptensäuremethylester:

416 mg (1,47 mmol) der nach Beispiel 113b dargestellten Verbindung setzt man in Analogie zu Beispiel 78a um und isoliert nach Aufarbeitung und Reinigung 776 mg (max. 1,47 mmol, ca. 50%ig) der Titelverbindung als farbloses Öl.

### Beispiel 113b:

### 7-[(1R,2S,5R)-2-Azidomethyl-5-fluor-cyclopentyl]-5(Z)-heptensäuremethylester:

502 mg (1,56 mmol) der nach Beispiel 113c dargestellten Verbindung setzt man in Analogie zu Beispiel 78b um und isoliert nach Aufarbeitung und Reinigung 416 mg (1,47 »mol, 94%) der Titelverbindung als farbloses Öl.
IR (Film): 2950, 2870, 2100, 1735, 1435, 1360, 1245 und 1160 cm⁻¹.

### Beispiel 113c:

### 7-[(1R,2S,5R)-2-Brommethyl-5-fluor-cyclopentyl]-5(Z)-heptensäuremethylester:

410 mg (1,59 mmol) der nach Beispiel 113c dargestellten Verbindung setzt man in Analogie zu Beispiel 78c um und isoliert nach Aufarbeitung und Reinigung 502 mg (1,56 mmol, 98%) der Titelverbindung als farbloses Öl.
IR (Film): 2950, 2870, 1735, 1435, 1360, 1245, 1225, 1170 und 950 cm⁻¹.

### Beispiel 113d:

### 7-[(1R,2S,5R)-2-Hydroxymethyl-5-fluor-cyclopentyl]-5(Z)-heptensäuremethylester (A) und 7-[(5S)-5-hydroxy-cyclopent-1-enyl]-5(Z)-heptensäuremethylester (B):

3,75 g des nach Beispiel 113e dargestellten Substanzgemisches setzt man in Analogie zu Beispiel 78d um und isoliert nach Aufarbeitung und Reinigung 941 mg (3,95 mmol, 38%) der Titelverbindung B als unpolare Komponente sowie 820 mg (3,17 mmol, 31%) der Titelverbindung A als polare Komponente, jeweils als farbloses Öl.
IR (Film) von A: 3200-3600, 3050, 2950, 2870, 1735, 1435, 1360, 1165, 1040, 945 und 875 cm⁻¹.
IR (Film) von B: 3200-3600, 3050, 2950, 2860, 1735, 1435, 1360, 1200, 1160 und 1025 cm⁻¹.

### Beispiel 113e:

### 7-[(1R,2S,5R)-2-tert.-Butyldiphenylsilyloxymethyl-5-fluor-cyclopentyl]-5(Z)-heptensäuremethylester und 7-[(5S)-5-(tert.-Butyldiphenylsilyloxymethyl)-cyclopent-1-enyl]-5(Z)-heptensäuremethylester:

5,08 g (10,3 mmol) der nach Beispiel 1i dargestellten Verbindung setzt man in Analogie zu Beispiel 1g um und isoliert nach Aufarbeitung und Reinigung 3,75 g eines Gemisches der beiden Titelverbindungen als farbloses Öl.

### Beispiel 114:

### 7-[(1R,2S,5R)-2-[(4-Chlorphenyl)-sulfonylaminomethyl]-5-fluor-cyclopentyl]-5(Z)-heptensäure:

49 mg (113 »mol) der nach Beispiel 113 dargestellten Verbindung verseift man in Analogie zu Beispiel 2 und isoliert nach Aufarbeitung und Reinigung 47 mg (112 »mol, 99%) der Titelverbindung als farbloses Öl.
¹H-NMR (CDCl₃): δ= 1,35-2,2(m,12H), 2,38(t,2H), 2,93(t,2H), 4,79(m,1H), 5,35-5,58(m,3H), 7,48(d,2H), 7,79(d,2H).

### Beispiel 115:

### 7-[(1R,2S,5R)-2-[(4⁻Fluorphenyl)-sulfonylaminomethyl]-5-fluor-cyclopentyl]-5(Z)-heptensäuremethylester:

129 mg (max. 245 »mol) der nach Beispiel 113a dargestellten Verbindung setzt man in Analogie zu Beispiel 1b unter Verwendung von 4-Fluorbenzolsulfonsäurechlorid um und isoliert nach Aufarbeitung und Reinigung 50 mg (120 »mol, 49%) der Titelverbindung als farbloses Öl.
IR (Film): 3200-3400, 3060, 3010, 2940, 2860, 1730, 1590, 1490, 1435, 1330, 1235, 1165, 1150, 1090, 840, 735, 700 und670 cm⁻¹.

### Beispiel 116:

### 7-[(1R,2S,5R)-2-[(4⁻Fluorphenyl)-sulfonylaminomethyl]-5-fluor-cyclopentyl]-5(Z)-heptensäure:

50 mg (120 »mol) der nach Beispiel 115 dargestellten Verbindung verseift man in Analogie zu Beispiel 2 und isoliert nach Aufarbeitung und Reinigung 44 mg (110 »mol, 91%) der Titelverbindung als farbloses Öl.
¹H-NMR (CDCl₃): δ= 1,35-2,2(m,12H), 2,38(t,2H), 2,92(t,2H), 4,77(m,1H), 5,35-5,58(m,3H), 7,2(m,2H), 7,79(m,2H).

### Beispiel 117:

### 7-[(1R,2S,5R)-2-[3-(4-Chlorphenyl)-ureidomethyl]-5-fluor-cyclopentyl]-5(Z)-heptensäuremethylester:

129 mg (max. 245 »mol) der nach Beispiel 113a dargestellten Verbindung setzt man in Analogie zu Beispiel 78 unter Verwendung von 4-Chlorphenylisocyanat um und isoliert nach Aufarbeitung und Reinigung 60 mg (146 »mol, 60%) der Titelverbindung als farbloses Öl.
IR (Film): 3200-3400, 3040, 2950, 2850, 1730, 1650, 1595, 1545, 1470, 1420, 1375, 1260, 1225, 1160, 785, 735 und 700 cm⁻¹.

### Beispiel 118:

### 7-[(1R,2S,5R)-2-[3-(4-Chlorphenyl)-ureidomethyl]-5-fluor-cyclo pentyl]-5(Z)-heptensäure:

60 mg (146 »mol) der nach Beispiel 117 dargestellten Verbindung verseift man in Analogie zu Beispiel 2 und isoliert nach Aufarbeitung und Reinigung 42 mg (106 »mol, 72%) der Titelverbindung als farbloses Öl.
¹H-NMR (CDCl₃): δ= 1,38-2,2(m,12H), 2,36(t,2H), 3,2(t,2H), 4,78(m,1H), 5,35-5,48(m,2H), 5,58(t,1H), 7,15-7,25(m,4H), 7,49(s,1H).

### Beispiel 119:

### 7-[(1R,2S,5R)-2-[3-(4-Nitrophenyl)-ureidomethyl]-5-fluor-cyclopentyl]-5(Z)-heptensäuremethylester:

129 mg (max. 245 »mol) der nach Beispiel 113a dargestellten Verbindung setzt man in Analogie zu Beispiel 78 unter Verwendung von 4-Nitrophenylisocyanat um und isoliert nach Aufarbeitung und Reinigung 53 mg (126 »mol, 51%) der Titelverbindung als farbloses Öl.
IR (Film): 3200-3400, 3080, 3010, 2950, 2860, 1730, 1700, 1670, 1610, 1600, 1550, 1500, 1325, 1300, 1230, 1175, 1110, 850, 735 und 700 cm⁻¹.

### Beispiel 120:

### 7-[(1R,2S,5R)-2-[3-(4-Nitrophenyl)-ureidomethyl]-5-fluor-cyclo pentyl]-5(Z)-heptensäure:

53 mg (126 »mol) der nach Beispiel 119 dargestellten Verbindung verseift man in Analogie zu Beispiel 2 und isoliert nach Aufarbeitung und Reinigung 44 mg (105 »mol, 83%) der Titelverbindung als gelbes Öl.
¹H-NMR (CDCl₃): δ= 1,32-2,15(m,12H), 2,29(t,2H), 3,12(t,2H), 4,72(m,1H), 5,25-5,4(m,2H), 5,5(t,1H), 7,08-7,15(m,4H), 7,4(s,1H).

### Beispiel 121:

### 7-[(1R,25,5R)-2-[(E/Z)-3-(3,4-Dichlorphenyl)-ureidoiminomethyl]-5-fluor-cyclopentyl]-5(Z)-heptensäuremethylester:

55 mg (max. 199 »mol) der nach Beispiel 121a dargestellten Verbindung setzt man in Analogie zu Beispiel 62b unter Verwendung von 4-(3,4-Dichlorphenyl)semicarbazid-Hydrochlorid um und isoliert nach Aufarbeitung und Reinigung 75 mg (164 »mol, 82%) der Titelverbindung als farbloses Öl.
IR (Film): 3360, 3200, 3100, 2940, 1730, 1690, 1580, 1520, 1470, 1390, 1290, 1220, 1130, 1025, 950, 875, 815, 740 und 690 cm⁻¹.

### Beispiel 121a:

### 7-[(1R,2S,5R)-2-Formyl-5-fluor-cyclopentyl]-5(Z)-heptensäuremethylester:

410 mg (1,59 mmol) der nach Beispiel 113d dargestellten Verbindung A setzt man in Analogie zu Beispiel 50b um und isoliert nach Aufarbeitung 440 mg (max. 1,59 mmol) der Titelverbindung als farbloses Öl, das ohne Reinigung weiter umgesetzt wird.

### Beispiel 122:

### 7-[(1R,2S,5R)-2-[(E/Z)-3-(3,4-Dichlorphenyl)-ureidoiminomethyl]-5-fluor-cyclopentyl]-5(Z)-heptensäure:

38 mg (83 »mol) der nach Beispiel 121 dargestellten Verbindung verseift man in Analogie zu Beispiel 2 und isoliert nach Aufarbeitung und Reinigung 28 mg (63 »mol, 76%) der Titelverbindung als farbloses Öl.
¹H-NMR (CDCl₃): δ= 1,62-2,5(m,14H), 2,77-2,88(m,0,25H), 4,78(m,1H), 5,2-5,3(m,0,25H), 5,42-5,58(m,1,75H), 6,52(d,0,25H), 7,21(d,0,75H), 7,23-7,4(m,2H), 7,72(dd,1H), 8,02(s,0,75H), 8,28(s,0,25H), 9,62(s,0,75H), 10,01(s,0,25H).

### Beispiel 123:

### 7-[(1R,2S,5R)-2-[(E/Z)-3-(4-Chlorphenyl)-ureidoiminomethyl]-5-fluor-cyclopentyl]-5(Z)-heptensäuremethylester:

165 mg (max. 596 »mol) der nach Beispiel 121a dargestellten Verbindung setzt man in Analogie zu Beispiel 62b unter Verwendung von 4-(4-Chlorphenyl)semicarbazid-Hydrochlorid um und isoliert nach Aufarbeitung und Reinigung 198 mg (467 »mol, 78%) der Titelverbindung als farbloses Öl.
IR (Film): 3370, 3200, 3100, 2940, 2870, 1730, 1685, 1590, 1525, 1400, 1310, 1225, 1090, 950, 825 und 740 cm⁻¹.

### Beispiel 124: 7-[(1R,2S,5R)-2-[(E/Z)-3-(4-Chlorphenyl)-ureidoiminomethyl]-5-fluor-cyclopentyl]-5(Z)-

### heptensäure:

60 mg (142 »mol) der nach Beispiel 123 dargestellten Verbindung verseift man in Analogie zu Beispiel 2 und isoliert nach Aufarbeitung und Reinigung 50 mg (122 »mol, 86%) der Titelverbindung als farbloses Öl.
¹H-NMR (CDCl₃): δ= 1,62-2,5(m,14H), 2,77-2,88(m,0,25H), 4,76(m,1H), 5,2-5,3(m,0,25H), 5,42-5,58(m,1,75H), 6,52(d,0,25H), 7,2(d,0,75H), 7,28(m,2H), 7,43(m,2H), 8,02(s,0,75H), 8,24(s,0,25H), 9,64(s,0,75H), 9,98(s,0,25H).

### Beispiel 125:

### 7-[(1R,2S,5R)-2-[3-(4-Nitrophenyl)-ureidomethyl]-5-fluor-cyclopentyl]-heptansäure:

22 mg (56 »mol) der nach Beispiel 120 dargestellten Verbindung setzt man in Analogie zu Beispiel 94 um und isoliert nach Aufarbeitung und Reinigung 18 mg (44 »mol, 79%) der Titelverbindung als gelbes Öl.
¹H-NMR (CD₃OD): δ= 1,25-2,(m,16H), 2,23(t,2H), 3,12-3,36(m,2H), 4,76(m,1H), 7,58 (d,2H), 8,13(d,2H).

### Beispiel 126:

### 7-[(1R,2S,5R)-2-[3-(4-Chlorphenyl)-ureidomethyl]-5-fluor-cyclo pentyl]-heptansäure:

20 mg (52 »mol) der nach Beispiel 118 dargestellten Verbindung setzt man in Analogie zu Beispiel 94 um und isoliert nach Aufarbeitung und Reinigung 20 mg (50 »mol, 96%) der Titelverbindung als farblosen Feststoff.
¹H-NMR (CD₃OD): δ= 1,25-2(m,16H), 2,23(t,2H), 3,12-3,32(m,2H), 4,75(m,1H), 7,21 (d,2H), 7,35(d,2H).

### Beispiel 127:

### 7-[(1R,2S,5R)-2-[(4-Fluorphenyl)-sulfonylaminomethyl]-5-fluor- cyclopentyl]-heptansäure:

22 mg (55 »mol) der nach Beispiel 116 dargestellten Verbindung setzt man in Analogie zu Beispiel 94 um und isoliert nach Aufarbeitung und Reinigung 19 mg (47 »mol, 86%) der Titelverbindung als farblosen Feststoff.
¹H-NMR (CDCl₃): δ= 1,1-2,(m,16H), 2,36(t,2H), 2,83-3,08(m,2H), 4,73(m,1H), 5,08(t,1H), 7,2(m,2H), 7,89(m,2H).

### Beispiel 128:

### 7-[(1R,2S,5R)-2-[(4-Chlorphenyl)-sulfonylaminomethyl]-5-fluor-cyclopentyl]-heptansäure:

23 mg (56 »mol) der nach Beispiel 114 dargestellten Verbindung setzt man in Analogie zu Beispiel 94 um und isoliert nach Aufarbeitung und Reinigung 20 mg (48 »mol, 85%) der Titelverbindung als farblosen Feststoff.
¹H-NMR (CDCl₃): δ= 1,1-1,95,(m,16H), 2,3(t,2H), 2,76-3,(m,2H), 4,68(m,1H), 5,02(t,1H), 7,42(d,2H), 7,73(d,2H).

### Beispiel 129:

### 7-[(1R,2S,5R)-2-[(E/Z)-2-(4-Fluorphenylsulfonyl)-hydrazonomethyl]-5-fluor-cyclopentyl]-5(Z)-heptensäuremethylester:

165 mg (max. 596 »mol) der nach Beispiel 121a dargestellten Verbindung setzt man in Analogie zu Beispiel 62b unter Verwendung von 4-Fluorbenzolsulfonylhydrazid um und isoliert nach Aufarbeitung und Reinigung 225 mg (525 »mol, 88%) der Titelverbindung als farbloses Öl.
IR (Film): 3180, 2950, 2860, 1730, 1710, 1590, 1490, 1435, 1365, 1320, 1235, 1170, 1155, 1090, 835 und 670 cm⁻¹.

### Beispiel 130:

### 7-[(1R,2S,5R)-2-[(E/Z)-2-(4-Fluorphenylsulfonyl)-hydrazonometh yl]-5-fluor-cyclopentyl]-5(Z)-heptensäure:

60 mg (140 »mol) der nach Beispiel 129 dargestellten Verbindung verseift man in Analogie zu Beispiel 2 und isoliert nach Aufarbeitung und Reinigung 52 mg (125 »mol, 90%) der Titelverbindung als farbloses Öl.
¹H-NMR (CDCl₃): δ= 1,54-2,5(m,14H), 4,8(m,1H), 5,22-5,56(m,1H), 7,1-7,23(m,3H).

### Beispiel 131:

### 7-[(1R,2S,5R)-2-[(E/Z)-2-(4-Methylphenylsulfonyl)-hydrazonomethyl]-5-fluor-cyclopentyl]-5(Z)-heptensäuremethylester:

55 mg (max. 199 »mol) der nach Beispiel 121a dargestellten Verbindung setzt man in Analogie zu Beispiel 62b unter Verwendung von 4-Toluolsulfonylhydrazid um und isoliert nach Aufarbeitung und Reinigung 78 mg (192 »mol, 97%) der Titelverbindung als farbloses Öl.
IR (Film): 3200, 3000, 2950, 2870, 1730, 1710, 1595, 1435, 1360, 1320, 1160, 1090, 1030, 930, 810, 705 und 670 cm⁻¹.

### Beispiel 132:

### 7-[(1R,2S,5R)-2-[(E/Z)-2-(4-Methylphenylsulfonyl)-hydrazonomethyl]-5-fluor-cyclopentyl]-5(Z)-heptensäure:

39 mg (96 »mol) der nach Beispiel 131 dargestellten Verbindung verseift man in Analogie zu Beispiel 2 und isoliert nach Aufarbeitung und Reinigung 33 mg (84 »mol, 88%) der Titelverbindung als farblosen Feststoff.
¹H-NMR (CDCl₃): δ= 1,55-2,2(m,12H), 2,32(t,2H), 2,42(s,3H), 4,77(m,1H), 5,23-5,55(m,2H), 6,69(d,0,1H), 7,13(d,0,9H), 7,3(d,2H), 7,8(d,2H).

### Beispiel 133:

### 7-[(1R,2S,5R)-2-[(E/Z)-2-(4-Fluorphenylsulfonyl)-hydrazonomethyl]-5-fluor-cyclopentyl]-heptansäure:

37 mg (89 »mol) der nach Beispiel 130 dargestellten Verbindung setzt man in Analogie zu Beispiel 94 um und isoliert nach Aufarbeitung und Reinigung 14 mg (34 »mol, 38%) der Titelverbindung als farbloses Öl.
¹H-NMR (CD₃OD): δ= 1,0-1,75(m,15H), 2,08-2,22(m,3H), 4,62(m,1H), 7,04(d,1H), 7,21(m,2H), 7,82(m,2H)

### Beispiel 134:

### 7-[(1R,2S,5R)-2-[(E/Z)-2-(4-Methylphenylsulfonyl)-hydrazonomethyl]-5-fluor-cyclopentyl]-heptansäure:

19 mg (49 »mol) der nach Beispiel 132 dargestellten Verbindung setzt man in Analogie zu Beispiel 94 um und isoliert nach Aufarbeitung und Reinigung 9 mg (23 »mol, 47%) der Titelverbindung als farbloses Öl.
¹H-NMR (CD₃OD): δ= 1,12-1,94(m,16H), 2,16-2,32(m,3H), 2,92(s,3H), 4,7(m,1H), 7,12(d,1H), 7,37(d,2H), 7,76(d,2H)

### Beispiel 135:

### 7-[(1R,2S,5R)-2-[(E/Z)-3-(4-Chlorphenyl)-ureidoiminomethyl]-5-fluor-cyclopentyl]-heptansäure (A) und 7-[(1R,2S,5R)-2-[3-(4-Chlorphenyl)-ureidoaminomethyl]-5-fluor-cyclopentyl]-heptansäure (B):

20 mg (49 »mol) der nach Beispiel 124 dargestellten Verbindung setzt man in Analogie zu Beispiel 94 unter Verwendung von PtO₂ um und isoliert nach Aufarbeitung und Reinigung 11 mg (27 »mol, 54%) der Titelverbindung A als unpolare Komponente, sowie 8 mg (19 »mol, 39%) der Titelverbindung B jeweils als wachsartigen Feststoff.
¹H-NMR (CD₃OD) von A: δ= 1,27-2,12(m,15H), 2,23(t,2H), 2,43(m,1H), 4,78(m,2H), 7,22(d,1H), 7,27(d,2H), 7,5(d,2H)
¹H-NMR (CD₃OD) von B: δ= 1,25-2(m,16H), 2,26(t,2H), 2,74(dd,1H), 2,95(dd,1H), 4,76(m,1H), 7,27(d,2H), 7,45(d,2H)

## Patentansprüche

1. Cyclopentanderivate der Formel I, 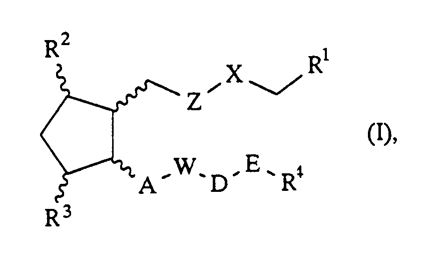 worin
R¹ 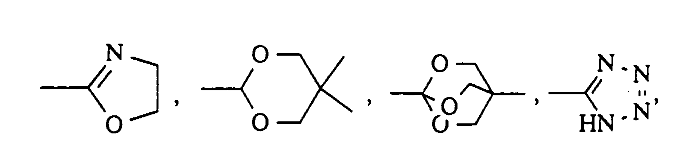 oder COOR⁵, wobei R⁵
Wasserstoff oder gegebenenfalls durch Halogen, Phenyl, C₁-C₄-Alkoxy oder Di-(C₁-C₄)-alkylamino substituiertes C₁-C₁₀-Alkyl, C₃-C₁₀-Cycloalkyl, C₇-C₁₆-Aralkyl, durch Y substituiertes Phenacyl oder C₆-C₁₂-Aryl oder einen 5- oder 6- gliedrigen heterocyclischen Rest mit wenigstens einem N-, O- oder S-Atom bedeuten kann, oder -CONHR⁷ mit R⁷ in der Bedeutung Wasserstoff, C₁-C₁₀-Alkyl, C₆-C₁₂-Arylsulfonyl, C₁-C₁₀-Alkanoyl oder C₁-C₁₀-Alkansulfonyl sein kann,
X -(CH₂)ₚ-, -CH₂-O-, oder -CH₂-S-,
Z, A unabhängig voneinander eine Direktbindung, (Z)-CH=CH-, (E)-CH=CH-, oder -C≡C-,
p 0 bis 5,
R² Fluor, OH,
n, r unabhängig voneinander 0 bis 2,
R³ OR⁶ oder R⁶, wobei R³ nur OR⁶ sein kann, wenn A allein oder zusammen mit W eine Direktbindung bedeutet
W eine Direktbindung, eine -[(CH₂)ₙ-V]_{q}-Gruppe, eine -(CH₂)ₙ-V-(CH₂)_{q}-V-Gruppe, eine freie oder funktionell abgewandelte Hydroxymethylengruppe oder eine freie oder funktionell abgewandelte 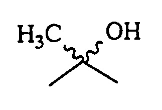 -Gruppe, wobei die Hydroxygruppe jeweils α- oder β-ständig sein kann,
q 1 oder 2,
D eine Direktbindung, eine geradkettige gesättigte Alkylengruppe mit 1-5 C-Atomen, eine verzweigte gesättigte Alkylengruppe oder eine geradkettige oder verzweigte ungesättigte Alkylengruppe mit 2-5 C-Atomen, die gegebenenfalls durch Fluoratome substituiert sein können, -(CH₂)ₙ-NH-SO₂-, 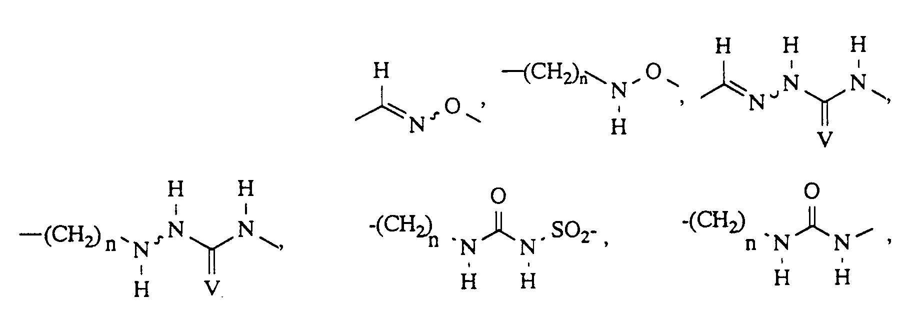 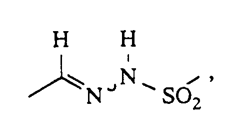 -(CH₂)ₙ-NH-NH-SO₂- oder 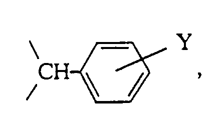 V ein O- oder S-Atom,
E eine Direktbindung, -C≡C- oder -CH≡CR⁸-, wobei R⁸ Wasserstoff, C₁-C₅-Alkyl, Halogen oder Trifluormethyl,
AWDE gemeinsam eine Direktbindung,
AW gemeinsam eine Direktbindung,
DE gemeinsam eine Direktbindung sein können, R⁴ 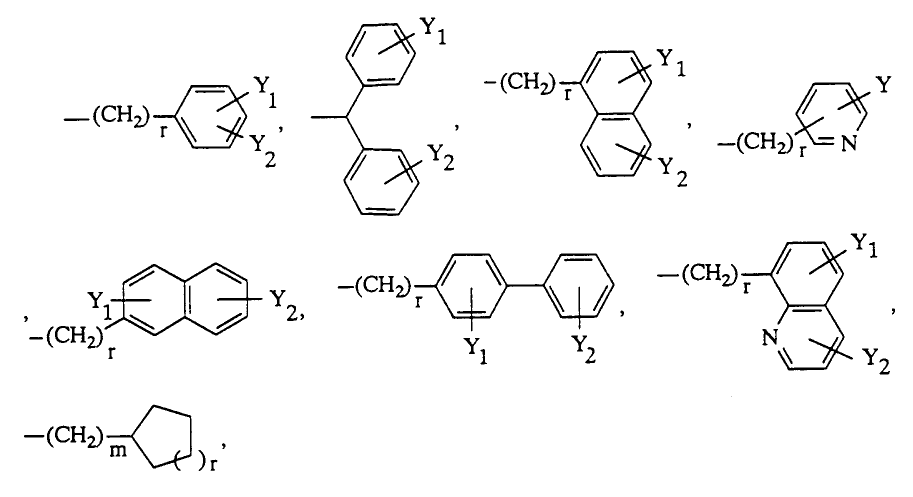 C₃-C₁₀-Cycloalkyl oder gegebenenfalls durch Y substituiertes C₁-C₁₀-Alkyl,
m 1 oder 2,
Y₁ und Y₂ gleich oder verschieden sind und Y bedeuten,
Y Wasserstoff, Halogen, N₃, CF₃, OR⁶, NO₂, NH₂, CN, COOR⁶ oder C₁-C₁₀-Alkyl,
R⁶ Wasserstoff, C₁-C₁₀-Alkyl, gegebenenfalls durch Halogen substituiertes C₆-C₁₂-Aryl oder C₇-C₁₆-Aralkyl sein kann und, falls R⁵ Wasserstoff bedeutet, deren Salze mit physiologisch verträglichen Basen, sowie die α-, β- oder γ-Cyclodextrinclathrate, sowie die mit Liposomen verkapselten Verbindungen der Formel I bedeuten.

2. Arzneimittel bestehend aus einer oder mehreren Verbindungen des Anspruchs 1 und üblichen Hilfs-, Träger- und Zusatzstoffen.

3. Verfahren zur Herstellung von Cyclopentan-Derivaten der Formel I, dadurch gekennzeichnet, daß man die Hydroxy-Verbindung der Formel II 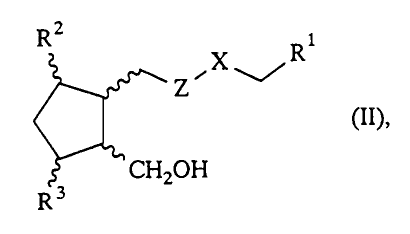 worin R¹, R², R³, R⁴, X und Z die oben angegebenen Bedeutungen haben und R¹ eine -COOR⁵-Estergruppe mit R⁵ in der mit Ausnahme von Wasserstoff oben angegebenen Bedeutung darstellt, mit einer Halogenverbindung der Formel Hal-W-R⁴ (III), worin Hal, W und R⁴ die oben genannten Bedeutungen haben oder nach Oxidation mit Oxalylchlorid/DMSO mit einem Dimethylphosphonat der Formel V 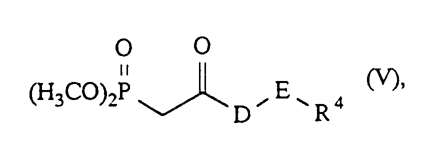 worin D, E und R⁴ die oben genannte Bedeutung haben, in Gegenwart von Natriumhydrid oder Natriumhydrid/Brom umsetzt und anschliessend reduziert und gegebenenfalls Bromwasserstoff abspaltet oder das Oxidationsprodukt aus II und Oxalylchlorid/DMSO mit einem Amin der Formel H₂N-O-R⁴ (X), H₂N-NH-SO₂-R⁴ (XIII) oder 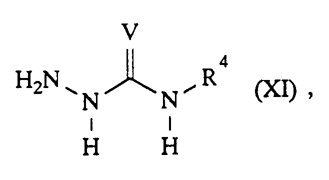 worin R⁴ die oben angegebene Bedeutung hat, umsetzt oder nach Oxidation und Umsetzung mit Thionylchlorid und Natriumazid das intermediäre Amin der Formel 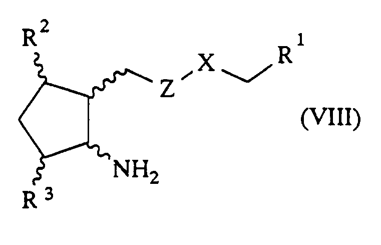 mit einer Verbindung der Formel Hal-SO₂-R⁴ (IX) oder O=C=N-R⁴ (XII), worin Hal und R⁴ die oben genannte Bedeutung hat, umsetzt oder nach Bromierung, Azidbildung und Reduktion das intermediäre Amin der Formel 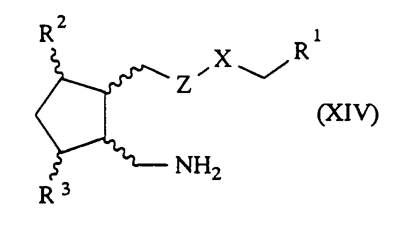 mit einer Verbindung der Formel Hal-SO₂-R⁴ (IX) oder O=C=N-R⁴ (XII), worin Hal und R⁴ die oben genannte Bedeutung hat, umsetzt, gegebenenfalls C-C-Mehrfachbindungen hydriert und die erhaltenen Ester verseift, in Salze überführt, in Cyclodextrinclathrate umwandelt oder mit Liposomen verkapselt.

## Claims

1. Cyclopentane derivatives of formula I 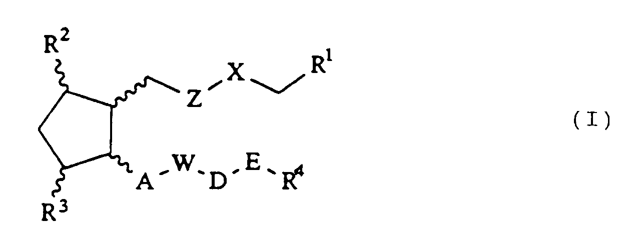 in which
R¹ may be 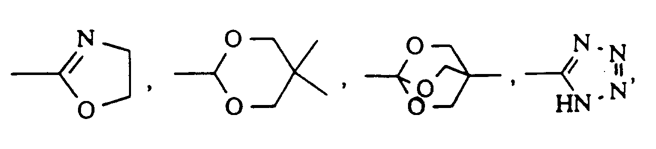 or COOR⁵, wherein R⁵ may represent hydrogen or C₁-C₁₀-alkyl that is optionally substituted by halogen, phenyl, C₁-C₄-alkoxy or by di-(C₁-C₄)-alkylamino, C₃-C₁₀-cycloalkyl, C₇-C₁₆-aralkyl, Y-substituted phenacyl or C₆-C₁₂-aryl, or a 5- or 6-membered heterocyclic radical having at least one N, O or S atom, or R¹ may be -CONHR⁷,
wherein R⁷ represents hydrogen, C₁-C₁₀-alkyl, C₆-C₁₂-arylsulphonyl, C₁-C₁₀-alkanoyl or C₁-C₁₀-alkanesulphonyl,
X may be (CH₂)ₚ-, -CH₂-O- or -CH₂-S-,
Z and A, each independently of the other, may be a direct bond, (Z)-CH=CH-, (E)-CH=CH- or -C≡C-,
p may be from 0 to 5,
R² may be fluorine or OH,
n and r, each independently of the other, may be from 0 to 2,
R³ may be OR⁶ or R⁶, it being possible for R³ to be OR⁶ only when A, on its own or together with W, represents a direct bond,
W may be a direct bond, a -[(CH₂)ₙ-V]_{q}- group, a -(CH₂)ₙ-V-(CH₂)_{q}-V- group, a free or functionally modified hydroxymethylene group or a free or functionally modified 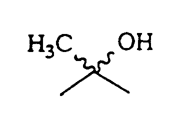 group, it being possible for the hydroxy group in each case to be in the α- or β-configuration,
q may be 1 or 2,
D may be a direct bond, a straight-chained saturated alkylene group having from 1 to 5 carbon atoms, a branched saturated alkylene group or a straight-chained or branched unsaturated alkylene group having from 2 to 5 carbon atoms, each of which may optionally be substituted by fluorine atoms, or D may be -(CH₂)ₙ-NH-SO₂-, 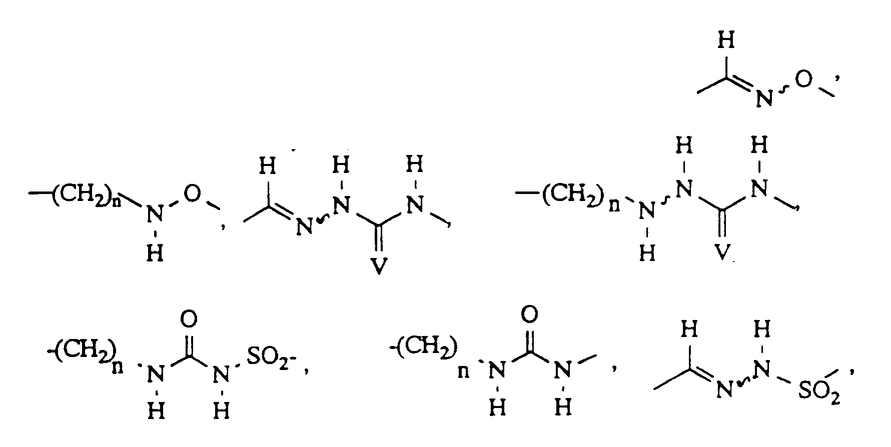 -(CH₂)ₙ-NH-NH-SO₂- or 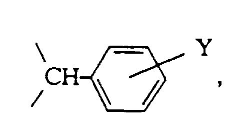
V may be an O or S atom,
E may be a direct bond, -C≡C- or -CH=CR⁸-, wherein R⁸ may be hydrogen, C₁-C₅-alkyl, halogen or trifluoromethyl,
AWDE together may be a direct bond,
AW together may be a direct bond,
DE together may be a direct bond,
R⁴ may be 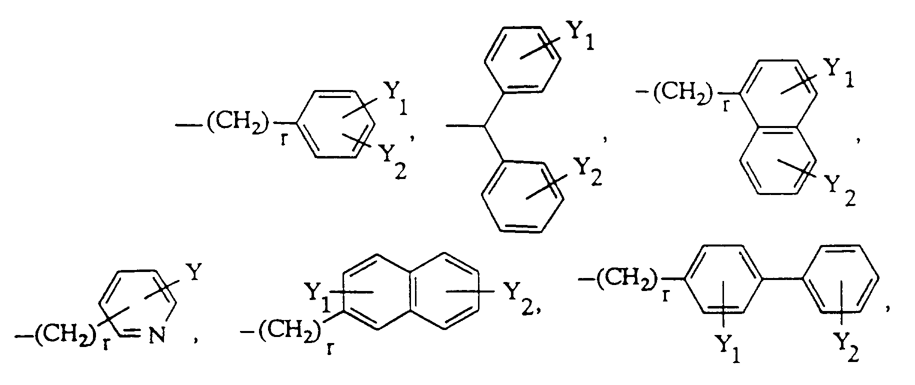 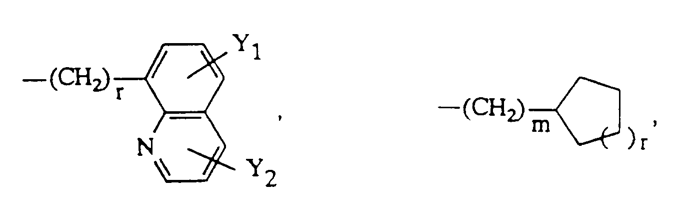 C₃-C₁₀-cycloalkyl, or C₁-C₁₀-alkyl that is optionally substituted by Y,
m may be 1 or 2,
Y₁ and Y₂ are identical or different and represent Y,
Y may be hydrogen, halogen, N₃, CF₃, OR⁶, NO₂, NH₂, CN, COOR⁶ or C₁-C₁₀-alkyl, and
R⁶ may be hydrogen, C₁-C₁₀-alkyl, optionally halo-substituted C₆-C₁₂-aryl, or C₇-C₁₆-aralkyl,
and, when R⁵ represents hydrogen, the salts thereof with physiologically tolerable bases, as well as the α-, β- or γ-cyclodextrin clathrates, as well as the compounds of formula I encapsulated with liposomes.

2. Medicament comprising one or more compounds of claim 1 and customary adjuvants, carriers and additives.

3. Process for the preparation of cyclopentane derivatives of formula I, characterised in that the hydroxy compound of formula II 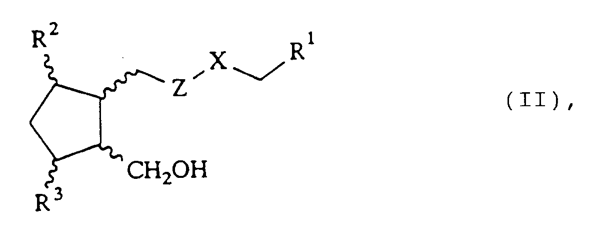 in which R¹, R², R³, R⁴, X and Z have the meanings given above and R¹ represents a -COOR⁵ ester group, wherein R⁵ has the meanings given above except for hydrogen, is reacted, in the presence of sodium hydride or sodium hydride/bromine, with a halogen compound of the formula Hal-W-R⁴ (III), wherein Hal, W and R⁴ have the meanings given above, or, after oxidation with oxalyl chloride/DMSO, with a dimethyl phosphonate of formula V 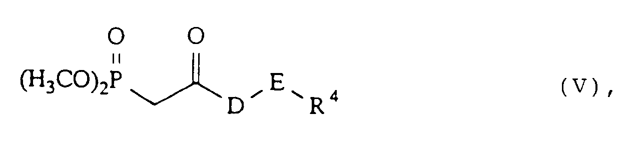 wherein D, E and R⁴ have the meanings given above, and then the resulting product is reduced and, where appropriate, hydrogen bromide is removed, or the oxidation product of II and oxalyl chloride/DMSO is reacted with an amine of the formula H₂N-O-R⁴ (X), H₂N-NH-SO₂-R⁴ (XIII) or 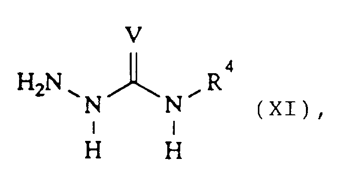 wherein R⁴ has the meanings given above, or, after oxidation and reaction with thionyl chloride and sodium azide, the intermediate amine of formula 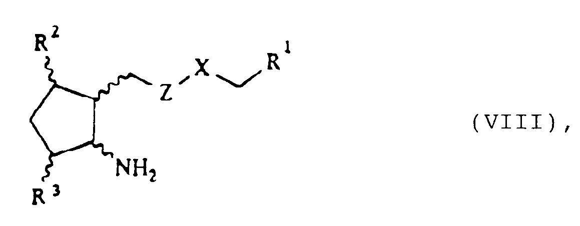 is reacted with a compound of the formula Hal-SO₂-R⁴ (IX) or O=C=N-R⁴ (XII), wherein Hal and R⁴ have the meanings given above, or, after bromination, azide formation and reduction, the intermediate amine of formula 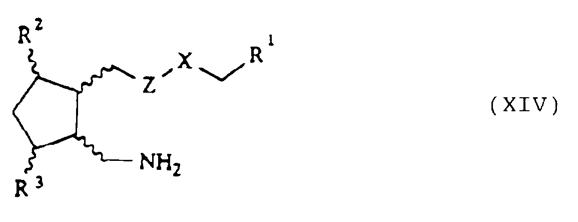 is reacted with a compound of the formula Hal-SO₂-R⁴ (IX) or O=C=N-R⁴ (XII), wherein Hal and R⁴ have the meanings given above, C-C multiple bonds are optionally hydrogenated, and the resulting esters are hydrolysed, converted into salts, converted into cyclodextrin clathrates or encapsulated with liposomes.

## Revendications

1. Dérivés de cyclopentane de formule I, 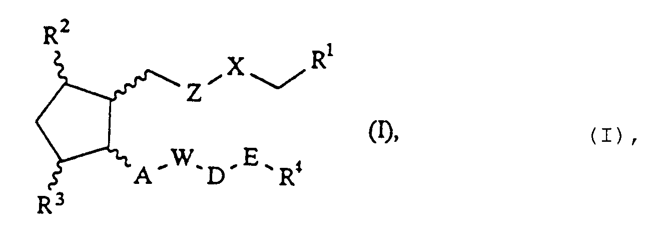 où
R¹ 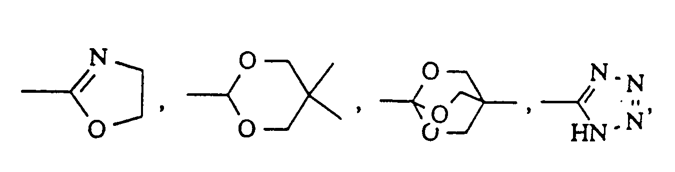 ou COOR⁵,
R⁵ pouvant représenter hydrogène ou aralkyle en C₇-C₁₆, cycloalkyle en C₃-C₁₀, alkyle en C₁-C₁₀ éventuellement substitué par halogène, phényle, alcoxy en C₁-C₄ ou (dialkyle en C₁-C₄)-amino, phénacyle substitué par Y ou aryle en C₆-C₁₂ ou un radical hétérocyclique à 5 ou 6 chaînons avec au moins un atome de N, O ou S, ou représente -CONHR⁷ avec R⁷ pouvant signifier hydrogène, alkyle en C₁-C₁₀, (aryle en C₆-C₁₂)-sulfonyle, alcanoyle en C₁-C₁₀ ou (alcane en C₁-C₁₀)-sulfonyle,
X -(CH₂)ₚ-, -CH₂-O- ou -CH₂-S-,
Z, A représentent, indépendamment l'un de l'autre, une liaison directe, (Z)-CH=CH, (E)-CH=CH-, ou -C≡C-
P va de 0 à 5,
R² représente fluor, OH,
n, r, indépendamment l'un de l'autre, vont de 0 à 2,
R³ représente OR⁶ ou R⁶, R³ pouvant être seulement OR⁶, lorsque A seul ou avec W signifie une liaison directe,
W représente une liaison directe, un groupe -[(CH₂)ₙ-V]_{q}, un groupe -(CH₂)ₙ-V-(CH₂)_{q}-V-, un groupe hydroxyméthylène libre ou fonctionnellement
modifié, ou un groupe 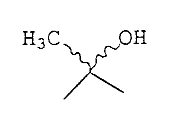 libre ou fonctionnellement modifié, le groupe hydroxy pouvant être, respectivement en position α ou β,
q vaut 1 ou 2,
D représente une liaison directe, un groupe alkylène ramifié saturé avec 1 à 5 atomes de C, un groupe alkylène saturé ramifié ou un groupe alkylène linéaire ou ramifié insaturé avec 2 à 5 atomes de C, qui peuvent être éventuellement substitués par des atomes de fluor, 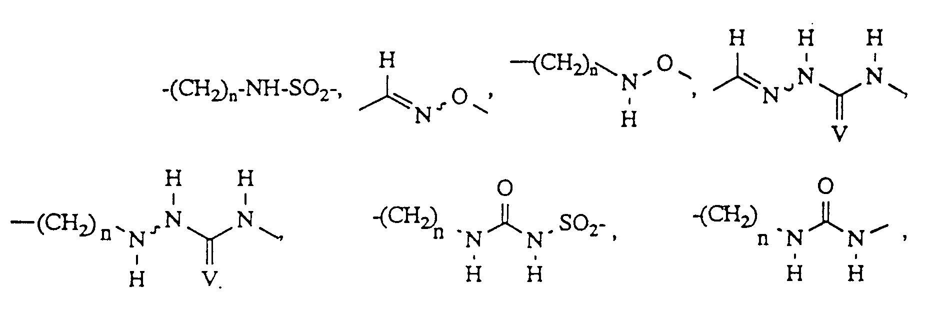 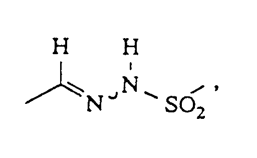 -(CH₂)ₙ-NH-NH-SO₂- ou 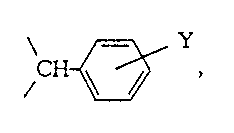
V représente un atome de O ou S,
E représente une liaison directe -C≡C- ou -CH=CR⁸-, R⁸ étant hydrogène, alkyle en C₁-C₅, halogène ou trifluorométhyle,
AWDE ensemble peuvent être une liaison directe,
AW ensemble peuvent être une liaison directe,
DE ensemble peuvent être une liaison directe, 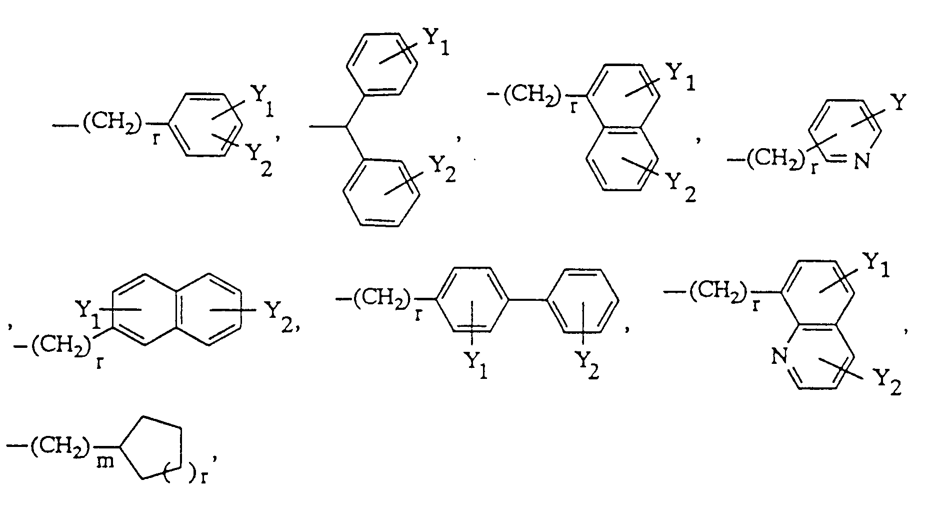 R⁴ cycloalkyle en C₃-C₁₀ ou alkyle en C₁-C₁₀ éventuellement substitué par Y,
m vaut 1 ou 2,
Y₁ et Y₂ sont identiques ou différents et représentent Y,
Y peut être hydrogène, halogène, N₃, CF₃, OR⁶, NO₂, NH₂CN, COOR⁶ ou alkyle en C₁-C₁₀,
R⁶ peut être hydrogène, alkyle en C₁-C₁₀, aralkyle en C₇-C₁₆, aryle en C₆-C₁₂ éventuellement substitué par halogène, dans le cas où R⁵ représente l' hydrogène, ses sels avec des bases physiologiquement compatibles, ainsi que les clathrates d'α-, de β- ou de γ-cyclodextrine ainsi que les composés de formule I encapsulés dans des liposomes.

2. Médicaments composés d'un ou plusieurs composés de la revendication 1 et des adjuvants, supports et additifs usuels.

3. Procédé pour la préparation de dérivés de cyclopentane de formule I, caractérisé en ce qu'on fait réagir le composé hydroxy de formule II : 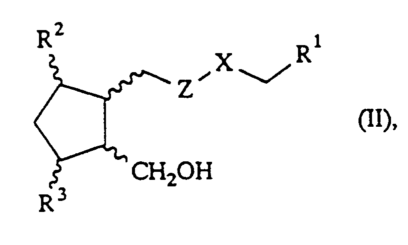 où R¹, R², R³, R⁴, X et Z ont les significations données ci-dessus et R représente un groupe -COOR⁵ avec R⁵ ayant la signification donnée ci-dessus à l'exception de l'hydrogène, avec un composé halogéné de formule Hal-W-R⁴ (III) dans laquelle Hal, W et R⁴ ont les significations données ci-dessus et après oxydation par le chlorure d'oxalyle/DMSO avec un phosphonate de diméthyle de formule V : 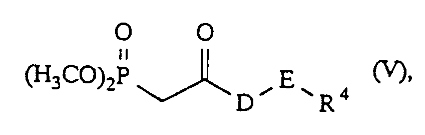 où D, E et R⁴ ont la signification donnée ci-dessus, en présence de l'hydrure de sodium ou de l'hydrure de sodium/brome et ensuite on concentre et éventuellement on sépare le bromure d'hydrogène ou le produit d'oxydation du II et on fait réagir le chlorure d'oxalyle/DMSO avec une amine de formule H₂N-O-R⁴ (X), H₂N-NH-SO₂-R⁴ (XIII) ou 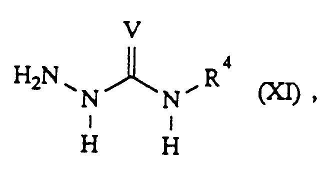 où R⁴ a la signification donnée ci-dessus, et après oxydation et réaction avec le chlorure de thionyle et l'azide de sodium, on fait réagir l'amine intermédiaire de formule : 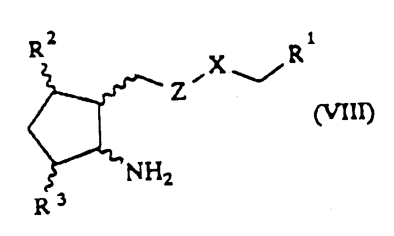 avec un composé de formule Hal-SO₂-R⁴ (IX) ou O=C=N-R⁴ (XII), où Hal et R⁴ ont la signification donnée ci-dessus, où après bromuration, formation d'azide et réduction, on fait réagir l'amine intermédiaire de formule : 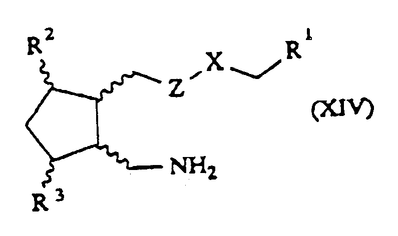 avec un composé de formule Hal-SO₂-R⁴ (IX) ou O=C=N-R⁴ (XII), où Hal et R⁴ ont la signification donnée ci-dessus, éventuellement on hydrogène les liaisons multiples C-C et on saponifie l'ester obtenu, on transforme en sels, on transforme en clathrates de cyclodextrine ou on encapsule dans des liposomes.
